# EUROPEAN PATENT APPLICATION

(11) **EP 4 537 810 A1**
(43) Date of publication of application: **16.04.2025**
(21) Application number: 23819880.8
(22) Date of filing: 07.06.2023
(51) Int. Cl.: A61K 8/68, A23G 1/00, A23L 33/105, A61K 8/06, A61K 8/9789, A61K 31/164, A61K 36/185, A61P 17/16, A61Q 5/00, A61Q 19/00

(54) **CACAO-DERIVED COMPOSITION, AND MOISTURIZER, SKIN TEXTURE IMPROVING AGENT, HAIR TEXTURE IMPROVING AGENT, SKIN COSMETIC, HAIR COSMETIC, PHARMACEUTICAL COMPOSITION AND FOOD COMPOSITION EACH CONTAINING CACAO-DERIVED COMPOSITION**

(30) Priority: 07.06.2022 JP 2022092603
(71) Applicant: MEIJI CO., LTD, Chuo-ku Tokyo 104-8306 (JP)
(72) Inventor: KOGA Jinichiro, Utsunomiya-shi, Tochigi 320-8551 (JP); YOSHIDA Haruna, Hachioji-shi, Tokyo 192-0919 (JP); MIYAZAKI Megumi, Hachioji-shi, Tokyo 192-0919 (JP); NANAMI Shintaro, Hachioji-shi, Tokyo 192-0919 (JP); NAKAYAMA Kai, Hachioji-shi, Tokyo 192-0919 (JP); MATSUDA Koki, Hachioji-shi, Tokyo 192-0919 (JP); BABA Kento, Hachioji-shi, Tokyo 192-0919 (JP); NISHIYAMA Yuri, Hachioji-shi, Tokyo 192-0919 (JP); SAKIYAMA Kazuya, Hachioji-shi, Tokyo 192-0919 (JP)
(74) Representative: TBK
(86) International application number: PCT/JP2023/021267
(87) International publication number: WO 2023/238900

(57) **Abstract**

An object of the present invention is to provide a cacao derived composition containing a free ceramide, as well as a moisturizing agent, a skin quality improving agent, a hair quality improving agent, a cosmetic for skin, a cosmetic for hair, a pharmaceutical composition and a food composition each containing the cacao derived composition, and the present invention provides a cacao derived composition containing an extract of cacao bean shell, as well as a moisturizing agent, a skin quality improving agent, a hair quality improving agent, a cosmetic for skin, a cosmetic for hair, a pharmaceutical composition and a food composition each containing the cacao derived composition, wherein the extract of cacao bean shell contains a cacao derived free ceramide, and wherein the extract of cacao bean shell is obtained by a method including: a step of winnowing a crushed product of cacao bean to separate it into a cacao bean nib fraction and a cacao bean shell fraction and obtaining the cacao bean shell fraction as a first cacao derived raw material; and a step of subjecting the first cacao derived raw material to an extraction treatment with an extraction solvent to obtain the extract of cacao bean shell.

## Description

### FIELD OF THE INVENTION

The present invention relates to a cacao derived composition, as well as a moisturizing agent, a skin quality improving agent, a hair quality improving agent, a cosmetic for skin, a cosmetic for hair, a pharmaceutical composition and a food composition each containing the cacao derived composition.

### BACKGROUND ART

Ceramides are the main component of intercellular lipids present in the stratum corneum of human epidermis, and are said to be an important component in the barrier function and moisturizing function of skin. It is also widely known that the ceramide amount (in particular, ceramide AP amount) decreases along with increasing age, and it is necessary to supplement ceramides by application to the skin or by oral ingestion.

Ceramides present in human epidermis are free ceramides (ceramides in free form). Free ceramides are structurally different from sphingoglycolipids (glycoceramides) generally contained in plants, and therefore, it is difficult to obtain a large amount of free ceramides from materials present in the natural world.

Patent Document 1 discloses a method for obtaining a free ceramide from chestnut skin.

### CITATION LIST

### PATENT DOCUMENT

Patent Document 1: International Publication No. WO 2018/021476

### SUMMARY OF INVENTION

### TECHNICAL PROBLEM

An object of the present invention is to provide a cacao derived composition containing a free ceramide, as well as a moisturizing agent, a skin quality improving agent, a hair quality improving agent, a cosmetic for skin, a cosmetic for hair, a pharmaceutical composition and a food composition each containing the cacao derived composition.

### SOLUTION TO PROBLEM

The present invention provides the following inventions.
[A1] A cacao derived composition containing a cacao derived component, wherein the cacao derived component contains a ceramide AP.
[A2] The cacao derived composition according to [A1], wherein the cacao derived component contains a free ceramide other than the ceramide AP, and wherein the mass ratio of the amount of the ceramide AP to the total amount of the ceramide AP and the free ceramide other than the ceramide AP is 0.30 or more.
[A3] The cacao derived composition according to [A1] or [A2], wherein the cacao derived component contains a glucosylceramide, and wherein the mass ratio of the amount of the ceramide AP to the amount of the glucosylceramide is 0.0020 or more.
[A4] The cacao derived composition according to any one of [A1] to [A3], wherein the cacao derived component contains a free ceramide other than the ceramide AP and a glucosylceramide, and wherein the mass ratio of the total amount of the ceramide AP and the free ceramide other than the ceramide AP to the amount of the glucosylceramide is 0.0050 or more.
[A5] The cacao derived composition according to any one of [A1] to [A4], wherein the cacao derived component contains a free ceramide other than the ceramide AP and a glucosylceramide, and wherein the mass ratio of the total amount of the ceramide AP and the free ceramide other than the ceramide AP to the total amount of the ceramide AP, the free ceramide other than the ceramide AP, and the glucosylceramide is 0.0050 or more.
[A6] The cacao derived composition according to any one of [A1] to [A5], wherein the cacao derived component contains a free ceramide other than the ceramide AP and a glucosylceramide, and wherein the mass ratio of the amount of the ceramide AP to the total amount of the ceramide AP, the free ceramide other than the ceramide AP, and the glucosylceramide is 0.0020 or more.
[A7] The cacao derived composition according to any one of [A1] to [A6], wherein the cacao derived component is a component obtained by subjecting one or two or more extraction raw materials selected from cacao pod shell, cacao pulp, cacao bean shell, cacao bean nib, and cacao bean germ to an extraction treatment with an extraction solvent.
[A8] The cacao derived composition according to [A7], wherein the extraction raw material contains cacao bean shell.
[A9] A moisturizing agent containing the cacao derived composition according to any one of [A1] to [A8].
[A10] The moisturizing agent according to [A9], wherein the amount of the ceramide AP is 0.001% by mass or more based on the mass of the moisturizing agent.
[A11] A skin quality improving agent containing the cacao derived composition according to any one of [A1] to [A8].
[A12] The skin quality improving agent according to [A11], wherein the amount of the ceramide AP is 0.001% by mass or more based on the mass of the skin quality improving agent.
[A13] A hair quality improving agent containing the cacao derived composition according to any one of [A1] to [A8].
[A14] The hair quality improving agent according to [A13], wherein the amount of the ceramide AP is 0.001% by mass or more based on the mass of the hair quality improving agent.
[A15] A cosmetic for skin containing the cacao derived composition according to any one of [A1] to [A8].
[A16] The cosmetic for skin according to [A15], wherein the amount of the ceramide AP is 0.001% by mass or more based on the mass of the cosmetic for skin.
[A17] The cosmetic for skin according to [A15] or [A16], wherein the cosmetic for skin is a cosmetic for scalp.
[A18] A cosmetic for hair containing the cacao derived composition according to any one of [A1] to [A8].
[A19] The cosmetic for hair according to [A18], wherein the amount of the ceramide AP is 0.001% by mass or more based on the mass of the cosmetic for hair.
[A20] A pharmaceutical composition containing the cacao derived composition according to any one of [A1] to [A8].
[A21] The pharmaceutical composition according to [A20], wherein the amount of the ceramide AP is 0.001% by mass or more based on the mass of the pharmaceutical composition.
[A22] A food composition containing the cacao derived composition according to any one of [A1] to [A8].
[A23] The food composition according to [A22], wherein the amount of the ceramide AP is 0.0006% by mass or more based on the mass of the food composition.
[A24] A method for moisturizing skin or hair of a subject, including applying the cacao derived composition according to any one of [A1] to [A8] to skin or hair of the subject.
[A25] A method for improving skin quality of a subject, including applying the cacao derived composition according to any one of [A1] to [A8] to skin of the subject.
[A26] A method for improving hair quality of a subject, including applying the cacao derived composition according to any one of [A1] to [A8] to hair of the subject.
[A27] Use of the cacao derived composition according to any one of [A1] to [A8] for moisturizing skin or hair of a subject.
[A28] Use of the cacao derived composition according to any one of [A1] to [A8] for improving skin quality of a subject.
[A29] Use of the cacao derived composition according to any one of [A1] to [A8] for improving hair quality of a subject.
[A30] Use of the cacao derived composition according to any one of [A1] to [A8] as an active component of a moisturizing agent.
[A31] Use of the cacao derived composition according to any one of [A1] to [A8] as an active component of a skin quality improving agent.
[A32] Use of the cacao derived composition according to any one of [A1] to [A8] as an active component of a hair quality improving agent.
[A33] Use of the cacao derived composition according to any one of [A1] to [A8] in production of a moisturizing agent.
[A34] Use of the cacao derived composition according to any one of [A1] to [A8] in production of a skin quality improving agent.
[A35] Use of the cacao derived composition according to any one of [A1] to [A8] in production of a hair quality improving agent.
[A36] Use of the cacao derived composition according to any one of [A1] to [A8] in production of a cosmetic for skin.
[A37] The use according to [A36], wherein the cosmetic for skin is a cosmetic for scalp.
[A38] Use of the cacao derived composition according to any one of [A1] to [A8] in production of a cosmetic for hair.
[A39] Use of the cacao derived composition according to any one of [A1] to [A8] in production of a pharmaceutical composition.
[A40] Use of the cacao derived composition according to any one of [A1] to [A8] in production of a food composition.

The present invention also provides the following inventions.
[B1] A cacao derived composition containing a cacao derived free ceramide, wherein the amount of the cacao derived free ceramide is 1.2% by mass or more based on the mass of the cacao derived composition.
[B2] A moisturizing agent containing the cacao derived composition according to [B1].
[B3] The moisturizing agent according to [B2], wherein the amount of the cacao derived free ceramide is 0.001% by mass or more based on the mass of the moisturizing agent.
[B4] A skin quality improving agent containing the cacao derived composition according to [B1].
[B5] The skin quality improving agent according to [B4], wherein the amount of the cacao derived free ceramide is 0.001% by mass or more based on the mass of the skin quality improving agent.
[B6] A hair quality improving agent containing the cacao derived composition according to [B1].
[B7] The hair quality improving agent according to [B6], wherein the amount of the cacao derived free ceramide is 0.001% by mass or more based on the mass of the hair quality improving agent.
[B8] A cosmetic for skin containing the cacao derived composition according to [B1].
[B9] The cosmetic for skin according to [B8], wherein the amount of the cacao derived free ceramide is 0.001% by mass or more based on the mass of the cosmetic for skin.
[B10] The cosmetic for skin according to [B8] or [B9], wherein the cosmetic for skin is a cosmetic for scalp.
[B11] A cosmetic for hair containing the cacao derived composition according to [B1].
[B12] The cosmetic for hair according to [B11], wherein the amount of the cacao derived free ceramide is 0.001% by mass or more based on the mass of the cosmetic for hair.
[B13] A pharmaceutical composition containing the cacao derived composition according to [B1].
[B14] The pharmaceutical composition according to [B13], wherein the amount of the cacao derived free ceramide is 0.001% by mass or more based on the mass of the pharmaceutical composition.
[B15] A food composition containing the cacao derived composition according to [B1].
[B16] The food composition according to [B15], wherein the amount of the cacao derived free ceramide is 0.0006% by mass or more based on the mass of the food composition.
[B17] A method for moisturizing skin or hair of a subject, including applying the cacao derived composition according to [B1] to skin or hair of the subject.
[B18] A method for improving skin quality of a subject, including applying the cacao derived composition according to [B1] to skin of the subject.
[B19] A method for improving hair quality of a subject, including applying the cacao derived composition according to [B1] to hair of the subject.
[B20] Use of the cacao derived composition according to [B1] for moisturizing skin or hair of a subject.
[B21] Use of the cacao derived composition according to [B1] for improving skin quality of a subject.
[B22] Use of the cacao derived composition according to [B1] for improving hair quality of a subject.
[B23] Use of the cacao derived composition according to [B1] as an active component of a moisturizing agent.
[B24] Use of the cacao derived composition according to [B1] as an active component of a skin quality improving agent.
[B25] Use of the cacao derived composition according to [B1] as an active component of a hair quality improving agent.
[B26] Use of the cacao derived composition according to [B1] in production of a moisturizing agent.
[B27] Use of the cacao derived composition according to [B1] in production of a skin quality improving agent.
[B28] Use of the cacao derived composition according to [B1] in production of a hair quality improving agent.
[B29] Use of the cacao derived composition according to [B1] in production of a cosmetic for skin.
[B30] The use according to [B29], wherein the cosmetic for skin is a cosmetic for scalp.
[B31] Use of the cacao derived composition according to [B1] in production of a cosmetic for hair.
[B32] Use of the cacao derived composition according to [B1] in production of a pharmaceutical composition.
[B33] Use of the cacao derived composition according to [B1] in production of a food composition.

The present invention also provides the following inventions.
[C1] A cacao derived composition containing an extract of cacao bean shell, wherein the extract of cacao bean shell contains a cacao derived free ceramide, and wherein the extract of cacao bean shell is obtained by a method including the following steps:
   (a) a step of winnowing a crushed product of cacao bean to separate it into a cacao bean nib fraction and a cacao bean shell fraction and obtaining the cacao bean shell fraction as a first cacao derived raw material;
   (b) optionally, a step of sorting out, from the first cacao derived raw material, a raw material whose size does not allow passing through a 16 mesh to obtain a second cacao derived raw material; and
   (c) a step of subjecting the first or second cacao derived raw material to an extraction treatment with an extraction solvent to obtain the extract of cacao bean shell.
[C2] The cacao derived composition according to [C1], wherein in the step (c), the first or second cacao derived raw material is subjected to extraction with an extraction solvent, and a resultant extract is then purified to obtain the extract of cacao bean shell.
[C3] A moisturizing agent containing the cacao derived composition according to [C1] or [C2].
[C4] The moisturizing agent according to [C3], wherein the amount of the cacao derived free ceramide is 0.001% by mass or more based on the mass of the moisturizing agent.
[C5] A skin quality improving agent containing the cacao derived composition according to [C1] or [C2].
[C6] The skin quality improving agent according to [C5], wherein the amount of the cacao derived free ceramide is 0.001% by mass or more based on the mass of the skin quality improving agent.
[C7] A hair quality improving agent containing the cacao derived composition according to [C1] or [C2].
[C8] The hair quality improving agent according to [C7], wherein the amount of the cacao derived free ceramide is 0.001% by mass or more based on the mass of the hair quality improving agent.
[C9] A cosmetic for skin containing the cacao derived composition according to [C1] or [C2].
[C10] The cosmetic for skin according to [C9], wherein the amount of the cacao derived free ceramide is 0.001% by mass or more based on the mass of the cosmetic for skin.
[C11] The cosmetic for skin according to [C9] or [C10], wherein the cosmetic for skin is a cosmetic for scalp.
[C12] A cosmetic for hair containing the cacao derived composition according to [C1] or [C2].
[C13] The cosmetic for hair according to [C12], wherein the amount of the cacao derived free ceramide is 0.001% by mass or more based on the mass of the cosmetic for hair.
[C14] A pharmaceutical composition containing the cacao derived composition according to [C1] or [C2].
[C15] The pharmaceutical composition according to [C14], wherein the amount of the cacao derived free ceramide is 0.001% by mass or more based on the mass of the pharmaceutical composition.
[C16] A food composition containing the cacao derived composition according to [C1] or [C2].
[C17] The food composition according to [C16], wherein the amount of the cacao derived free ceramide is 0.0006% by mass or more based on the mass of the food composition.
[C18] A method for moisturizing skin or hair of a subject, including applying the cacao derived composition according to [C1] or [C2] to skin or hair of the subject.
[C19] A method for improving skin quality of a subject, including applying the cacao derived composition according to [C1] or [C2] to skin of the subject.
[C20] A method for improving hair quality of a subject, including applying the cacao derived composition according to [C1] or [C2] to hair of the subject.
[C21] Use of the cacao derived composition according to [C1] or [C2] for moisturizing skin or hair of a subject.
[C22] Use of the cacao derived composition according to [C1] or [C2] for improving skin quality of a subject.
[C23] Use of the cacao derived composition according to [C1] or [C2] for improving hair quality of a subject.
[C24] Use of the cacao derived composition according to [C1] or [C2] as an active component of a moisturizing agent.
[C25] Use of the cacao derived composition according to [C1] or [C2] as an active component of a skin quality improving agent.
[C26] Use of the cacao derived composition according to [C1] or [C2] as an active component of a hair quality improving agent.
[C27] Use of the cacao derived composition according to [C1] or [C2] in production of a moisturizing agent.
[C28] Use of the cacao derived composition according to [C1] or [C2] in production of a skin quality improving agent.
[C29] Use of the cacao derived composition according to [C1] or [C2] in production of a hair quality improving agent.
[C30] Use of the cacao derived composition according to [C1] or [C2] in production of a cosmetic for skin.
[C31] The use according to [C30], wherein the cosmetic for skin is a cosmetic for scalp.
[C32] Use of the cacao derived composition according to [C1] or [C2] in production of a cosmetic for hair.
[C33] Use of the cacao derived composition according to [C1] or [C2] in production of a pharmaceutical composition.
[C34] Use of the cacao derived composition according to [C1] or [C2] in production of a food composition.

### ADVANTAGEOUS EFFECTS OF INVENTION

According to the present invention, there is provided a cacao derived composition containing a free ceramide, as well as a moisturizing agent, a skin quality improving agent, a hair quality improving agent, a cosmetic for skin, a cosmetic for hair, a pharmaceutical composition and a food composition each containing the cacao derived composition.

### DETAILED DESCRIPTION OF THE INVENTION

### <<Cacao derived composition>>

According to one aspect, the present invention relates to a cacao derived composition.

The form of the cacao derived composition is not particularly limited. Examples of the form of the cacao derived composition include powder, paste, and liquid forms.

The cacao derived composition is a composition containing one or two or more cacao derived components. The cacao derived composition may contain, in addition to the one or two or more cacao derived components, one or two or more other components. In the present specification, the one or two or more cacao derived components are simply referred to as "cacao-derived component".

A cacao pod (cacao fruit) has a hard shell and contains the flesh (pulp) and cacao beans (seeds) inside the shell. A cacao bean has an outer skin (shell) and contains the endosperm (nib) and the embryo bud (germ) inside the outer skin. In the present specification, the shell of a cacao pod is referred to as "cacao pod shell", the flesh inside a cacao pod is referred to as "cacao pulp", the outer skin of a cacao bean is referred to as "cacao bean shell", the endosperm of a cacao bean is referred to as "cacao bean nib", and the embryo bud of a cacao bean is referred to as "cacao bean germ".

The cacao derived component is a component obtained using the entire cacao pod or a portion thereof as a raw material. The breed and place of production of cacao is not particularly restricted. Examples of the breed of cacao include the Forastero, Criollo, and Trinitario groups, as well as derivatives or hybrids thereof. Examples of the place of production of cacao include Ghana, Cote d'Ivoire, Nigeria, Brazil, Venezuela, Trinidad and Tobago, and Dominica. Examples of the portion of a cacao pod used as a raw material include cacao pod shell, cacao pulp, cacao bean shell, cacao bean nib, and cacao bean germ. One portion of a cacao pod may be used as a raw material, or two or more portions of a cacao pod may be used as a raw material. From the viewpoint of efficiently obtaining the target cacao derived component (free ceramide (for example, ceramide AP and/or free ceramide other than the ceramide AP), glucosylceramide, and others, in particular free ceramide), the portion of a cacao pod used as a raw material preferably contains a cacao bean or a portion thereof (for example, cacao bean shell, cacao bean nib, cacao bean germ, and others), and more preferably contains cacao bean shell. Examples of the cacao derived component include a ceramide AP, a free ceramide other than the ceramide AP, and a glucosylceramide. The cacao derived component can contain one or two or more among these.

A cacao bean can be harvested from a cacao pod along with the pulp. A harvested cacao bean may be used as it is as a raw material, or a cacao bean that has been subjected to one or two or more treatments after harvesting may be used as a raw material. The one or two or more treatments performed after harvesting can be selected from, for example, a fermentation treatment, a pulp removal treatment, a crushing treatment, sieving of a crushed product, a grinding treatment, a drying treatment, a sterilization treatment, and a roasting treatment. From the viewpoint of efficiently obtaining the target cacao derived component (free ceramide (for example, ceramide AP and/or free ceramide other than the ceramide AP), glucosylceramide, and others, in particular free ceramide), the temperature of the roasting treatment is preferably lower. Also, from the same viewpoint, the duration of the fermentation treatment is preferably shorter. Note that the fermentation treatment is a treatment in which a cacao bean is kept under fermentable conditions, and fermentation of the cacao bean can occur immediately after taking out the cacao bean from a cacao pod.

The cacao derived component is preferably a component obtained by subjecting one or two or more extraction raw materials selected from cacao pod shell, cacao pulp, cacao bean shell, cacao bean nib, and cacao bean germ to an extraction treatment with an extraction solvent. That is, the cacao derived composition preferably contains one or two or more selected from an extract of cacao pod shell, an extract of cacao pulp, an extract of cacao bean shell, an extract of cacao bean nib, and an extract of cacao bean germ. The cacao derived composition may be composed of one or two or more selected from these extracts. Each of these extracts contains the cacao derived component (free ceramide (for example, ceramide AP and/or free ceramide other than the ceramide AP), glucosylceramide, and others, in particular free ceramide).

From the viewpoint of improving the moisturizing action, skin quality improving action, and hair quality improving action of the cacao derived composition, the extraction raw material preferably contains cacao bean shell. That is, the cacao derived composition preferably contains an extract of cacao bean shell. The extraction raw material may contain, in addition to cacao bean shell, one or two or more selected from cacao pod shell, cacao pulp, cacao bean nib, and cacao bean germ. That is, the cacao derived composition may contain, in addition to an extract of cacao bean shell, one or two or more selected from an extract of cacao pod shell, an extract of cacao pulp, an extract of cacao bean nib, and an extract of cacao bean germ.

The extract of cacao pod shell, the extract of cacao pulp, the extract of cacao bean shell, the extract of cacao bean nib, and the extract of cacao bean germ are extracts obtained using cacao pod shell, cacao pulp, cacao bean shell, cacao bean nib, and cacao bean germ as extraction raw materials, respectively. Cacao pod shell, cacao pulp, cacao bean shell, cacao bean nib, and cacao bean germ can each be obtained by separating them from a cacao pod according to conventional methods.

The extract can be obtained by subjecting the extraction raw material to a treatment such as drying treatment, crushing treatment, and sieving of a crushed product, as necessary, and then subjecting the extraction raw material to an extraction treatment with an extraction solvent. Prior to the extraction treatment, the extraction raw material may be subjected to a degreasing treatment with a non-polar solvent (for example, hexane).

The extraction treatment is a treatment in which the target cacao derived component (free ceramide (for example, ceramide AP and/or free ceramide other than the ceramide AP), glucosylceramide, and others, in particular free ceramide) is extracted with an extraction solvent from the extraction raw material, and can be performed according to conventional methods. The extraction treatment can be performed by, for example, contacting the extraction raw material with the extraction solvent. The amount of the extraction solvent used is, for example, 1 to 8 L per 100 g of the extraction raw material. The temperature at which the extraction raw material is contacted with the extraction solvent is, for example, 25 to 80°C. The time during which the extraction raw material is contacted with the extraction solvent is, for example, 1 to 24 hours. The contact between the extraction raw material and the extraction solvent can be performed by, for example, immersing the extraction raw material in the extraction solvent and stirring them as necessary. After the extraction treatment, the mixture of the extraction raw material and the extraction solvent can be subjected to a solid liquid separation treatment to remove the extraction residue, thereby obtaining an extracted liquid. The solid liquid separation treatment can be selected from, for example, filtration (for example, natural filtration, suction filtration, and others), centrifugation, decantation, and others. The extracted liquid can be diluted or concentrated, thereby obtaining a diluted liquid or a concentrated liquid. By drying the extracted liquid, diluted liquid, or concentrated liquid, a dried product can be obtained. By purifying the extracted liquid, diluted liquid, concentrated liquid, or dried product, a crudely purified product or a purified product can be obtained. Dilution, concentration, drying, and purification can each be performed according to conventional methods. The extract encompasses all of the following forms: extracted liquid, diluted liquid, concentrated liquid, dried product, crudely purified product, and purified product.

The extraction solvent is not particularly limited as long as it can extract the target cacao derived component (free ceramide (for example, ceramide AP and/or free ceramide other than the ceramide AP), glucosylceramide, and others, in particular free ceramide). A single solvent may be used as the extraction solvent, or a mixed solvent of two or more may be used as the extraction solvent. The extraction solvent is preferably used at room temperature or at a temperature of the boiling point or lower.

The extraction solvent can be selected from polar solvents, preferably aliphatic alcohols having 1 to 6 carbon atoms. Examples of the aliphatic alcohols having 1 to 6 carbon atoms include methanol, ethanol, propyl alcohol, butyl alcohol, pentyl alcohol, and hexyl alcohol. The aliphatic alcohols may be linear, or may be branched. Taking propyl alcohol as an example, it may be n-propyl alcohol, or may be isopropyl alcohol. From the viewpoint of efficiently extracting the target cacao derived component (free ceramide (for example, ceramide AP and/or free ceramide other than the ceramide AP), glucosylceramide, and others, in particular free ceramide), aliphatic alcohols having 2 to 6 carbon atoms are preferred, and ethanol is particularly preferred.

From the viewpoint of efficiently extracting the target cacao derived component (free ceramide (for example, ceramide AP and/or free ceramide other than the ceramide AP), glucosylceramide, and others, in particular free ceramide), the amount of methanol in the extraction solvent is preferably smaller. The content of methanol is preferably 30% by volume or less, more preferably 20% by volume or less, and even more preferably 10% by volume or less, based on the volume of the extraction solvent. The lower limit is zero.

From the viewpoint of efficiently extracting the target cacao derived component (free ceramide (for example, ceramide AP and/or free ceramide other than the ceramide AP), glucosylceramide, and others, in particular free ceramide), the amount of non-polar solvents (for example, pentane, octane, hexane, and others) in the extraction solvent is preferably smaller. The total content of non-polar solvents is preferably 60% by volume or less, more preferably 50% by volume or less, and even more preferably 40% by volume or less, based on the volume of the extraction solvent. The lower limit is zero.

From the viewpoint of efficiently extracting the target cacao derived component (free ceramide (for example, ceramide AP and/or free ceramide other than the ceramide AP), glucosylceramide, and others, in particular free ceramide), the amount of water in the extraction solvent is preferably smaller. The content of water is preferably 5% by volume or less, more preferably 1% by volume or less, and even more preferably 0.5% by volume or less, based on the volume of the extraction solvent. The lower limit is zero.

From the viewpoint of efficiently extracting the target cacao derived component (free ceramide (for example, ceramide AP and/or free ceramide other than the ceramide AP), glucosylceramide, and others, in particular free ceramide), the amount of polyhydric alcohols (for example, propylene glycol, butylene glycol, glycerin, and others) in the extraction solvent is preferably smaller. The total content of polyhydric alcohols is preferably 20% by volume or less, more preferably 10% by volume or less, and even more preferably 5% by volume or less, based on the volume of the extraction solvent. The lower limit is zero.

Hereinafter, embodiments of the cacao derived composition will be described.

### <First embodiment>

The cacao derived composition according to the first embodiment has Feature A described later. The cacao derived composition having Feature A described later has a moisturizing action, a skin quality improving action, and a hair quality improving action. From the viewpoint of improving the moisturizing action, skin quality improving action, and hair quality improving action of the cacao derived composition, the cacao derived composition according to the first embodiment preferably has, in addition to Feature A described later, one or two or more of Features B to H described later.

The moisturizing action is demonstrated through, for example, an action of suppressing the amount of transepidermal water loss. However, the moisturizing action is not limited to a moisturizing action demonstrated through an action of suppressing the amount of transepidermal water loss. The skin quality improving action is demonstrated through, for example, a moisturizing action. However, the skin quality improving action is not limited to a skin quality improving action demonstrated through a moisturizing action. The target skin may be skin on any part of the body, and examples thereof include skin on the face, head, neck, back, shoulders, breasts, abdomen, arms, and legs. Skin quality improvement encompasses preventing, treating, or improving symptoms caused by a decline in the moisturizing function of skin (including aging symptoms), such as dry skin, rough skin, sagging, dullness, formation of spots or wrinkles, a decline in the flexibility or elasticity of skin, and a decline in the skin barrier function. The hair quality improving action is demonstrated through, for example, a moisturizing action. However, the hair quality improving action is not limited to a hair quality improving action demonstrated through a moisturizing action. The target hair may be hair that grows on any part of the body, and examples thereof include hair on the head, eyebrows, axillary hair, mustache and beard, and body hair. Hair quality improvement encompasses preventing, treating, or improving symptoms caused by a decline in the moisturizing function of hair (including aging symptoms), such as dryness of hair, roughness of hair, and a decline in the flexibility or elasticity of hair.

### [Feature A]

The cacao derived component preferably contains a ceramide AP. The cacao derived component may contain one type of ceramide AP, or may contain two or more types of ceramide APs.

The ceramide AP is a compound composed of phytosphingosine and an α-hydroxy fatty acid that is amide bonded to the phytosphingosine. The amide bond is formed by the amino group of phytosphingosine and the carboxyl group of an α-hydroxy fatty acid. The ceramide AP is a type of free ceramide.

From the viewpoint of improving the moisturizing action, skin quality improving action, and hair quality improving action of the cacao derived composition, the number of carbon atoms in the α-hydroxy fatty acid constituting the ceramide AP is preferably 16 to 36, more preferably 20 to 26, and even more preferably 24 to 26.

From the viewpoint of improving the moisturizing action, skin quality improving action, and hair quality improving action of the cacao derived composition, the number of carbon-carbon double bonds in the α-hydroxy fatty acid constituting the ceramide AP is preferably 0 to 2, more preferably 0 or 1, and even more preferably 0.

From the viewpoint of improving the moisturizing action, skin quality improving action, and hair quality improving action of the cacao derived composition, the number of hydroxyl groups in the α-hydroxy fatty acid constituting the ceramide AP is preferably 1, but may be 2 or more. The α-hydroxy fatty acid has an α-hydroxyl group. The α-hydroxy fatty acid may have a hydroxyl group other than an α-hydroxyl group (for example, β-hydroxyl group).

### [Feature B]

The cacao derived component preferably contains a free ceramide other than the ceramide AP. The cacao derived component may contain one type of free ceramide other than the ceramide AP, or may contain two or more types of free ceramides other than the ceramide AP.

The free ceramide is a compound composed of a ceramide skeleton. The ceramide skeleton is composed of a sphingoid base and a fatty acid that is amide bonded to the sphingoid base. The amide bond is formed by the amino group of a sphingoid base and the carboxyl group of a fatty acid. No saccharide, phosphoric acid, or other substance is bonded to hydroxyl groups in the sphingoid base in the free ceramide.

From the viewpoint of improving the moisturizing action, skin quality improving action, and hair quality improving action of the cacao derived composition, the number of carbon atoms in the sphingoid base constituting the free ceramide other than the ceramide AP is preferably 14 to 24, more preferably 16 to 20, and even more preferably 18.

From the viewpoint of improving the moisturizing action, skin quality improving action, and hair quality improving action of the cacao derived composition, the number of carbon-carbon double bonds in the sphingoid base constituting the free ceramide other than the ceramide AP is preferably 0 to 2, more preferably 0 or 1, and even more preferably 0. In the case where the number of carbon-carbon double bonds is 1, the position and EZ configuration of the carbon-carbon double bond is, for example, 8Z, 8E, or the like. In the case where the number of carbon-carbon double bonds is 2, the position and EZ configuration of the carbon-carbon double bonds is, for example, (4E,8Z), (4E,8E), or the like.

From the viewpoint of improving the moisturizing action, skin quality improving action, and hair quality improving action of the cacao derived composition, the number of hydroxyl groups in the sphingoid base constituting the free ceramide other than the ceramide AP is preferably 1 to 3, more preferably 2 or 3, and even more preferably 3.

Examples of the sphingoid base constituting the free ceramide other than the ceramide AP include dihydrosphingosine, sphingosine, phytosphingosine, 6-hydroxysphingosine, 4-hydroxysphingenine, and 4,8-sphingadienine. From the viewpoint of improving the moisturizing action, skin quality improving action, and hair quality improving action of the cacao derived composition, dihydrosphingosine and 4-hydroxysphingenine are preferred, and 4-hydroxysphingenine is more preferred.

From the viewpoint of improving the moisturizing action, skin quality improving action, and hair quality improving action of the cacao derived composition, the number of carbon atoms in the fatty acid constituting the free ceramide other than the ceramide AP is preferably 16 to 36, more preferably 20 to 26, and even more preferably 24 to 26.

From the viewpoint of improving the moisturizing action, skin quality improving action, and hair quality improving action of the cacao derived composition, the number of carbon-carbon double bonds in the fatty acid constituting the free ceramide other than the ceramide AP is preferably 0 to 2, more preferably 0 or 1, and even more preferably 0.

From the viewpoint of improving the moisturizing action, skin quality improving action, and hair quality improving action of the cacao derived composition, the number of hydroxyl groups in the fatty acid constituting the free ceramide other than the ceramide AP is preferably 0 to 2, more preferably 1 or 2, and even more preferably 1.

The fatty acid constituting the free ceramide other than the ceramide AP can be selected from, for example, nonhydroxy fatty acids, monohydroxy fatty acids, dihydroxy fatty acids, ester ω-hydroxy fatty acids, and others.

Examples of the combination of (sphingoid base) - (fatty acid) constituting the free ceramide other than the ceramide AP include the following combinations.
(1) The combination of (sphingoid base in which the number of hydroxyl groups is 2, the number of carbon atoms is 14 to 24 (in particular, 16 to 20), and the number of carbon-carbon double bonds is 0) - (fatty acid in which the number of carbon atoms is 16 to 36 (in particular, 20 to 26) and the number of carbon-carbon double bonds is 0)
(2) The combination of (sphingoid base in which the number of hydroxyl groups is 2, the number of carbon atoms is 14 to 24 (in particular, 16 to 20), and the number of carbon-carbon double bonds is 1) - (fatty acid in which the number of carbon atoms is 16 to 36 (in particular, 20 to 26) and the number of carbon-carbon double bonds is 0)
(3) The combination of (sphingoid base in which the number of hydroxyl groups is 2, the number of carbon atoms is 14 to 24 (in particular, 16 to 20), and the number of carbon-carbon double bonds is 2) - (fatty acid in which the number of carbon atoms is 16 to 36 (in particular, 20 to 26) and the number of carbon-carbon double bonds is 0)
(4) The combination of (sphingoid base in which the number of hydroxyl groups is 3, the number of carbon atoms is 14 to 24 (in particular, 16 to 20), and the number of carbon-carbon double bonds is 0) - (fatty acid in which the number of carbon atoms is 16 to 36 (in particular, 20 to 26) and the number of carbon-carbon double bonds is 0)
(5) The combination of (sphingoid base in which the number of hydroxyl groups is 3, the number of carbon atoms is 14 to 24 (in particular, 16 to 20), and the number of carbon-carbon double bonds is 1) - (fatty acid in which the number of carbon atoms is 16 to 36 (in particular, 20 to 26) and the number of carbon-carbon double bonds is 0)
(6) The combination of (sphingoid base in which the number of hydroxyl groups is 3, the number of carbon atoms is 14 to 24 (in particular, 16 to 20), and the number of carbon-carbon double bonds is 2) - (fatty acid in which the number of carbon atoms is 16 to 36 (in particular, 20 to 26) and the number of carbon-carbon double bonds is 0)

From the viewpoint of improving the moisturizing action, skin quality improving action, and hair quality improving action of the cacao derived composition, the cacao derived component preferably contains one or two or more selected from free ceramides other than the ceramide AP wherein the combinations of (sphingoid base) - (fatty acid) in the free ceramides other than the ceramide AP are the combinations (1) to (6). In the combinations (1) to (6), the number of hydroxyl groups in the fatty acid is preferably 0 to 2, more preferably 1 or 2, and even more preferably 1. In the combinations (1) to (6), the fatty acid may have an α-hydroxyl group. In the combinations (1) to (6), the fatty acid may have a hydroxyl group other than an α-hydroxyl group (for example, β-hydroxyl group).

The free ceramide encompasses the following free ceramides, which result from bonding patterns between sphingoid bases and fatty acids.

**[Table A]**

| | Nonhydroxy fatty acid | α-Hydroxy fatty acid | Ester ω-hydroxy fatty acid |
|---|---|---|---|
| Dihydrosphingosine | Ceramide NDS | Ceramide ADS | Ceramide EODS |
| Sphingosine | Ceramide NS | Ceramide AS | Ceramide EOS |
| Phytosphingosine | Ceramide NP | Ceramide AP | Ceramide EOP |
| 6-Hydroxysphingosine | Ceramide NH | Ceramide AH | Ceramide EOH |

From the viewpoint of improving the moisturizing action, skin quality improving action, and hair quality improving action of the cacao derived composition, the cacao derived component preferably contains one or two or more selected from free ceramides other than the ceramide AP wherein the combination of (sphingoid base) - (fatty acid) in the free ceramides other than the ceramide AP is (sphingoid base in which the number of hydroxyl groups is 2 or 3, the number of carbon atoms is 18, and the number of carbon-carbon double bonds is 0 or 1) - (fatty acid in which the number of carbon atoms is 20 to 26, the number of carbon-carbon double bonds is 0, and the number of hydroxyl groups is 0 to 2).

### [Feature C]

The cacao derived component preferably contains a glucosylceramide. The cacao derived component may contain one type of glucosylceramide, or may contain two or more types of glucosylceramides.

The glucosylceramide is a compound composed of a ceramide skeleton and glucose that is glycosidically bonded to a hydroxyl group of a sphingoid base in the ceramide skeleton.

The ceramide skeleton is composed of a sphingoid base and a fatty acid that is amide bonded to the sphingoid base. The amide bond is formed by the amino group of a sphingoid base and the carboxyl group of a fatty acid.

From the viewpoint of improving the moisturizing action, skin quality improving action, and hair quality improving action of the cacao derived composition, the number of carbon atoms in the sphingoid base constituting the glucosylceramide is preferably 14 to 24, more preferably 16 to 20, and even more preferably 18.

From the viewpoint of improving the moisturizing action, skin quality improving action, and hair quality improving action of the cacao derived composition, the number of carbon-carbon double bonds in the sphingoid base constituting the glucosylceramide is preferably 0 to 2, and more preferably 1 or 2. In the case where the number of carbon-carbon double bonds is 1, the position and EZ configuration of the carbon-carbon double bond is, for example, 8Z, 8E, or the like. In the case where the number of carbon-carbon double bonds is 2, the position and EZ configuration of the carbon-carbon double bonds is, for example, (4E,8Z), (4E,8E), or the like.

From the viewpoint of improving the moisturizing action, skin quality improving action, and hair quality improving action of the cacao derived composition, the number of hydroxyl groups in the sphingoid base constituting the glucosylceramide is preferably 1 to 3, more preferably 2 or 3, and even more preferably 3. Note that the number of hydroxyl groups in the sphingoid base constituting the glucosylceramide includes the hydroxyl group to which glucose is glycosidically bonded, as well.

Examples of the sphingoid base constituting the glucosylceramide include dihydrosphingosine, sphingosine, phytosphingosine, 6-hydroxysphingosine, 4-hydroxysphingenine, and 4,8-sphingadienine. From the viewpoint of improving the moisturizing action, skin quality improving action, and hair quality improving action of the cacao derived composition, dihydrosphingosine and 4-hydroxysphingenine are preferred, and 4-hydroxysphingenine is more preferred.

From the viewpoint of improving the moisturizing action, skin quality improving action, and hair quality improving action of the cacao derived composition, the number of carbon atoms in the fatty acid constituting the glucosylceramide is preferably 16 to 36, more preferably 20 to 26, and even more preferably 24 to 26.

From the viewpoint of improving the moisturizing action, skin quality improving action, and hair quality improving action of the cacao derived composition, the number of carbon-carbon double bonds in the fatty acid constituting the glucosylceramide is preferably 0 to 2, more preferably 0 or 1, and even more preferably 0.

From the viewpoint of improving the moisturizing action, skin quality improving action, and hair quality improving action of the cacao derived composition, the number of hydroxyl groups in the fatty acid constituting the glucosylceramide is preferably 0 to 2, more preferably 1 or 2, and even more preferably 1.

The fatty acid constituting the glucosylceramide can be selected from, for example, nonhydroxy fatty acids, monohydroxy fatty acids, dihydroxy fatty acids, ester ω-hydroxy fatty acids, and others. The fatty acid constituting the glucosylceramide may have an α-hydroxy group (that is, it may be an α-hydroxy fatty acid). The fatty acid constituting the glucosylceramide may have a hydroxyl group other than an α-hydroxyl group (for example, β-hydroxyl group).

Examples of the combination of (sphingoid base) - (fatty acid) constituting the glucosylceramide include the combinations (1) to (6) described above. From the viewpoint of improving the moisturizing action, skin quality improving action, and hair quality improving action of the cacao derived composition, the cacao derived component preferably contains one or two or more selected from glucosylceramides wherein the combinations of (sphingoid base) - (fatty acid) in the glucosylceramides are the combinations (1) to (6). In the combinations (1) to (6), the number of hydroxyl groups in the fatty acid is preferably 0 to 2, more preferably 1 or 2, and even more preferably 1. In the combinations (1) to (6), the fatty acid may have an α-hydroxyl group. In the combinations (1) to (6), the fatty acid may have a hydroxyl group other than an α-hydroxyl group (for example, β-hydroxyl group).

The cacao derived component may contain one or two or more glycosylceramides other than the glucosylceramide. The glycosylceramide is a compound composed of a ceramide skeleton and a saccharide that is glycosidically bonded to a hydroxyl group of a sphingoid base in the ceramide skeleton. Examples of the glycosylceramide other than the glucosylceramide include a galactosylceramide. The galactosylceramide is a compound composed of a ceramide skeleton and galactose that is glycosidically bonded to a hydroxyl group of a sphingoid base in the ceramide skeleton.

### [Feature D]

The cacao derived component preferably contains a ceramide AP, a free ceramide other than the ceramide AP, and a glucosylceramide. The cacao derived component may contain one type of free ceramide other than the ceramide AP, or may contain two or more types of free ceramides other than the ceramide AP. The cacao derived component may contain one type of glucosylceramide, or may contain two or more types of glucosylceramides.

The explanations about the ceramide AP, the free ceramide other than the ceramide AP, and the glucosylceramide are as described above.

### [Feature E]

In the case where the cacao derived component contains a ceramide AP and a free ceramide other than the ceramide AP, the mass ratio of the amount of the ceramide AP to the total amount of the ceramide AP and the free ceramide other than the ceramide AP (hereinafter, referred to as "ratio A") is preferably 0.30 or more, more preferably 0.35 or more, even more preferably 0.40 or more, and even more preferably 0.45 or more.

The upper limit of the ratio A is not particularly limited, but the ratio A is preferably 0.99 or less, more preferably 0.95 or less, even more preferably 0.90 or less, even more preferably 0.85 or less, and even more preferably 0.80 or less, in mass ratio. Each of these upper limits may be combined with any of the lower limits described above.

The "amount of the ceramide AP" means, in the case where the cacao derived component contains one type of ceramide AP, the amount of the one type of ceramide AP, and in the case where the cacao derived component contains two or more types of ceramide APs, the total amount of the two or more types of ceramide APs.

The "amount of the free ceramide other than the ceramide AP" means, in the case where the cacao derived component contains one type of free ceramide other than the ceramide AP, the amount of the one type of free ceramide, and in the case where the cacao derived component contains two or more types of free ceramides other than the ceramide AP, the total amount of the two or more types of free ceramides.

The ratio A is calculated based on the amount of the ceramide AP contained in the cacao derived component and the amount of the free ceramide contained in the cacao derived component. The cacao derived composition may contain or may not contain a ceramide AP other than the ceramide AP contained in the cacao derived component. In the case where the cacao derived composition contains a ceramide AP other than the ceramide AP contained in the cacao derived component, the amount of the ceramide AP other than the ceramide AP contained in the cacao derived component is not used when calculating the ratio A. The cacao derived composition may contain or may not contain a free ceramide other than the free ceramide contained in the cacao derived component. In the case where the cacao derived composition contains a free ceramide other than the free ceramide contained in the cacao derived component, the amount of the free ceramide other than the free ceramide contained in the cacao derived component is not used when calculating the ratio A.

### [Feature F]

In the case where the cacao derived component contains a ceramide AP and a glucosylceramide, the mass ratio of the amount of the ceramide AP to the amount of the glucosylceramide (hereinafter, referred to as "ratio B") is preferably 0.0020 or more, more preferably 0.040 or more, even more preferably 0.080 or more, even more preferably 0.12 or more, and even more preferably 0.16 or more.

The upper limit of the ratio B is not particularly limited, but the ratio B is preferably 5.0 or less, more preferably 4.0 or less, even more preferably 3.0 or less, and even more preferably 2.0 or less, in mass ratio. Each of these upper limits may be combined with any of the lower limits described above.

The meaning of the "amount of the ceramide AP" is as described above.

The "amount of the glucosylceramide" means, in the case where the cacao derived component contains one type of glucosylceramide, the amount of the one type of glucosylceramide, and in the case where the cacao derived component contains two or more types of glucosylceramides, the total amount of the two or more types of glucosylceramides.

The ratio B is calculated based on the amount of the ceramide AP contained in the cacao derived component and the amount of the glucosylceramide contained in the cacao derived component. The cacao derived composition may contain or may not contain a ceramide AP other than the ceramide AP contained in the cacao derived component. In the case where the cacao derived composition contains a ceramide AP other than the ceramide AP contained in the cacao derived component, the amount of the ceramide AP other than the ceramide AP contained in the cacao derived component is not used when calculating the ratio B. The cacao derived composition may contain or may not contain a glucosylceramide other than the glucosylceramide contained in the cacao derived component. In the case where the cacao derived composition contains a glucosylceramide other than the glucosylceramide contained in the cacao derived component, the amount of the glucosylceramide other than the glucosylceramide contained in the cacao derived component is not used when calculating the ratio B.

### [Feature G]

In the case where the cacao derived component contains a ceramide AP, a free ceramide other than the ceramide AP, and a glucosylceramide, the mass ratio of the total amount of the ceramide AP and the free ceramide other than the ceramide AP to the amount of the glucosylceramide (hereinafter, referred to as "ratio C") is preferably 0.0050 or more, more preferably 0.50 or more, even more preferably 1.0 or more, and even more preferably 2.0 or more.

The upper limit of the ratio C is not particularly limited, but the ratio C is preferably 10 or less, more preferably 9.0 or less, even more preferably 8.0 or less, and even more preferably 7.0 or less, in mass ratio. Each of these upper limits may be combined with any of the lower limits described above.

The meanings of the "amount of the ceramide AP", the "amount of the free ceramide other than the ceramide AP", and the "amount of the glucosylceramide" are as described above.

The ratio C is calculated based on the amount of the ceramide AP contained in the cacao derived component, the amount of the free ceramide contained in the cacao derived component, and the amount of the glucosylceramide contained in the cacao derived component. The cacao derived composition may contain or may not contain a ceramide AP other than the ceramide AP contained in the cacao derived component. In the case where the cacao derived composition contains a ceramide AP other than the ceramide AP contained in the cacao derived component, the amount of the ceramide AP other than the ceramide AP contained in the cacao derived component is not used when calculating the ratio C. The cacao derived composition may contain or may not contain a free ceramide other than the free ceramide contained in the cacao derived component. In the case where the cacao derived composition contains a free ceramide other than the free ceramide contained in the cacao derived component, the amount of the free ceramide other than the free ceramide contained in the cacao derived component is not used when calculating the ratio C. The cacao derived composition may contain or may not contain a glucosylceramide other than the glucosylceramide contained in the cacao derived component. In the case where the cacao derived composition contains a glucosylceramide other than the glucosylceramide contained in the cacao derived component, the amount of the glucosylceramide other than the glucosylceramide contained in the cacao derived component is not used when calculating the ratio C.

### [Feature H]

In the case where the cacao derived component contains a ceramide AP, a free ceramide other than the ceramide AP, and a glucosylceramide, the mass ratio of the total amount of the ceramide AP and the free ceramide other than the ceramide AP to the total amount of the ceramide AP, the free ceramide other than the ceramide AP, and the glucosylceramide (hereinafter, referred to as "ratio D") is preferably 0.0050 or more, more preferably 0.10 or more, even more preferably 0.20 or more, and even more preferably 0.30 or more.

The upper limit of the ratio D is not particularly limited, but the ratio D is preferably 3.0 or less, more preferably 2.0 or less, and even more preferably 1.0 or less, in mass ratio. Each of these upper limits may be combined with any of the lower limits described above.

The meanings of the "amount of the ceramide AP", the "amount of the free ceramide other than the ceramide AP", and the "amount of the glucosylceramide" are as described above.

The ratio D is calculated based on the amount of the ceramide AP contained in the cacao derived component, the amount of the free ceramide contained in the cacao derived component, and the amount of the glucosylceramide contained in the cacao derived component. The cacao derived composition may contain or may not contain a ceramide AP other than the ceramide AP contained in the cacao derived component. In the case where the cacao derived composition contains a ceramide AP other than the ceramide AP contained in the cacao derived component, the amount of the ceramide AP other than the ceramide AP contained in the cacao derived component is not used when calculating the ratio D. The cacao derived composition may contain or may not contain a free ceramide other than the free ceramide contained in the cacao derived component. In the case where the cacao derived composition contains a free ceramide other than the free ceramide contained in the cacao derived component, the amount of the free ceramide other than the free ceramide contained in the cacao derived component is not used when calculating the ratio D. The cacao derived composition may contain or may not contain a glucosylceramide other than the glucosylceramide contained in the cacao derived component. In the case where the cacao derived composition contains a glucosylceramide other than the glucosylceramide contained in the cacao derived component, the amount of the glucosylceramide other than the glucosylceramide contained in the cacao derived component is not used when calculating the ratio D.

### [Feature I]

In the case where the cacao derived component contains a ceramide AP, a free ceramide other than the ceramide AP, and a glucosylceramide, the mass ratio of the amount of the ceramide AP to the total amount of the ceramide AP, the free ceramide other than the ceramide AP, and the glucosylceramide (hereinafter, referred to as "ratio E") is preferably 0.0020 or more, more preferably 0.040 or more, even more preferably 0.080 or more, even more preferably 0.12 or more, and even more preferably 0.16 or more.

The upper limit of the ratio E is not particularly limited, but the ratio E is preferably 1.0 or less, more preferably 0.9 or less, even more preferably 0.8 or less, and even more preferably 0.7 or less, in mass ratio. Each of these upper limits may be combined with any of the lower limits described above.

The meanings of the "amount of the ceramide AP", the "amount of the free ceramide other than the ceramide AP", and the "amount of the glucosylceramide" are as described above.

The ratio E is calculated based on the amount of the ceramide AP contained in the cacao derived component, the amount of the free ceramide contained in the cacao derived component, and the amount of the glucosylceramide contained in the cacao derived component. The cacao derived composition may contain or may not contain a ceramide AP other than the ceramide AP contained in the cacao derived component. In the case where the cacao derived composition contains a ceramide AP other than the ceramide AP contained in the cacao derived component, the amount of the ceramide AP other than the ceramide AP contained in the cacao derived component is not used when calculating the ratio E. The cacao derived composition may contain or may not contain a free ceramide other than the free ceramide contained in the cacao derived component. In the case where the cacao derived composition contains a free ceramide other than the free ceramide contained in the cacao derived component, the amount of the free ceramide other than the free ceramide contained in the cacao derived component is not used when calculating the ratio E. The cacao derived composition may contain or may not contain a glucosylceramide other than the glucosylceramide contained in the cacao derived component. In the case where the cacao derived composition contains a glucosylceramide other than the glucosylceramide contained in the cacao derived component, the amount of the glucosylceramide other than the glucosylceramide contained in the cacao derived component is not used when calculating the ratio E.

The amount of the ceramide AP, the amount of the free ceramide other than the ceramide AP, and the amount of the glucosylceramide can be measured by analyzing the cacao derived composition with a liquid chromatograph mass spectrometer (LC-MS/MS). The LC-MS/MS analysis can be performed according to the conditions and procedures described in the Examples.

### <Second embodiment>

In the cacao derived composition according to the second embodiment, the cacao derived component contains a free ceramide. In the second embodiment, the free ceramide contained in the cacao derived component may be referred to as "cacao derived free ceramide".

In the cacao derived composition according to the second embodiment, the cacao derived component may contain one type of free ceramide, or may contain two or more types of free ceramides. The cacao derived component can contain one or two or more free ceramides selected from a ceramide AP and a free ceramide other than the ceramide AP. The explanations about the ceramide AP and the free ceramide other than the ceramide AP in the first embodiment (see Features A and B) are also applicable to the second embodiment.

In the cacao derived composition according to the second embodiment, the cacao derived component may contain a glucosylceramide. The explanations about the glucosylceramide in the first embodiment (see Feature C) are also applicable to the second embodiment.

The cacao derived composition according to the second embodiment has a moisturizing action, a skin quality improving action, and a hair quality improving action.

The moisturizing action is demonstrated through, for example, an action of suppressing the amount of transepidermal water loss. However, the moisturizing action is not limited to a moisturizing action demonstrated through an action of suppressing the amount of transepidermal water loss. The skin quality improving action is demonstrated through, for example, a moisturizing action. However, the skin quality improving action is not limited to a skin quality improving action demonstrated through a moisturizing action. The target skin may be skin on any part of the body, and examples thereof include skin on the face, head, neck, back, shoulders, breasts, abdomen, arms, and legs. Skin quality improvement encompasses preventing, treating, or improving symptoms caused by a decline in the moisturizing function of skin (including aging symptoms), such as dry skin, rough skin, sagging, dullness, formation of spots or wrinkles, a decline in the flexibility or elasticity of skin, and a decline in the skin barrier function. The hair quality improving action is demonstrated through, for example, a moisturizing action. However, the hair quality improving action is not limited to a hair quality improving action demonstrated through a moisturizing action. The target hair may be hair that grows on any part of the body, and examples thereof include hair on the head, eyebrows, axillary hair, mustache and beard, and body hair. Hair quality improvement encompasses preventing, treating, or improving symptoms caused by a decline in the moisturizing function of hair (including aging symptoms), such as dryness of hair, roughness of hair, and a decline in the flexibility or elasticity of hair.

From the viewpoint of improving the moisturizing action, skin quality improving action, and hair quality improving action of the cacao derived composition, the cacao derived composition according to the second embodiment preferably has at least one of Feature A and Feature B, more preferably has at least Feature A among Feature A and Feature B, and even more preferably has both Feature A and Feature B. The explanations about Feature A and Feature B are as described above.

The cacao derived composition according to the second embodiment may have Feature C or Feature D. The explanations about Feature C and Feature D are as described above.

From the viewpoint of improving the moisturizing action, skin quality improving action, and hair quality improving action of the cacao derived composition, the amount of the cacao derived free ceramide in the cacao derived composition according to the second embodiment is preferably 1.2% by mass or more, more preferably 2.5% by mass or more, even more preferably 5.0% by mass or more, even more preferably 7.5% by mass or more, even more preferably 10.0% by mass or more, even more preferably 12.0% by mass or more, and even more preferably 24.0% by mass or more, based on the mass of the cacao derived composition. The upper limit is not particularly limited, and may be 99.9% by mass or less, 99.0% by mass or less, or 97.0% by mass or less. Each of these upper limits may be combined with any of the lower limits described above.

The "amount of the cacao derived free ceramide" means, in the case where the cacao derived component contains one type of free ceramide, the amount of the one type of free ceramide, and in the case where the cacao derived component contains two or more types of free ceramides, the total amount of the two or more types of free ceramides.

The amount of the free ceramide can be measured by analyzing the cacao derived composition by high performance liquid chromatography (HPLC). The HPLC analysis can be performed according to the conditions and procedures described in the Examples. In the case where HPLC analysis of a sample containing two or more types of free ceramides is performed according to the conditions and procedures described in the Examples, the peaks of the two or more types of free ceramides are overlapped and are detected as a single peak. The amount determined based on the peak area of the single peak detected and the calibration curve prepared using a ceramide AP as the preparation for the calibration curve is defined as the total amount of the two or more types of free ceramides. In other words, the total amount of the two or more types of free ceramides is determined as the ceramide AP equivalent amount (the amount in terms of the ceramide AP). The calibration curve is created from the peak areas obtained by using a ceramide AP (hydroxyphytoceramide_C24:0) (Avanti) as the preparation for the calibration curve and subjecting it to HPLC analysis at every predetermined concentration.

The cacao derived composition according to the second embodiment is preferably obtained by the following method.

### Provision of extraction raw material

An extraction raw material is provided. The extraction raw material preferably contains cacao bean shell. The extraction raw material may contain, in addition to cacao bean shell, one or two or more selected from cacao pod shell, cacao pulp, cacao bean nib, and cacao bean germ.

### Acquisition of extracted liquid

The extraction raw material is subjected to an extraction treatment with an extraction solvent to obtain an extracted liquid. The explanations about the extraction treatment with an extraction solvent are as described above.

### Acquisition of dried product

The extracted liquid is dried to obtain a dried product. Before drying, the extract may be concentrated. The concentration of the extract can be performed by, for example, using an evaporator. The drying of the extracted liquid can be performed by, for example, drying under reduced pressure.

The resultant dried product is preferably frozen and crushed to obtain a dried and crushed product. By freezing and crushing the dried product, the dried product can be homogenized. The freezing and crushing can be performed in the presence of liquid nitrogen.

### Purification of dried product or dried and crushed product

The dried product or dried and crushed product (preferably, dried and crushed product) is purified.

The purification of the dried product or dried and crushed product (preferably, dried and crushed product) is preferably performed by the following method 1, 2, 3, 4, or 5.

### <Method 1>

The method 1 includes the following treatments.

### Treatment A1: washing with water

The dried product or dried and crushed product is washed with water.

As the water, for example, purified water (for example, Milli-Q water) can be used. The amount of water used is, for example, 1 to 10 mL, preferably 2 to 4 mL, per 1 g of the dried product or dried and crushed product. The washing with water can be performed by, for example, shaking and admixing a mixture of the dried product or dried and crushed product with water.

The conditions for the shaking and admixing are, for example, as follows.
Temperature: for example, 4 to 50°C, preferably 20 to 30°C
Shaking speed: for example, 10 to 500 rpm, preferably 100 to 200 rpm
Time: for example, 1 to 30 minutes, preferably 10 to 20 minutes

### Treatment A2: centrifugation

After the treatment A1, the mixture is centrifuged.

The conditions for the centrifugation are, for example, as follows.
Temperature: for example, 1 to 40°C, preferably 2 to 5°C
Rotation speed: for example, 100 to 15000 rpm, preferably 2000 to 4000 rpm
Time: for example, 5 to 30 minutes, preferably 10 to 20 minutes

### Treatment A3: recovery of precipitates

After the treatment A2, the supernatant is removed and the precipitates are recovered.

### Treatment A4: repetition of treatments A1 to A3

After the treatment A3, the precipitates recovered are subjected to the treatments A1 to A3 (one cycle), for example, 1 to 10 times, preferably 2 to 4 times, and the precipitates are recovered. The treatment A4 may be omitted depending on the situation.

### Treatment A5: washing with ethanol

After the treatment A4 (or after the treatment A3 in the case where the treatment A4 is omitted), the precipitates recovered are washed with ethanol.

As the ethanol, for example, ethanol of 40 to 99.5% by volume, preferably 50 to 70% by volume, can be used. The amount of ethanol used is, for example, 1 to 10 mL, preferably 2 to 4 mL, per 1 g of the precipitates. The washing with ethanol can be performed by, for example, shaking and admixing a mixture of the precipitates with ethanol.

The conditions for the shaking and admixing are, for example, as follows.
Temperature: for example, 4 to 50°C, preferably 20 to 30°C
Shaking speed: for example, 10 to 500 rpm, preferably 100 to 200 rpm
Time: for example, 1 to 30 minutes, preferably 10 to 20 minutes

### Treatment A6: centrifugation

After the treatment A5, the mixture is centrifuged.

The conditions for the centrifugation are, for example, as follows.
Temperature: for example, 1 to 40°C, preferably 2 to 5°C
Rotation speed: for example, 100 to 15000 rpm, preferably 2000 to 4000 rpm
Time: for example, 1 to 30 minutes, preferably 10 to 20 minutes

### Treatment A7: recovery of precipitates

After the treatment A6, the supernatant is removed and the precipitates are recovered.

### Treatment A8: repetition of treatments A5 to A7

After the treatment A7, the precipitates recovered are subjected to the treatments A5 to A7 (one cycle), for example, 1 to 10 times, preferably 2 to 4 times, and the precipitates are recovered. The treatment A8 may be omitted depending on the situation.

### Treatment A9: drying

After the treatment A8 (or after the treatment A7 in the case where the treatment A8 is omitted), the precipitates recovered are dried.

The drying of the precipitates can be performed by, for example, drying under reduced pressure.

### Treatment A10: activated carbon treatment

After the treatment A9, the resultant dried product is treated with activated carbon.

The activated carbon treatment can be performed by, for example, contacting the dried product with activated carbon in ethanol. As the ethanol, for example, ethanol of 40 to 99.8% by volume, preferably 95 to 99.5% by volume, can be used. The amount of ethanol used is, for example, 1 to 20 mL, preferably 5 to 10 mL, per 1 g of the dried product. The contact between the dried product and activated carbon in ethanol can be performed by, for example, shaking and admixing a mixture of ethanol, the dried product, and activated carbon. The concentration of activated carbon in the mixture of ethanol, the dried product, and activated carbon is, for example, 0.01 to 1 w/v%, preferably 0.05 to 0.2 w/v%.

The conditions for the shaking and admixing are, for example, as follows.
Temperature: for example, 4 to 50°C, preferably 20 to 30°C
Shaking speed: for example, 10 to 500 rpm, preferably 100 to 200 rpm
Time: for example, 5 to 30 minutes, preferably 10 to 20 minutes

### Treatment A11: filtration

After the treatment A10, the mixture is filtered and the filtrate is recovered.

Examples of the filtration include natural filtration and suction filtration.

### Treatment A12: drying

After the treatment A11, the resultant filtrate is dried.

The drying of the filtrate can be performed by, for example, drying under reduced pressure.

The dried product obtained in the treatment A12 is an example of the cacao derived composition according to the second embodiment. The amount of the cacao derived free ceramide is preferably 2.0% by mass or more, more preferably 3.0% by mass or more, and even more preferably 4.0% by mass or more, based on the dry mass of the dried product. The upper limit is not particularly limited, and may be 99.0% by mass or less, 70.0% by mass or less, or 40.0% by mass or less. Each of these upper limits may be combined with any of the lower limits described above.

### <Method 2>

The method 2 includes the following treatments.

### Treatment B1: washing with water

The dried product or dried and crushed product is washed with water.

The treatment B1 can be performed in the same manner as the treatment A1. The explanations about the treatment A1 are also applicable to the treatment B1.

### Treatment B2: centrifugation

After the treatment B1, the mixture is centrifuged.

The treatment B2 can be performed in the same manner as the treatment A2. The explanations about the treatment A2 are also applicable to the treatment B2.

### Treatment B3: recovery of precipitates

After the treatment B2, the supernatant is removed and the precipitates are recovered.

### Treatment B4: repetition of treatments B1 to B3

After the treatment B3, the precipitates recovered are subjected to the treatments B1 to B3 (one cycle), for example, 1 to 10 times, preferably 2 to 4 times, and the precipitates are recovered. The treatment B4 may be omitted depending on the situation.

### Treatment B5: alkali treatment

After the treatment B4 (or after the treatment B3 in the case where the treatment B4 is omitted), the precipitates recovered are treated with an alkali.

As the alkali, for example, an aqueous solution of an alkali metal hydroxide can be used. Examples of the alkali metal hydroxide include NaOH and KOH, but it is preferably NaOH. The concentration of the aqueous solution of an alkali metal hydroxide is, for example, 0.01 to 0.8 M, preferably 0.3 to 0.5 M. The amount of the aqueous solution of an alkali metal hydroxide used is, for example, 1 to 10 mL, preferably 2 to 4 mL, per 1 g of the precipitates. The alkali treatment can be performed by, for example, shaking and admixing a mixture of the precipitates with the aqueous solution of an alkali metal hydroxide.

The conditions for the shaking and admixing are, for example, as follows.
Temperature: for example, 4 to 80°C, preferably 30 to 40°C
Shaking speed: for example, 10 to 500 rpm, preferably 100 to 200 rpm
Time: for example, 5 to 30 minutes, preferably 10 to 20 minutes

### Treatment B6: centrifugation

After the treatment B5, the mixture is centrifuged.

The conditions for the centrifugation are, for example, as follows.
Temperature: for example, 4 to 60°C, preferably 10 to 20°C
Rotation speed: for example, 100 to 15000 rpm, preferably 2000 to 4000 rpm
Time: for example, 1 to 30 minutes, preferably 10 to 20 minutes

### Treatment B7: recovery of precipitates

After the treatment B6, the supernatant is removed and the precipitates are recovered.

### Treatment B8: washing with water

After the treatment B7, the precipitates recovered are washed with water.

The washing with water can be performed by adding water to the precipitates and then inverting and admixing them. The washing with water may be performed in the same manner as the treatment A1.

### Treatment B9: centrifugation

After the treatment B8, the mixture is centrifuged.

The conditions for the centrifugation are, for example, as follows.
Temperature: for example, 4 to 60°C, preferably 10 to 20°C
Rotation speed: for example, 100 to 15000 rpm, preferably 2000 to 4000 rpm
Time: for example, 1 to 30 minutes, preferably 10 to 20 minutes

### Treatment B10: recovery of precipitates

After the treatment B9, the supernatant is removed and the precipitates are recovered.

### Treatment B11: repetition of treatments B8 to B10

After the treatment B10, the precipitates recovered are subjected to the treatments B8 to B10 (one cycle), for example, 1 to 10 times, preferably 2 to 4 times, and the precipitates are recovered. The treatment B11 may be omitted depending on the situation.

### Treatment B12: drying

After the treatment B11 (or after the treatment B10 in the case where the treatment B11 is omitted), the precipitates recovered are dried.

The drying of the precipitates can be performed by, for example, drying under reduced pressure.

The dried product obtained in the treatment B12 is an example of the cacao derived composition according to the second embodiment. The amount of the cacao derived free ceramide is preferably 1.2% by mass or more, more preferably 1.5% by mass or more, and even more preferably 2.0% by mass or more, based on the dry mass of the dried product. The upper limit is not particularly limited, and may be 99.0% by mass or less, 70.0% by mass or less, or 40.0% by mass or less. Each of these upper limits may be combined with any of the lower limits described above.

### <Method 3>

The method 3 includes the following treatments.

### Treatment C1: re-dissolution

The dried product or dried and crushed product is re-dissolved in ethanol.

As the ethanol, for example, ethanol of 40 to 99.8% by volume, preferably 95 to 99.5% by volume, can be used. The amount of ethanol used is, for example, 1 to 10 mL, preferably 4 to 6 mL, per 1 g of the dried product or dried and crushed product. The re-dissolution can be performed by, for example, subjecting a mixture of the dried product or dried and crushed product with ethanol to an ultrasonic treatment, if necessary, and then shaking and admixing the mixture.

The conditions for the ultrasonic treatment are, for example, as follows.
Temperature: for example, 4 to 60°C, preferably 20 to 40°C
Time: for example, 1 to 30 minutes, preferably 10 to 20 minutes

The conditions for the shaking and admixing are, for example, as follows.
Temperature: for example, 4 to 80°C, preferably 30 to 50°C
Shaking speed: for example, 10 to 500 rpm, preferably 100 to 200 rpm
Time: for example, 5 to 30 minutes, preferably 10 to 20 minutes

### Treatment C2: centrifugation

After the treatment C1, the mixture is centrifuged.

The conditions for the centrifugation are, for example, as follows.
Temperature: for example, 4 to 50°C, preferably 20 to 30°C
Rotation speed: for example, 100 to 15000 rpm, preferably 2000 to 4000 rpm
Time: for example, 1 to 30 minutes, preferably 10 to 20 minutes

### Treatment C3: recovery of precipitates

After the treatment C2, the supernatant and the precipitates are separated and recovered.

### Treatment C4: repetition of treatments C1 to C3

After the treatment C3, the precipitates recovered are subjected to the treatments C1 to C3 (one cycle), for example, 1 to 10 times, preferably 2 to 4 times, and the supernatant is recovered. The supernatant recovered in the treatment C3 is combined with the supernatant recovered in the treatment C4, and the combined supernatant is used in the next treatment. The treatment C4 may be omitted depending on the situation.

### Treatment C5: activated carbon treatment

After the treatment C4 (or after the treatment C3 in the case where the treatment C4 is omitted), the resultant supernatant is treated with activated carbon.

The activated carbon treatment can be performed by, for example, contacting the supernatant with activated carbon. The contact between the supernatant and activated carbon can be performed by, for example, shaking and admixing a mixture of the supernatant with activated carbon. The concentration of activated carbon in the mixture of the supernatant with activated carbon is, for example, 0.01 to 1 w/v%, preferably 0.05 to 0.2 w/v%.

The conditions for the shaking and admixing are, for example, as follows.
Temperature: for example, 4 to 80°C, preferably 30 to 50°C
Shaking speed: for example, 10 to 500 rpm, preferably 100 to 200 rpm
Time: for example, 5 to 30 minutes, preferably 10 to 20 minutes

### Treatment C6: filtration

After the treatment C5, the mixture is filtered and the filtrate is recovered.

Examples of the filtration include natural filtration and suction filtration.

In the case where the filtration is performed using filter paper, it is preferable to wash the filter paper used for the filtration with ethanol and to use the filtrate recovered (ethanol used for the washing) in the next step, together with the filtrate recovered earlier. As the ethanol, for example, ethanol of 40 to 99.8% by volume, preferably 95 to 99.5% by volume, can be used. The amount of ethanol used is, for example, 1 to 10 mL, preferably 4 to 6 mL.

### Treatment C7: deposition

After the treatment C6, water is added to the filtrate recovered.

As the water, for example, purified water (for example, Milli-Q water) can be used. The amount of water added is, for example, 0.1 to 10 times, preferably 0.5 to 2 times, the volume of the filtrate. The addition of water causes the free ceramide to be deposited.

### Treatment C8: centrifugation

After the treatment C7, the mixture is centrifuged.

The conditions for the centrifugation are, for example, as follows.
Temperature: for example, 1 to 40°C, preferably 2 to 5°C
Rotation speed: for example, 100 to 15000 rpm, preferably 4000 to 6000 rpm
Time: for example, 1 to 30 minutes, preferably 10 to 20 minutes

### Treatment C9: recovery of precipitates

After the treatment C8, the supernatant is removed and the precipitates are recovered.

### Treatment C10: washing with hexane

After the treatment C9, the precipitates recovered are washed with hexane.

The amount of hexane used is, for example, 0.1 to 5 mL, preferably 1 to 2 mL, per 1 g of the precipitates. The washing with hexane can be performed by, for example, stirring a mixture of the precipitates with hexane. The stirring can be performed by, for example, using a vortex mixer. The stirring time is, for example, 0.1 to 5 minutes, preferably 1 to 2 minutes.

### Treatment C11: centrifugation

After the treatment C10, the mixture is centrifuged.

The conditions for the centrifugation are, for example, as follows.
Temperature: for example, 1 to 40°C, preferably 2 to 5°C
Rotation speed: for example, 100 to 20000 rpm, preferably 12000 to 16000 rpm
Time: for example, 5 to 30 minutes, preferably 10 to 20 minutes

### Treatment C12: recovery of precipitates

After the treatment C11, the supernatant is removed and the precipitates are recovered.

### Treatment C13: repetition of treatments C10 to C12

After the treatment C12, the precipitates recovered are subjected to the treatments C10 to C12 (one cycle), for example, 1 to 10 times, preferably 2 to 4 times, and the precipitates are recovered. The treatment C13 may be omitted depending on the situation.

### Treatment C14: drying

After the treatment C13 (or after the treatment C12 in the case where the treatment C13 is omitted), the precipitates recovered are dried.

The drying of the precipitates can be performed by, for example, drying under reduced pressure.

The dried product obtained in the treatment C14 is an example of the cacao derived composition according to the second embodiment. The amount of the cacao derived free ceramide is preferably 5.0% by mass or more, more preferably 15.0% by mass or more, and even more preferably 25.0% by mass or more, based on the dry mass of the dried product. The upper limit is not particularly limited, and may be 99.0% by mass or less, 70.0% by mass or less, or 40.0% by mass or less. Each of these upper limits may be combined with any of the lower limits described above.

### <Method 4>

The method 4 is an improved version of the method 1. The method 4 includes the following treatments.

### Treatment D1: washing with water

The dried product or dried and crushed product is washed with water.

The treatment D1 can be performed in the same manner as the treatment A1. The explanations about the treatment A1 are also applicable to the treatment D1, unless otherwise specified. The amount of water used is, for example, 1 to 10 mL, preferably 3 to 5 mL, per 1 g of the dried product or dried and crushed product.

### Treatment D2: centrifugation

After the treatment D1, the mixture is centrifuged.

The treatment D2 can be performed in the same manner as the treatment A2. The explanations about the treatment A2 are also applicable to the treatment D2.

### Treatment D3: recovery of precipitates

After the treatment D2, the supernatant is removed and the precipitates are recovered.

### Treatment D4: repetition of treatments D1 to D3

After the treatment D3, the precipitates recovered are subjected to the treatments D1 to D3 (one cycle), for example, 1 to 10 times, preferably 2 to 4 times, and the precipitates are recovered. The treatment D4 may be omitted depending on the situation.

### Treatment D5: washing with ethanol

After the treatment D4 (or after the treatment D3 in the case where the treatment D4 is omitted), the precipitates recovered are washed with ethanol.

The treatment D5 can be performed in the same manner as the treatment A5. The explanations about the treatment A5 are also applicable to the treatment D5, unless otherwise specified. The amount of ethanol used is, for example, 1 to 10 mL, preferably 3 to 5 mL, per 1 g of the precipitates.

### Treatment D6: centrifugation

After the treatment D5, the mixture is centrifuged.

The treatment D6 can be performed in the same manner as the treatment A6. The explanations about the treatment A6 are also applicable to the treatment D6.

### Treatment D7: recovery of precipitates

After the treatment D6, the supernatant is removed and the precipitates are recovered.

### Treatment D8: repetition of treatments D5 to D7

After the treatment D7, the precipitates recovered are subjected to the treatments D5 to D7 (one cycle), for example, 1 to 10 times, preferably 2 to 4 times, and the precipitates are recovered. The treatment D8 may be omitted depending on the situation.

### Treatment D9: drying under reduced pressure

After the treatment D8 (or after the treatment D7 in the case where the treatment D8 is omitted), the precipitates recovered are dried.

### Treatment D10: activated carbon treatment

After the treatment D9, the resultant dried product is treated with activated carbon.

The treatment D10 can be performed in the same manner as the treatment A10. The explanations about the treatment A10 are also applicable to the treatment D10, unless otherwise specified. The amount of ethanol used is, for example, 1 to 20 mL, preferably 5 to 10 mL, per 1 g of the dried product. The concentration of activated carbon in the mixture of ethanol, the dried product, and activated carbon is, for example, 0.01 to 1 w/v%, preferably 0.05 to 0.2 w/v%.

### Treatment D11: filtration

After the treatment D10, the mixture is filtered, and the filtrate and the residue are recovered.

Examples of the filtration include natural filtration and suction filtration.

### Treatment D12: extraction

The residue obtained in the treatment D11 is subjected to extraction with ethanol, the resultant extracted liquid is filtered, and the filtrate is recovered.

The extraction treatment can be performed by, for example, bringing the residue into contact with ethanol. As the ethanol, for example, ethanol of 40 to 99.8% by volume, preferably 95 to 99.5% by volume, can be used. The amount of ethanol used is, for example, 1 to 100 mL, preferably 10 to 40 mL, per 1 g of the residue. Examples of the filtration include natural filtration and suction filtration.

### Treatment D13: drying under reduced pressure

The filtrate recovered in the treatment D11 is combined with the filtrate recovered in the treatment D12, and the combined filtrate is dried.

The drying of the filtrate can be performed by, for example, drying under reduced pressure.

The dried product obtained in the treatment D13 is an example of the cacao derived composition according to the second embodiment. The amount of the cacao derived free ceramide is preferably 2.8% by mass or more, more preferably 3.5% by mass or more, and even more preferably 4.2% by mass or more, based on the dry mass of the dried product. The upper limit is not particularly limited, and may be 99.0% by mass or less, 70.0% by mass or less, or 40.0% by mass or less. Each of these upper limits may be combined with any of the lower limits described above.

### <Method 5>

The method 5 is an improved version of the method 3. The method 5 includes the following treatments.

### Treatment E1: re-dissolution

The dried product or dried and crushed product is re-dissolved in ethanol.

The treatment E1 can be performed in the same manner as the treatment C1. The explanations about the treatment C1 are also applicable to the treatment E1, unless otherwise specified. The amount of ethanol used is, for example, 1 to 20 mL, preferably 3 to 8 mL, per 1 g of the dried product or dried and crushed product.

### Treatment E2: centrifugation

After the treatment E1, the mixture is centrifuged.

The treatment E2 can be performed in the same manner as the treatment C2. The explanations about the treatment C2 are also applicable to the treatment E2.

### Treatment E3: recovery of precipitates

After the treatment E2, the supernatant and the precipitates are separated and recovered.

### Treatment E4: repetition of treatments E1 to E3

After the treatment E3, the precipitates recovered are subjected to the treatments E1 to E3 (one cycle), for example, 1 to 10 times, preferably 2 to 4 times, and the supernatant is recovered. The supernatant recovered in the treatment E3 is combined with the supernatant recovered in the treatment E4, and the combined supernatant is used in the next treatment. The treatment E4 may be omitted depending on the situation.

### Treatment E5: activated carbon treatment

After the treatment E4 (or after the treatment E3 in the case where the treatment E4 is omitted), the resultant supernatant is treated with activated carbon.

The treatment E5 can be performed in the same manner as the treatment C5. The explanations about the treatment C5 are also applicable to the treatment E5.

### Treatment E6: filtration

After the treatment E5, the mixture is filtered and the filtrate is recovered.

The treatment E6 can be performed in the same manner as the treatment C6. The explanations about the treatment C6 are also applicable to the treatment E6.

### Treatment E7: deposition

After the treatment E6, water is added to the filtrate recovered.

The treatment E7 can be performed in the same manner as the treatment C7. The explanations about the treatment C7 are also applicable to the treatment E7.

### Treatment E8: centrifugation

After the treatment E7, the mixture is centrifuged.

The treatment E8 can be performed in the same manner as the treatment C8. The explanations about the treatment C8 are also applicable to the treatment E8.

### Treatment E9: recovery of precipitates

After the treatment E8, the supernatant is removed and the precipitates are recovered.

### Treatment E10: drying under reduced pressure

After the treatment C9, the precipitates recovered are dried.

The drying of the precipitates can be performed by, for example, drying under reduced pressure.

### Treatment E11: washing with hexane

After the treatment E10, the resultant dried product is washed with hexane.

The treatment E11 may be performed two or more times (for example, 1 to 10 times, preferably 2 to 4 times). The treatment E11 can be performed in the same manner as the treatment C10. The explanations about the treatment C10 are also applicable to the treatment E11.

### Treatment E12: centrifugation

After the treatment E11, the mixture is centrifuged.

The treatment E12 can be performed in the same manner as the treatment C11. The explanations about the treatment C11 are also applicable to the treatment E12.

### Treatment E13: recovery of precipitates

After the treatment E12, the supernatant is removed and the precipitates are recovered.

### Treatment E14: drying under reduced pressure

After the treatment E13, the precipitates recovered are dried.

The drying of the precipitates can be performed by, for example, drying under reduced pressure.

The dried product obtained in the treatment E14 is an example of the cacao derived composition according to the second embodiment. The amount of the cacao derived free ceramide is preferably 13% by mass or more, more preferably 14% by mass or more, and even more preferably 15% by mass or more, based on the dry mass of the dried product. The upper limit is not particularly limited, and may be 99.0% by mass or less, 70.0% by mass or less, or 40.0% by mass or less. Each of these upper limits may be combined with any of the lower limits described above.

### <Third embodiment>

In the cacao derived composition according to the third embodiment, the cacao derived component contains an extract of cacao bean shell, and the extract of cacao bean shell contains a free ceramide. In the third embodiment, the free ceramide contained in the extract of cacao bean shell may be referred to as "cacao derived free ceramide".

In the cacao derived composition according to the third embodiment, the extract of cacao bean shell may contain one type of free ceramide, or may contain two or more types of free ceramides. The extract of cacao bean shell can contain one or two or more free ceramides selected from a ceramide AP and a free ceramide other than the ceramide AP. The explanations about the ceramide AP and the free ceramide other than the ceramide AP in the first embodiment (see Features A and B) are also applicable to the third embodiment.

In the cacao derived composition according to the third embodiment, the extract of cacao bean shell may contain a glucosylceramide. The explanations about the glucosylceramide in the first embodiment (see Feature C) are also applicable to the third embodiment.

The cacao derived composition according to the third embodiment has a moisturizing action, a skin quality improving action, and a hair quality improving action.

The moisturizing action is demonstrated through, for example, an action of suppressing the amount of transepidermal water loss. However, the moisturizing action is not limited to a moisturizing action demonstrated through an action of suppressing the amount of transepidermal water loss. The skin quality improving action is demonstrated through, for example, a moisturizing action. However, the skin quality improving action is not limited to a skin quality improving action demonstrated through a moisturizing action. The target skin may be skin on any part of the body, and examples thereof include skin on the face, head, neck, back, shoulders, breasts, abdomen, arms, and legs. Skin quality improvement encompasses preventing, treating, or improving symptoms caused by a decline in the moisturizing function of skin (including aging symptoms), such as dry skin, rough skin, sagging, dullness, formation of spots or wrinkles, a decline in the flexibility or elasticity of skin, and a decline in the skin barrier function. The hair quality improving action is demonstrated through, for example, a moisturizing action. However, the hair quality improving action is not limited to a hair quality improving action demonstrated through a moisturizing action. The target hair may be hair that grows on any part of the body, and examples thereof include hair on the head, eyebrows, axillary hair, mustache and beard, and body hair. Hair quality improvement encompasses preventing, treating, or improving symptoms caused by a decline in the moisturizing function of hair (including aging symptoms), such as dryness of hair, roughness of hair, and a decline in the flexibility or elasticity of hair.

From the viewpoint of improving the moisturizing action, skin quality improving action, and hair quality improving action of the cacao derived composition, the cacao derived composition according to the third embodiment preferably has at least one of Feature A and Feature B, more preferably has at least Feature A among Feature A and Feature B, and more preferably has both Feature A and Feature B. The explanations about Feature A and Feature B are as described above.

The cacao derived composition according to the third embodiment may have Feature C or Feature D. The explanations about Feature C and Feature D are as described above.

In the cacao derived composition according to the third embodiment, the extract of cacao bean shell is preferably obtained by a method including the following steps:
(a) a step of winnowing a crushed product of cacao bean to separate it into a cacao bean nib fraction and a cacao bean shell fraction and obtaining the cacao bean shell fraction as a first cacao derived raw material;
(b) optionally, a step of sorting out, from the first cacao derived raw material, a raw material whose size does not allow passing through a 16 mesh to obtain a second cacao derived raw material; and
(c) a step of subjecting the first or second cacao derived raw material to an extraction treatment with an extraction solvent to obtain the extract of cacao bean shell.

As a result, the amount of the free ceramide contained in the extract of cacao bean shell can be increased.

Hereinafter, each step will be described.

### Step (a)

The step (a) is a step of winnowing a crushed product of cacao bean to separate it into a cacao bean nib fraction and a cacao bean shell fraction and obtaining the cacao bean shell fraction as a first cacao derived raw material.

The crushed product of cacao bean can be obtained by crushing a cacao bean. The crushing can be performed by, for example, using a mill. The crushed product of cacao bean contains cacao bean shell and cacao bean nib. The crushed product of cacao bean may contain cacao bean germ.

The winnowing of the crushed product of cacao bean is a treatment in which cacao bean nib and cacao bean shell are sorted out and separated by utilizing the shape difference between them. The winnowing of the crushed product of cacao bean can be performed according to conventional methods using a known winnowing machine (a device called winnower).

The winnowing of the crushed product of cacao bean can separate the crushed product of cacao bean into a cacao bean nib fraction and a cacao bean shell fraction, and the cacao bean shell fraction separated can be obtained as a first cacao derived raw material.

The cacao bean shell fraction is a fraction that contains a larger amount of cacao bean shell than the cacao bean nib fraction. The cacao bean shell fraction may contain cacao bean germ in addition to cacao bean shell.

The amount of cacao bean nib in the cacao bean shell fraction (the first cacao derived raw material) is preferably 20% by mass or less, more preferably 15% by mass or less, and even more preferably 10% by mass or less, based on the mass of the cacao bean shell fraction (the first cacao derived raw material). The lower limit may be 0% by mass, or may be more than 0% by mass. For example, the lower limit may be 1% by mass or more, 3% by mass or more, or 5% by mass or more. Each of these lower limits may be combined with any of the upper limits described above.

The free ceramide is contained in a larger amount in cacao bean shell than in cacao bean nib. Accordingly, by performing an extraction treatment using the first cacao derived raw material as the extraction raw material in the step (c), the amount of the free ceramide contained in the extract of cacao bean shell can be increased.

### Step (b)

The step (b) is, optionally, a step of sorting out, from the first cacao derived raw material, a raw material whose size does not allow passing through a 16 mesh to obtain a second cacao derived raw material.

The step (b) is an optional step, and may be performed or may not be performed, but it is preferably performed. By performing the step (b), the amount of the free ceramide contained in the extract of cacao bean shell can be further increased.

The sorting out of a raw material whose size does not allow passing through a 16 mesh from the first cacao derived raw material can be performed by sieving the first cacao derived raw material using a 16 mesh sieve or a sieve with a larger aperture than a 16 mesh. The raw material whose size does not allow passing through a 16 mesh can be obtained as a plus sieve fraction. In the 16 mesh sieve, the aperture varies depending on the wire diameter, but is, for example, 0.888 to 1.388 mm (cited from a catalog of commercially available 16 mesh wire gauze). In the 16 mesh sieve, the method of weaving is, for example, plain weave, the wire diameter ϕ is, for example, 0.20 to 0.70 mm, and the aperture ratio is, for example, 31.25 to 76.39% (all cited from a catalog of commercially available 16 mesh wire gauze). As the sieve with a larger aperture than a 16 mesh, for example, a 12 to 14 mesh sieve can be used.

The raw material sorted out whose size does not allow passing through a 16 mesh is a second cacao derived raw material. The second cacao derived raw material contains cacao bean shell.

The sorting out of a raw material whose size does not allow passing through a 16 mesh from the first cacao derived raw material can remove a fraction with a smaller particle diameter from the first cacao derived raw material. Most of the cacao bean nib contained in the first cacao derived raw material is contained in the fraction with a smaller particle diameter. Accordingly, the sorting out of a raw material whose size does not allow passing through a 16 mesh from the first cacao derived raw material can remove the cacao bean nib contained in the first cacao derived raw material, and can increase the amount of the free ceramide contained in the second cacao derived raw material. Accordingly, by performing an extraction treatment using the second cacao derived raw material as the extraction raw material in the step (c), the amount of the free ceramide contained in the extract of cacao bean shell can be increased.

The mass percentage of the amount of the free ceramide contained in the second cacao derived raw material to the amount of the free ceramide contained in the first cacao derived raw material is, for example, 105% by mass or more, preferably 115% by mass or more, and more preferably 125% by mass or more. The upper limit is not particularly limited, and for example, may be 300% by mass or less, 200% by mass or less, or 150% by mass or less. Each of these upper limits may be combined with any of the lower limits described above.

The "amount of the free ceramide contained in the first cacao derived raw material" means, in the case where the first cacao derived raw material contains one type of free ceramide, the amount of the one type of free ceramide, and in the case where the first cacao derived raw material contains two or more types of free ceramides, the total amount of the two or more types of free ceramides.

The "amount of the free ceramide contained in the second cacao derived raw material" means, in the case where the second cacao derived raw material contains one type of free ceramide, the amount of the one type of free ceramide, and in the case where the second cacao derived raw material contains two or more types of free ceramides, the total amount of the two or more types of free ceramides.

The amount of the free ceramide can be measured by analyzing the cacao derived composition by high performance liquid chromatography (HPLC). The HPLC analysis can be performed according to the conditions and procedures described in the Examples. In the case where HPLC analysis of a sample containing two or more types of free ceramides is performed according to the conditions and procedures described in the Examples, the peaks of the two or more types of free ceramides are overlapped and are detected as a single peak. The amount determined based on the peak area of the single peak detected and the calibration curve prepared using a ceramide AP as the preparation for the calibration curve is defined as the total amount of the two or more types of free ceramides. In other words, the total amount of the two or more types of free ceramides is determined as the ceramide AP equivalent amount (the amount in terms of the ceramide AP). The calibration curve is created from the peak areas obtained by using a ceramide AP (hydroxyphytoceramide_C24:0) (Avanti) as the preparation for the calibration curve and subjecting it to HPLC analysis at every predetermined concentration.

From the viewpoint of further increasing the amount of the free ceramide contained in the second cacao derived raw material, in the step (b), it is preferable to sort out from the first cacao derived raw material a raw material whose size does not allow passing through a 14 mesh, and it is preferable to sort out from the first cacao derived raw material a raw material whose size does not allow passing through a 12 mesh.

The sorting out of a raw material whose size does not allow passing through a 14 mesh from the first cacao derived raw material can be performed by sieving the first cacao derived raw material using a 14 mesh sieve or a sieve with a larger aperture than a 14 mesh. The raw material whose size does not allow passing through a 14 mesh can be obtained as a plus sieve fraction. In the 14 mesh sieve, the aperture varies depending on the wire diameter, but is, for example, 0.914 to 1.594 mm (cited from a catalog of commercially available 14 mesh wire gauze). In the 14 mesh sieve, the method of weaving is, for example, plain weave, the wire diameter ϕ is, for example, 0.22 to 0.90 mm, and the aperture ratio is, for example, 25.40 to 77.22% (all cited from a catalog of commercially available 14 mesh wire gauze). As the sieve with a larger aperture than a 14 mesh, for example, a 10 to 12 mesh sieve can be used.

The sorting out of a raw material whose size does not allow passing through a 12 mesh from the first cacao derived raw material can be performed by sieving the first cacao derived raw material using a 12 mesh sieve or a sieve with a larger aperture than a 12 mesh. The raw material whose size does not allow passing through a 12 mesh can be obtained as a plus sieve fraction. In the 12 mesh sieve, the aperture varies depending on the wire diameter, but is, for example, 1.217 to 1.867 mm (cited from a catalog of commercially available 12 mesh wire gauze). In the 12 mesh sieve, the method of weaving is, for example, plain weave, the wire diameter ϕ is, for example, 0.25 to 0.90 mm, and the aperture ratio is, for example, 33.04 to 77.77% (all cited from a catalog of commercially available 12 mesh wire gauze). As the sieve with a larger aperture than a 12 mesh, for example, a 8 to 10 mesh sieve can be used.

### Step (c)

The step (c) is a step of subjecting the first or second cacao derived raw material to an extraction treatment with an extraction solvent to obtain the extract of cacao bean shell.

By performing an extraction treatment using the first cacao derived raw material as the extraction raw material, the amount of the free ceramide contained in the extract of cacao bean shell can be increased. By performing an extraction treatment using the second cacao derived raw material as the extraction raw material, the amount of the free ceramide contained in the extract of cacao bean shell can be further increased.

The explanations about the extraction treatment with an extraction solvent are as described above.

It is preferable that in the step (c), the first or second cacao derived raw material is subjected to extraction with an extraction solvent, and the resultant extract is then purified to obtain the extract of cacao bean shell. The extract obtained by subjecting the first or second cacao derived raw material to extraction with an extraction solvent (that is, the extract to be purified) may be in any of the following forms: extracted liquid, diluted liquid, concentrated liquid, and dried product. The purification is preferably performed by the method 1, 2, 3, 4, or 5 described above.

The step (c) can be performed, for example, as follows.

### Acquisition of extracted liquid

The first or second cacao derived raw material is subjected to an extraction treatment with an extraction solvent to obtain an extracted liquid. The explanations about the extraction treatment with an extraction solvent are as described above.

### Acquisition of dried product

The extracted liquid is dried to obtain a dried product. Before drying, the extract may be concentrated. The concentration of the extract can be performed by, for example, using an evaporator. The drying of the extracted liquid can be performed by, for example, drying under reduced pressure.

The resultant dried product is preferably frozen and crushed to obtain a dried and crushed product. By freezing and crushing the dried product, the dried product can be homogenized. The freezing and crushing can be performed in the presence of liquid nitrogen.

### Purification of dried product or dried and crushed product

The dried product or dried and crushed product (preferably, dried and crushed product) is purified.

The purification of the dried product or dried and crushed product (preferably, dried and crushed product) is preferably performed by the method 1, 2, 3, 4, or 5 described above.

### <<Moisturizing agent>>

The present invention relates to a moisturizing agent containing the cacao derived composition. The explanations about the cacao derived composition are as described above.

In one embodiment, the moisturizing agent contains the cacao derived composition according to the first embodiment. The explanations about the cacao derived composition according to the first embodiment are as described above.

In another embodiment, the moisturizing agent contains the cacao derived composition according to the second embodiment. The explanations about the cacao derived composition according to the second embodiment are as described above.

In still another embodiment, the moisturizing agent contains the cacao derived composition according to the third embodiment. The explanations about the cacao derived composition according to the third embodiment are as described above.

The moisturizing agent can be used by applying it to the skin and/or hair of a subject, and can demonstrate a moisturizing action through the moisturizing action of the cacao derived composition. Accordingly, the moisturizing agent is useful for a skin moisturizing application and/or a hair moisturizing application. The subject is preferably a mammal, more preferably a human. The skin of the subject may be skin on any part of the body of the subject, and examples thereof include skin on the face, head, neck, back, shoulders, breasts, abdomen, arms, and legs. The hair of the subject may be hair that grows on any part of the body of the subject, and examples thereof include hair on the head, eyebrows, axillary hair, mustache and beard, and body hair.

The moisturizing agent may be composed of the cacao derived composition, or may be formed by formulating the cacao derived composition. The cacao derived composition can be formulated into a dosage form, such as liquid, suspension, emulsion, lotion, gel, cream, tablet, pill, powder, granule, and capsule, according to conventional methods, using pharmaceutically acceptable additives. The additives can be selected from, for example, excipients, binders, disintegrants, lubricants, stabilizers, taste and odor correctives, emulsifiers, and others.

The moisturizing agent can be used by, for example, applying it to the skin and/or hair as an ointment, a liquid for external use, a patch, or the like, and it can also be used by blending it into other compositions (for example, cosmetics for skin, pharmaceutical compositions, food compositions, and others).

The moisturizing agent may contain the cacao derived composition as the sole moisturizing component, or may contain other moisturizing components.

The amount of the cacao derived composition blended can be adjusted as appropriate depending on the dosage form of the moisturizing agent. The amount of the cacao derived composition blended is, for example, 0.01 to 100% by mass, preferably 0.1 to 99% by mass, based on the mass of the moisturizing agent.

In the case where the moisturizing agent contains the cacao derived composition according to the first embodiment, the amount of the ceramide AP derived from the cacao derived composition according to the first embodiment is, for example, 0.001% by mass or more, preferably 0.01% by mass or more, more preferably 0.03% by mass or more, even more preferably 0.05% by mass or more, and even more preferably 0.1% by mass or more, based on the mass of the moisturizing agent. The upper limit is not particularly limited, and for example, may be 10.0% by mass or less, 5.0% by mass or less, or 1.0% by mass or less. Each of these upper limits may be combined with any of the lower limits described above.

In the case where the moisturizing agent contains the cacao derived composition according to the second embodiment, the amount of the free ceramide derived from the cacao derived composition according to the second embodiment is, for example, 0.001% by mass or more, preferably 0.01% by mass or more, more preferably 0.03% by mass or more, even more preferably 0.05% by mass or more, and even more preferably 0.1% by mass or more, based on the mass of the moisturizing agent. The upper limit is not particularly limited, and for example, may be 10.0% by mass or less, 5.0% by mass or less, or 1.0% by mass or less. Each of these upper limits may be combined with any of the lower limits described above.

In the case where the moisturizing agent contains the cacao derived composition according to the third embodiment, the amount of the free ceramide derived from the cacao derived composition according to the third embodiment is, for example, 0.001% by mass or more, preferably 0.01% by mass or more, more preferably 0.03% by mass or more, even more preferably 0.05% by mass or more, and even more preferably 0.1% by mass or more, based on the mass of the moisturizing agent. The upper limit is not particularly limited, and for example, may be 10.0% by mass or less, 5.0% by mass or less, or 1.0% by mass or less. Each of these upper limits may be combined with any of the lower limits described above.

The "amount of the ceramide AP" means, in the case where the cacao derived composition according to the first embodiment contains one type of ceramide AP, the amount of the one type of ceramide AP, and in the case where the cacao derived composition according to the first embodiment contains two or more types of ceramide APs, the total amount of the two or more types of ceramide APs. The amount of the ceramide AP can be measured by LC-MS/MS analysis. The LC-MS/MS analysis can be performed according to the conditions and procedures described in the Examples.

The "amount of the free ceramide" means, in the case where the cacao derived composition according to the second or third embodiment contains one type of free ceramide, the amount of the one type of free ceramide, and in the case where the cacao derived composition according to the second or third embodiment contains two or more types of free ceramides, the total amount of the two or more types of free ceramides. The amount of the free ceramide can be measured by HPLC analysis. The HPLC analysis can be performed according to the conditions and procedures described in the Examples.

### <<Skin quality improving agent>>

The present invention relates to a skin quality improving agent containing the cacao derived composition. The explanations about the cacao derived composition are as described above.

In one embodiment, the skin quality improving agent contains the cacao derived composition according to the first embodiment. The explanations about the cacao derived composition according to the first embodiment are as described above.

In another embodiment, the skin quality improving agent contains the cacao derived composition according to the second embodiment. The explanations about the cacao derived composition according to the second embodiment are as described above.

In still another embodiment, the skin quality improving agent contains the cacao derived composition according to the third embodiment. The explanations about the cacao derived composition according to the third embodiment are as described above.

The skin quality improving agent can be used by applying it to the skin of a subject, and can demonstrate a skin quality improving action through the skin quality improving action of the cacao derived composition. Accordingly, the skin quality improving agent is useful for a skin quality improving application. The subject is preferably a mammal, more preferably a human. The skin of the subject may be skin on any part of the body of the subject, and examples thereof include skin on the face, head, neck, back, shoulders, breasts, abdomen, arms, and legs. The skin quality improving application encompasses preventing, treating, or improving symptoms caused by a decline in the moisturizing function of skin (including aging symptoms), such as dry skin, rough skin, sagging, dullness, formation of spots or wrinkles, a decline in the flexibility or elasticity of skin, and a decline in the skin barrier function.

The skin quality improving agent may be composed of the cacao derived composition, or may be formed by formulating the cacao derived composition. The cacao derived composition can be formulated into a dosage form, such as liquid, suspension, emulsion, lotion, gel, cream, tablet, pill, powder, granule, and capsule, according to conventional methods, using pharmaceutically acceptable additives. The additives can be selected from, for example, excipients, binders, disintegrants, lubricants, stabilizers, taste and odor correctives, emulsifiers, and others.

The skin quality improving agent can be used by, for example, applying it to the skin as an ointment, a liquid for external use, a patch, or the like, and it can also be used by blending it into other compositions (for example, cosmetics for skin, pharmaceutical compositions, food compositions, and others).

The skin quality improving agent may contain the cacao derived composition as the sole skin quality improving component, or may contain other skin quality improving components.

The amount of the cacao derived composition blended can be adjusted as appropriate depending on the dosage form of the skin quality improving agent. The amount of the cacao derived composition blended is, for example, 0.01 to 100% by mass, preferably 0.1 to 99% by mass, based on the mass of the skin quality improving agent.

In the case where the skin quality improving agent contains the cacao derived composition according to the first embodiment, the amount of the ceramide AP derived from the cacao derived composition according to the first embodiment is, for example, 0.001% by mass or more, preferably 0.01% by mass or more, more preferably 0.03% by mass or more, even more preferably 0.05% by mass or more, and even more preferably 0.1% by mass or more, based on the mass of the skin quality improving agent. The upper limit is not particularly limited, and for example, may be 10.0% by mass or less, 5.0% by mass or less, or 1.0% by mass or less. Each of these upper limits may be combined with any of the lower limits described above.

In the case where the skin quality improving agent contains the cacao derived composition according to the second embodiment, the amount of the free ceramide derived from the cacao derived composition according to the second embodiment is, for example, 0.001% by mass or more, preferably 0.01% by mass or more, more preferably 0.03% by mass or more, even more preferably 0.05% by mass or more, and even more preferably 0.1% by mass or more, based on the mass of the skin quality improving agent. The upper limit is not particularly limited, and for example, may be 10.0% by mass or less, 5.0% by mass or less, or 1.0% by mass or less. Each of these upper limits may be combined with any of the lower limits described above.

In the case where the skin quality improving agent contains the cacao derived composition according to the third embodiment, the amount of the free ceramide derived from the cacao derived composition according to the third embodiment is, for example, 0.001% by mass or more, preferably 0.01% by mass or more, more preferably 0.03% by mass or more, even more preferably 0.05% by mass or more, and even more preferably 0.1% by mass or more, based on the mass of the skin quality improving agent. The upper limit is not particularly limited, and for example, may be 10.0% by mass or less, 5.0% by mass or less, or 1.0% by mass or less. Each of these upper limits may be combined with any of the lower limits described above.

The "amount of the ceramide AP" means, in the case where the cacao derived composition according to the first embodiment contains one type of ceramide AP, the amount of the one type of ceramide AP, and in the case where the cacao derived composition according to the first embodiment contains two or more types of ceramide APs, the total amount of the two or more types of ceramide APs. The amount of the ceramide AP can be measured by LC-MS/MS analysis. The LC-MS/MS analysis can be performed according to the conditions and procedures described in the Examples.

The "amount of the free ceramide" means, in the case where the cacao derived composition according to the second or third embodiment contains one type of free ceramide, the amount of the one type of free ceramide, and in the case where the cacao derived composition according to the second or third embodiment contains two or more types of free ceramides, the total amount of the two or more types of free ceramides. The amount of the free ceramide can be measured by HPLC analysis. The HPLC analysis can be performed according to the conditions and procedures described in the Examples.

### <<Skin quality improving agent>>

The present invention relates to a hair quality improving agent containing the cacao derived composition. The explanations about the cacao derived composition are as described above.

In one embodiment, the hair quality improving agent contains the cacao derived composition according to the first embodiment. The explanations about the cacao derived composition according to the first embodiment are as described above.

In another embodiment, the hair quality improving agent contains the cacao derived composition according to the second embodiment. The explanations about the cacao derived composition according to the second embodiment are as described above.

In still another embodiment, the hair quality improving agent contains the cacao derived composition according to the third embodiment. The explanations about the cacao derived composition according to the third embodiment are as described above.

The hair quality improving agent can be used by applying it to the hair of a subject, and can demonstrate a hair quality improving action through the hair quality improving action of the cacao derived composition. Accordingly, the hair quality improving agent is useful for a hair quality improving application. The subject is preferably a mammal, more preferably a human. The hair of the subject may be hair that grows on any part of the body of the subject, and examples thereof include hair on the head, eyebrows, axillary hair, mustache and beard, and body hair. Hair quality improvement encompasses preventing, treating, or improving symptoms caused by a decline in the moisturizing function of hair (including aging symptoms), such as dryness of hair, roughness of hair, and a decline in the flexibility or elasticity of hair.

The hair quality improving agent may be composed of the cacao derived composition, or may be formed by formulating the cacao derived composition. The cacao derived composition can be formulated into a dosage form, such as liquid, suspension, emulsion, lotion, gel, cream, tablet, pill, powder, granule, and capsule, according to conventional methods, using pharmaceutically acceptable additives. The additives can be selected from, for example, excipients, binders, disintegrants, lubricants, stabilizers, taste and odor correctives, emulsifiers, and others.

The hair quality improving agent can be used by, for example, applying it to the hair as an ointment, a liquid for external use, a patch, or the like, and it can also be used by blending it into other compositions (for example, cosmetics for hair, pharmaceutical compositions, food compositions, and others).

The hair quality improving agent may contain the cacao derived composition as the sole hair quality improving component, or may contain other hair quality improving components.

The amount of the cacao derived composition blended can be adjusted as appropriate depending on the dosage form of the hair quality improving agent. The amount of the cacao derived composition blended is, for example, 0.01 to 100% by mass, preferably 0.1 to 99% by mass, based on the mass of the hair quality improving agent.

In the case where the hair quality improving agent contains the cacao derived composition according to the first embodiment, the amount of the ceramide AP derived from the cacao derived composition according to the first embodiment is, for example, 0.001% by mass or more, preferably 0.01% by mass or more, more preferably 0.03% by mass or more, even more preferably 0.05% by mass or more, and even more preferably 0.1% by mass or more, based on the mass of the hair quality improving agent. The upper limit is not particularly limited, and for example, may be 10.0% by mass or less, 5.0% by mass or less, or 1.0% by mass or less. Each of these upper limits may be combined with any of the lower limits described above.

In the case where the hair quality improving agent contains the cacao derived composition according to the second embodiment, the amount of the free ceramide derived from the cacao derived composition according to the second embodiment is, for example, 0.001% by mass or more, preferably 0.01% by mass or more, more preferably 0.03% by mass or more, even more preferably 0.05% by mass or more, and even more preferably 0.1% by mass or more, based on the mass of the hair quality improving agent. The upper limit is not particularly limited, and for example, may be 10.0% by mass or less, 5.0% by mass or less, or 1.0% by mass or less. Each of these upper limits may be combined with any of the lower limits described above.

In the case where the hair quality improving agent contains the cacao derived composition according to the third embodiment, the amount of the free ceramide derived from the cacao derived composition according to the third embodiment is, for example, 0.001% by mass or more, preferably 0.01% by mass or more, more preferably 0.03% by mass or more, even more preferably 0.05% by mass or more, and even more preferably 0.1% by mass or more, based on the mass of the hair quality improving agent. The upper limit is not particularly limited, and for example, may be 10.0% by mass or less, 5.0% by mass or less, or 1.0% by mass or less. Each of these upper limits may be combined with any of the lower limits described above.

The "amount of the ceramide AP" means, in the case where the cacao derived composition according to the first embodiment contains one type of ceramide AP, the amount of the one type of ceramide AP, and in the case where the cacao derived composition according to the first embodiment contains two or more types of ceramide APs, the total amount of the two or more types of ceramide APs. The amount of the ceramide AP can be measured by LC-MS/MS analysis. The LC-MS/MS analysis can be performed according to the conditions and procedures described in the Examples.

The "amount of the free ceramide" means, in the case where the cacao derived composition according to the second or third embodiment contains one type of free ceramide, the amount of the one type of free ceramide, and in the case where the cacao derived composition according to the second or third embodiment contains two or more types of free ceramides, the total amount of the two or more types of free ceramides. The amount of the free ceramide can be measured by HPLC analysis. The HPLC analysis can be performed according to the conditions and procedures described in the Examples.

### <<Cosmetic for skin>>

The present invention relates to a cosmetic for skin containing the cacao derived composition. The explanations about the cacao derived composition are as described above.

In one embodiment, the cosmetic for skin contains the cacao derived composition according to the first embodiment. The explanations about the cacao derived composition according to the first embodiment are as described above.

In another embodiment, the cosmetic for skin contains the cacao derived composition according to the second embodiment. The explanations about the cacao derived composition according to the second embodiment are as described above.

In still another embodiment, the cosmetic for skin contains the cacao derived composition according to the third embodiment. The explanations about the cacao derived composition according to the third embodiment are as described above.

The cacao derived composition may be blended into the cosmetic for skin as it is, or it may be formulated into the form of a moisturizing agent and/or a skin quality improving agent and then blended into the cosmetic for skin. The cosmetic for skin can be used by applying it to the skin of a subject, and can demonstrate a moisturizing action and/or a skin quality improving action through the moisturizing action and/or the skin quality improving action of the cacao derived composition. Accordingly, the cosmetic for skin is useful for a skin moisturizing application and/or a skin quality improving application. The subject is preferably a mammal, more preferably a human. The skin of the subject may be skin on any part of the body of the subject, and examples thereof include skin on the face, head, neck, back, shoulders, breasts, abdomen, arms, and legs. The skin quality improving application encompasses preventing, treating, or improving symptoms caused by a decline in the moisturizing function of skin (including aging symptoms), such as dry skin, rough skin, sagging, dullness, formation of spots or wrinkles, a decline in the flexibility or elasticity of skin, and a decline in the skin barrier function.

Examples of the cosmetic for skin include a skin toner, a cosmetic liquid, a cream, a lotion, a sunscreen, a cleaning agent, a shaving agent, a facial rinse, a pack, a cosmetic oil, a body rinse, and a foundation.

The cosmetic for skin is, for example, a cosmetic for scalp. Examples of the cosmetic for scalp include a hair conditioner, a hair grower, a scalp care product, a hair colorant, a hair washing product, a hair rinse, and a treatment.

The amount of the cacao derived composition blended can be adjusted as appropriate depending on the type of cosmetic for skin. The amount of the cacao derived composition blended is, for example, 0.01 to 100% by mass, preferably 0.1 to 99% by mass, based on the mass of the cosmetic for skin.

In the case where the cosmetic for skin contains the cacao derived composition according to the first embodiment, the amount of the ceramide AP derived from the cacao derived composition according to the first embodiment is, for example, 0.001% by mass or more, preferably 0.01% by mass or more, more preferably 0.03% by mass or more, even more preferably 0.05% by mass or more, and even more preferably 0.1% by mass or more, based on the mass of the cosmetic for skin. The upper limit is not particularly limited, and for example, may be 10.0% by mass or less, 5.0% by mass or less, or 1.0% by mass or less. Each of these upper limits may be combined with any of the lower limits described above.

In the case where the cosmetic for skin contains the cacao derived composition according to the second embodiment, the amount of the free ceramide derived from the cacao derived composition according to the second embodiment is, for example, 0.001% by mass or more, preferably 0.01% by mass or more, more preferably 0.03% by mass or more, even more preferably 0.05% by mass or more, and even more preferably 0.1% by mass or more, based on the mass of the cosmetic for skin. The upper limit is not particularly limited, and for example, may be 10.0% by mass or less, 5.0% by mass or less, or 1.0% by mass or less. Each of these upper limits may be combined with any of the lower limits described above.

In the case where the cosmetic for skin contains the cacao derived composition according to the third embodiment, the amount of the free ceramide derived from the cacao derived composition according to the third embodiment is, for example, 0.001% by mass or more, preferably 0.01% by mass or more, more preferably 0.03% by mass or more, even more preferably 0.05% by mass or more, and even more preferably 0.1% by mass or more, based on the mass of the cosmetic for skin. The upper limit is not particularly limited, and for example, may be 10.0% by mass or less, 5.0% by mass or less, or 1.0% by mass or less. Each of these upper limits may be combined with any of the lower limits described above.

The "amount of the ceramide AP" means, in the case where the cacao derived composition according to the first embodiment contains one type of ceramide AP, the amount of the one type of ceramide AP, and in the case where the cacao derived composition according to the first embodiment contains two or more types of ceramide APs, the total amount of the two or more types of ceramide APs. The amount of the ceramide AP can be measured by LC-MS/MS analysis. The LC-MS/MS analysis can be performed according to the conditions and procedures described in the Examples.

The "amount of the free ceramide" means, in the case where the cacao derived composition according to the second or third embodiment contains one type of free ceramide, the amount of the one type of free ceramide, and in the case where the cacao derived composition according to the second or third embodiment contains two or more types of free ceramides, the total amount of the two or more types of free ceramides. The amount of the free ceramide can be measured by HPLC analysis. The HPLC analysis can be performed according to the conditions and procedures described in the Examples.

The cosmetic for skin can be produced by using the cacao derived composition and ordinary raw materials for cosmetics for skin. Examples of the base component for the cosmetic for skin include ethanol, polyhydric alcohols, water, hydrocarbons, higher fatty acids, higher alcohols, oils and fats, waxes, ethers, esters, and silicones. In addition, components such as surfactants, moisturizing components, barrier function improving components, antioxidant components, whitening components, antibacterial components, stabilizing agents, flavors, colorants, and preservatives may also be used.

The cosmetic for skin may contain the cacao derived composition as the sole moisturizing component or the sole skin quality improving component, or may contain other moisturizing components and/or other skin quality improving components.

The cosmetic for skin can be produced by ordinary methods for producing cosmetics for skin. The method for producing the cosmetic for skin can include, for example, steps such as mixing of raw materials, heating, dissolution, drying, and filling.

### <<Cosmetic for hair>>

The present invention relates to a cosmetic for hair containing the cacao derived composition. The explanations about the cacao derived composition are as described above.

In one embodiment, the cosmetic for hair contains the cacao derived composition according to the first embodiment. The explanations about the cacao derived composition according to the first embodiment are as described above.

In another embodiment, the cosmetic for hair contains the cacao derived composition according to the second embodiment. The explanations about the cacao derived composition according to the second embodiment are as described above.

In still another embodiment, the cosmetic for hair contains the cacao derived composition according to the third embodiment. The explanations about the cacao derived composition according to the third embodiment are as described above.

The cacao derived composition may be blended into the cosmetic for hair as it is, or it may be formulated into the form of a moisturizing agent and/or a hair quality improving agent and then blended into the cosmetic for hair. The cosmetic for hair can be used by applying it to the hair of a subject, and can demonstrate a moisturizing action and/or a hair quality improving action through the moisturizing action and/or the hair quality improving action of the cacao derived composition. Accordingly, the cosmetic for hair is useful for a hair moisturizing application and/or a hair quality improving application. The subject is preferably a mammal, more preferably a human. The hair of the subject may be hair that grows on any part of the body of the subject, and examples thereof include hair on the head, eyebrows, axillary hair, mustache and beard, and body hair. Hair quality improvement encompasses preventing, treating, or improving symptoms caused by a decline in the moisturizing function of hair (including aging symptoms), such as dryness of hair, roughness of hair, and a decline in the flexibility or elasticity of hair.

Examples of the cosmetic for hair include a skin toner, a cosmetic liquid, a cream, a lotion, a sunscreen, a cleaning agent, a shaving agent, a facial rinse, a pack, a cosmetic oil, a body rinse, and a foundation.

The cosmetic for hair is, for example, a cosmetic for hair on the head. Examples of the cosmetic for hair on the head include a hair conditioner, a hair grower, a hair colorant, a hair washing product, a hair rinse, and a treatment.

The amount of the cacao derived composition blended can be adjusted as appropriate depending on the type of cosmetic for hair. The amount of the cacao derived composition blended is 0.01 to 100% by mass, preferably 0.1 to 99% by mass, based on the mass of the cosmetic for hair.

In the case where the cosmetic for hair contains the cacao derived composition according to the first embodiment, the amount of the ceramide AP derived from the cacao derived composition according to the first embodiment is, for example, 0.001% by mass or more, preferably 0.01% by mass or more, more preferably 0.03% by mass or more, even more preferably 0.05% by mass or more, and even more preferably 0.1% by mass or more, based on the mass of the cosmetic for hair. The upper limit is not particularly limited, and for example, may be 10.0% by mass or less, 5.0% by mass or less, or 1.0% by mass or less. Each of these upper limits may be combined with any of the lower limits described above.

In the case where the cosmetic for hair contains the cacao derived composition according to the second embodiment, the amount of the free ceramide derived from the cacao derived composition according to the second embodiment is, for example, 0.001% by mass or more, preferably 0.01% by mass or more, more preferably 0.03% by mass or more, even more preferably 0.05% by mass or more, and even more preferably 0.1% by mass or more, based on the mass of the cosmetic for hair. The upper limit is not particularly limited, and for example, may be 10.0% by mass or less, 5.0% by mass or less, or 1.0% by mass or less. Each of these upper limits may be combined with any of the lower limits described above.

In the case where the cosmetic for hair contains the cacao derived composition according to the third embodiment, the amount of the free ceramide derived from the cacao derived composition according to the third embodiment is, for example, 0.001% by mass or more, preferably 0.01% by mass or more, more preferably 0.03% by mass or more, even more preferably 0.05% by mass or more, and even more preferably 0.1% by mass or more, based on the mass of the cosmetic for hair. The upper limit is not particularly limited, and for example, may be 10.0% by mass or less, 5.0% by mass or less, or 1.0% by mass or less. Each of these upper limits may be combined with any of the lower limits described above.

The "amount of the ceramide AP" means, in the case where the cacao derived composition according to the first embodiment contains one type of ceramide AP, the amount of the one type of ceramide AP, and in the case where the cacao derived composition according to the first embodiment contains two or more types of ceramide APs, the total amount of the two or more types of ceramide APs. The amount of the ceramide AP can be measured by LC-MS/MS analysis. The LC-MS/MS analysis can be performed according to the conditions and procedures described in the Examples.

The "amount of the free ceramide" means, in the case where the cacao derived composition according to the second or third embodiment contains one type of free ceramide, the amount of the one type of free ceramide, and in the case where the cacao derived composition according to the second or third embodiment contains two or more types of free ceramides, the total amount of the two or more types of free ceramides. The amount of the free ceramide can be measured by HPLC analysis. The HPLC analysis can be performed according to the conditions and procedures described in the Examples.

The cosmetic for hair can be produced by using the cacao derived composition and ordinary raw materials for cosmetics for hair. Examples of the base component for the cosmetic for hair include ethanol, polyhydric alcohols, water, hydrocarbons, higher fatty acids, higher alcohols, oils and fats, waxes, ethers, esters, and silicones. In addition, components such as surfactants, moisturizing components, barrier function improving components, antioxidant components, whitening components, antibacterial components, stabilizing agents, flavors, colorants, and preservatives may also be used.

The cosmetic for hair may contain the cacao derived composition as the sole moisturizing component or the sole hair quality improving component, or may contain other moisturizing components and/or other hair quality improving components.

The cosmetic for hair can be produced by ordinary methods for producing cosmetics for hair. The method for producing the cosmetic for hair can include, for example, steps such as mixing of raw materials, heating, dissolution, drying, and filling.

### <<Pharmaceutical composition>>

The present invention relates to a pharmaceutical composition containing the cacao derived composition. The explanations about the cacao derived composition are as described above.

In one embodiment, the pharmaceutical composition contains the cacao derived composition according to the first embodiment. The explanations about the cacao derived composition according to the first embodiment are as described above.

In another embodiment, the pharmaceutical composition contains the cacao derived composition according to the second embodiment. The explanations about the cacao derived composition according to the second embodiment are as described above.

In still another embodiment, the pharmaceutical composition contains the cacao derived composition according to the third embodiment. The explanations about the cacao derived composition according to the third embodiment are as described above.

The cacao derived composition may be blended into the pharmaceutical composition as it is, or it may be formulated into the form of one or two or more of the following: moisturizing agent, skin quality improving agent, and hair quality improving agent and then blended into the pharmaceutical composition. The pharmaceutical composition can be used by applying it to the skin and/or hair of a subject, and can demonstrate one or two or more of the following: moisturizing action, skin quality improving action, and hair quality improving action through one or two or more of the moisturizing action, skin quality improving action, and hair quality improving action of the cacao derived composition. Accordingly, the pharmaceutical composition is useful for one or two or more of the following: skin moisturizing application, skin quality improving application, and hair quality improving application. The subject is preferably a mammal, more preferably a human. The skin of the subject may be skin on any part of the body of the subject, and examples thereof include skin on the face, head, neck, back, shoulders, breasts, abdomen, arms, and legs. The skin quality improving application encompasses preventing, treating, or improving symptoms caused by a decline in the moisturizing function of skin (including aging symptoms), such as dry skin, rough skin, sagging, dullness, formation of spots or wrinkles, a decline in the flexibility or elasticity of skin, and a decline in the skin barrier function. The hair of the subject may be hair that grows on any part of the body of the subject, and examples thereof include hair on the head, eyebrows, axillary hair, mustache and beard, and body hair. Hair quality improvement encompasses preventing, treating, or improving symptoms caused by a decline in the moisturizing function of hair (including aging symptoms), such as dryness of hair, roughness of hair, and a decline in the flexibility or elasticity of hair.

The pharmaceutical composition may be composed of the cacao derived composition, or may be formed by formulating the cacao derived composition. The cacao derived composition can be formulated into a dosage form, such as liquid, suspension, emulsion, lotion, gel, cream, tablet, pill, powder, granule, and capsule, according to conventional methods, using pharmaceutically acceptable additives. The additives can be selected from, for example, excipients, binders, disintegrants, lubricants, stabilizers, taste and odor correctives, emulsifiers, and others.

The pharmaceutical composition can be used by, for example, applying it to the skin and/or hair as an ointment, a liquid for external use, a patch, or the like.

The pharmaceutical composition may contain the cacao derived composition as the sole moisturizing component, the sole skin quality improving component, or the sole hair quality improving component, or may contain one or two or more of the following: other moisturizing components, other skin quality improving components, and other hair quality improving components.

The amount of the cacao derived composition blended can be adjusted as appropriate depending on the dosage form of the pharmaceutical composition. The amount of the cacao derived composition blended is, for example, 0.01 to 100% by mass, preferably 0.1 to 99% by mass, based on the mass of the pharmaceutical composition.

In the case where the pharmaceutical composition contains the cacao derived composition according to the first embodiment, the amount of the ceramide AP derived from the cacao derived composition according to the first embodiment is, for example, 0.001% by mass or more, preferably 0.01% by mass or more, more preferably 0.03% by mass or more, even more preferably 0.05% by mass or more, and even more preferably 0.1% by mass or more, based on the mass of the pharmaceutical composition. The upper limit is not particularly limited, and for example, may be 10.0% by mass or less, 5.0% by mass or less, or 1.0% by mass or less. Each of these upper limits may be combined with any of the lower limits described above.

In the case where the pharmaceutical composition contains the cacao derived composition according to the second embodiment, the amount of the free ceramide derived from the cacao derived composition according to the second embodiment is, for example, 0.001% by mass or more, preferably 0.01% by mass or more, more preferably 0.03% by mass or more, even more preferably 0.05% by mass or more, and even more preferably 0.1% by mass or more, based on the mass of the pharmaceutical composition. The upper limit is not particularly limited, and for example, may be 10.0% by mass or less, 5.0% by mass or less, or 1.0% by mass or less. Each of these upper limits may be combined with any of the lower limits described above.

In the case where the pharmaceutical composition contains the cacao derived composition according to the third embodiment, the amount of the free ceramide derived from the cacao derived composition according to the third embodiment is, for example, 0.001% by mass or more, preferably 0.01% by mass or more, more preferably 0.03% by mass or more, even more preferably 0.05% by mass or more, and even more preferably 0.1% by mass or more, based on the mass of the pharmaceutical composition. The upper limit is not particularly limited, and for example, may be 10.0% by mass or less, 5.0% by mass or less, or 1.0% by mass or less. Each of these upper limits may be combined with any of the lower limits described above.

The "amount of the ceramide AP" means, in the case where the cacao derived composition according to the first embodiment contains one type of ceramide AP, the amount of the one type of ceramide AP, and in the case where the cacao derived composition according to the first embodiment contains two or more types of ceramide APs, the total amount of the two or more types of ceramide APs. The amount of the ceramide AP can be measured by LC-MS/MS analysis. The LC-MS/MS analysis can be performed according to the conditions and procedures described in the Examples.

The "amount of the free ceramide" means, in the case where the cacao derived composition according to the second or third embodiment contains one type of free ceramide, the amount of the one type of free ceramide, and in the case where the cacao derived composition according to the second or third embodiment contains two or more types of free ceramides, the total amount of the two or more types of free ceramides. The amount of the free ceramide can be measured by HPLC analysis. The HPLC analysis can be performed according to the conditions and procedures described in the Examples.

### <<Food composition>>

The present invention relates to a food composition containing the cacao derived composition. The explanations about the cacao derived composition are as described above.

In one embodiment, the food composition contains the cacao derived composition according to the first embodiment. The explanations about the cacao derived composition according to the first embodiment are as described above.

In another embodiment, the food composition contains the cacao derived composition according to the second embodiment. The explanations about the cacao derived composition according to the second embodiment are as described above.

In still another embodiment, the food composition contains the cacao derived composition according to the third embodiment. The explanations about the cacao derived composition according to the third embodiment are as described above.

The cacao derived composition may be blended into the food composition as it is, or it may be formulated into the form of one or two or more of the following: moisturizing agent, skin quality improving agent, and hair quality improving agent and then blended into the food composition. The food composition can demonstrate one or two or more of the following: moisturizing action, skin quality improving action, and hair quality improving action through one or two or more of the moisturizing action, skin quality improving action, and hair quality improving action of the cacao derived composition. Accordingly, the food composition is useful for one or two or more of the following: skin moisturizing application, skin quality improving application, and hair quality improving application. The subject to which the food composition is administered is preferably a mammal, more preferably a human. Examples of the target skin include skin on the face, head, neck, back, shoulders, breasts, abdomen, arms, and legs. The skin quality improving application encompasses preventing, treating, or improving symptoms caused by a decline in the moisturizing function of skin (including aging symptoms), such as dry skin, rough skin, sagging, dullness, formation of spots or wrinkles, a decline in the flexibility or elasticity of skin, and a decline in the skin barrier function. The target hair may be hair that grows on any part of the body, and examples thereof include hair on the head, eyebrows, axillary hair, mustache and beard, and body hair. Hair quality improvement encompasses preventing, treating, or improving symptoms caused by a decline in the moisturizing function of hair (including aging symptoms), such as dryness of hair, roughness of hair, and a decline in the flexibility or elasticity of hair.

Examples of the food composition include processed foods, confectioneries, beverages, and health foods. The food composition widely includes general foods, health foods (functional foods and beverages), foods with health claims (foods for specified health uses, foods with nutrient function claims), quasi drugs, pharmaceuticals, and others that are orally ingested. The food composition is preferably a food composition that can indicate the action effect of the cacao derived composition on the food composition or packaging thereof, and is more preferably a food with health claims (food for specified health uses, food with function claims, food with nutrient function claims), a quasi drug, or a pharmaceutical.

The food composition may contain the cacao derived composition as the sole moisturizing component, the sole skin quality improving component, or the sole hair quality improving component, or may contain one or two or more of the following: other moisturizing components, other skin quality improving components, and other hair quality improving components.

The amount of the cacao derived composition blended can be adjusted as appropriate depending on the type of the food composition. The amount of the cacao derived composition blended is, for example, 0.01 to 100% by mass, preferably 0.1 to 99% by mass, based on the mass of the food composition.

In the case where the food composition contains the cacao derived composition according to the first embodiment, the amount of the ceramide AP derived from the cacao derived composition according to the first embodiment is, preferably 0.0006% by mass or more, more preferably 0.0012% by mass or more, and even more preferably 0.0018% by mass or more, based on the mass of the food composition. The upper limit is not particularly limited, and for example, may be 10% by mass or less, 5% by mass or less, or 1% by mass or less. Each of these upper limits may be combined with any of the upper limits described above.

In the case where the food composition contains the cacao derived composition according to the second embodiment, the amount of the free ceramide derived from the cacao derived composition according to the second embodiment is, preferably 0.0006% by mass or more, more preferably 0.0012% by mass or more, and even more preferably 0.0018% by mass or more, based on the mass of the food composition. The upper limit is not particularly limited, and for example, may be 10% by mass or less, 5% by mass or less, or 1% by mass or less. Each of these upper limits may be combined with any of the upper limits described above.

In the case where the food composition contains the cacao derived composition according to the third embodiment, the amount of the free ceramide derived from the cacao derived composition according to the third embodiment is, preferably 0.0006% by mass or more, more preferably 0.0012% by mass or more, and even more preferably 0.0018% by mass or more, based on the mass of the food composition. The upper limit is not particularly limited, and for example, may be 10% by mass or less, 5% by mass or less, or 1% by mass or less. Each of these upper limits may be combined with any of the upper limits described above.

The "amount of the ceramide AP" means, in the case where the cacao derived composition according to the first embodiment contains one type of ceramide AP, the amount of the one type of ceramide AP, and in the case where the cacao derived composition according to the first embodiment contains two or more types of ceramide APs, the total amount of the two or more types of ceramide APs. The amount of the ceramide AP can be measured by LC-MS/MS analysis. The LC-MS/MS analysis can be performed according to the conditions and procedures described in the Examples.

The "amount of the free ceramide" means, in the case where the cacao derived composition according to the second or third embodiment contains one type of free ceramide, the amount of the one type of free ceramide, and in the case where the cacao derived composition according to the second or third embodiment contains two or more types of free ceramides, the total amount of the two or more types of free ceramides. The amount of the free ceramide can be measured by HPLC analysis. The HPLC analysis can be performed according to the conditions and procedures described in the Examples.

The food composition can be produced by using the cacao derived composition and ordinary raw materials for food compositions.

The food composition can be produced by ordinary methods for producing food compositions. The method for producing the food composition can include, for example, steps such as mixing of raw materials, heating, dissolution, drying, and filling.

Although there are no particular restrictions on the target to which the moisturizing agent, skin quality improving agent, hair quality improving agent, cosmetic for skin, cosmetic for hair, pharmaceutical composition, or food composition is applied, they are suitable for humans who are concerned about skin deterioration (such as sagging, wrinkles, dullness, and rough skin) and/or hair deterioration (such as dryness of hair, roughness of hair, and a decline in the flexibility or elasticity of hair) due to aging. For example, they are suitable for women who are 20 years old or older, 30 years old or older, 40 years old or older, 50 years old or older, or 60 years old or older.

### EXAMPLES

### [Example 1] Preparation and component analysis of cacao bean shell extract

### (1) Preparation of cacao bean shell extract

Cacao bean shell extracts 1A and 1B were obtained by the following methods.

### [Cacao bean shell extract 1A]

Cacao bean shell was crushed with a mill. To 0.2 g of the crushed cacao bean shell, 8.0 mL of ethanol of 99% by volume was added, and the mixture was subjected to an extraction treatment at normal temperature (about 25°C) for 16 hours while shaking it at 150 rpm. At a temperature of 25°C, the extraction treated product was centrifuged at 15000 rpm for 30 minutes, and from the resultant supernatant liquid, a cacao bean shell extract 1A was obtained.

### [Cacao bean shell extract 1B]

Cacao bean shell was crushed with a mill. To 100 g of the crushed cacao bean shell, 4 L of ethanol of 99.5% by volume was added, and the mixture was subjected to an extraction treatment for 24 hours while stirring it with a hot plate stirrer (set temperature of 37°C). The extraction treated product was filtered through filter paper (manufactured by ADVANTEC, No. 2), and then concentrated to 1 L with an evaporator to obtain a cacao bean shell extract 1B.

### (2) Component analysis of cacao bean shell extract

Component analysis of the cacao bean shell extracts 1A and 1B was performed by the following methods.

### [Component analysis of cacao bean shell extract 1A]

To 0.04 mL of the cacao bean shell extract 1A, 0.76 mL of ethanol of 99% by volume and 0.2 mL of 0.2 M hydrochloric acid were mixed, and then at a temperature of 4°C, the mixture was centrifuged at 20000 rpm for 30 minutes. At a temperature of 4°C, the resultant supernatant liquid was further centrifuged at 20000 rpm for 30 minutes, and the resultant supernatant liquid was used as an analysis sample.

### [Component analysis of cacao bean shell extract 1B]

0.7 g of a dried and solidified product of the cacao bean shell extract 1B was immersed in 500.0 mL of ethanol of 99% by volume, which was subjected to a re-extraction treatment. The re-extraction treatment was performed at normal temperature (about 25°C) for 24 hours. 55.7 µL of the cacao bean shell extract obtained by the re-extraction treatment, 744 µL of ethanol of 99% by volume, and 200 µL of 0.2 M hydrochloric acid were mixed, and then at a temperature of 4°C, the mixture was centrifuged at 15000 rpm for 20 minutes. At a temperature of 4°C, the resultant supernatant liquid was further centrifuged at 15000 rpm for 20 minutes, and the resultant supernatant liquid was used as an analysis sample.

The above centrifugal supernatant liquids obtained from the cacao bean shell extracts 1A and 1B were each subjected to LC-ESI-MS/MS (liquid chromatography-electrospray ionization-tandem mass spectrometry) and analyzed according to the method of Yumoto et al. (Bioscience, Biotechnology, and Biochemistry (2021) 85, 205-210). That is, Agilent 6460 with Agilent 1200 separation module was used as the ESI-MS/MS, and the TSKgel ODS-120A column (2.1 mm i.d. × 25 cm, manufactured by Tosoh Corporation, registered trademark) was used as the column for high performance liquid chromatography. According to the LC-MS/MS conditions in Table B below, a glucosylceramide and a free ceramide were separated and detected in the ESI-positive ion mode. The m/z [M+H]⁺ of the precursor ion for detecting the glucosylceramide and free ceramide, the m/z of the product ion, and the collision energy (CE) (eV) are as shown in Table C below. The amount of the glucosylceramide and free ceramide detected was determined using as reference standards glucosylceramides purchased from Nagara Science Co., Ltd., phytoceramides purchased from Cayman Chemical Company, and free ceramides produced from glucosylceramides by imiglucerase (manufactured by Sanofi S.A.), which is human-derived glucocerebrosidase.

### <LC-MS/MS analysis conditions>

· Column temperature: 40°C
· Flow rate: 0.2 mL/min
· Mobile phases: mobile phase A 0.1 vol% formic acid aqueous solution, mobile phase B 0.1 vol% formic acid methanol solution
· Gradient:

**[Table B]**

| Time (min) | Mobile phase A (%) | Mobile phase B (%) |
|---|---|---|
| 0 | 10 | 90 |
| 30 | 2 | 98 |
| 50 | 2 | 98 |
| 50.1 | 0 | 100 |
| 60 | 0 | 100 |

**[Table C]**

| Type | Simplified designation | precursor ion *m*/*z* [M+H]⁺ | product ion *m*/*z* | CE (eV) |
|---|---|---|---|---|
| Glucosyl ceramide | d18:2(4*E*,8*Z*)-C16h:0-GluCer | 714.6 | 262.2 | 36 |
| | d18:2(4*E*,8*E*)-C16h:0-GluCer | 714.6 | 262.2 | 36 |
| | d18:2(4*E*,8*Z*)-C18h:0-GluCer | 742.6 | 262.2 | 36 |
| | d18:2(4*E*,8*E*)-C18h:0-GluCer | 742.6 | 262.2 | 36 |
| | d18:2(4*E*,8*Z*)-C20h:0-GluCer | 770.6 | 262.2 | 36 |
| | d18:2(4*E*,8*E*)-C20h:0-GluCer | 770.6 | 262.2 | 36 |
| | d18:2(4*E*,8*Z*)-C22h:0-GluCer | 798.6 | 262.2 | 40 |
| | d18:2(4*E*,8*E*)-C22h:0-GluCer | 798.6 | 262.2 | 40 |
| | d18:2(4*E*,8*Z*)-C24h:0-GluCer | 826.6 | 262.2 | 40 |
| | d18:2(4*E*,8*E*)-C24h:0-GluCer | 826.6 | 262.2 | 40 |
| | t18:1(8*Z*)-C22h:0-GluCer | 816.6 | 262.2 | 46 |
| | t18:1(8*E*)-C22h:0-GluCer | 816.6 | 262.2 | 46 |
| | t18:1(8*Z*)-C24h:0-GluCer | 844.6 | 262.2 | 50 |
| | t18:1(8*E*)-C24h:0-GluCer | 844.6 | 262.2 | 50 |
| | t18:1(8*Z*)-C26h:0-GluCer | 872.6 | 262.2 | 50 |
| | t18:1(8*E*)-C26h:0-GluCer | 872.6 | 262.2 | 50 |
| Free ceramide | d18:2(4*E*,8*Z*)-C16h:0-Cer | 552.6 | 262.2 | 24 |
| | d18:2(4*E*,8*E*)-C16h:0-Cer | 552.6 | 262.2 | 24 |
| | d18:2(4*E*,8*Z*)-C18h:0-Cer | 580.6 | 262.2 | 26 |
| | d18:2(4*E*,8*E*)-C18h:0-Cer | 580.6 | 262.2 | 26 |
| | d18:2(4*E*,8*Z*)-C20h:0-Cer | 608.6 | 262.2 | 28 |
| | d18:2(4*E*,8*E*)-C20h:0-Cer | 608.6 | 262.2 | 28 |
| | d18:2(4*E*,8*Z*)-C22h:0-Cer | 636.6 | 262.2 | 32 |
| | d18:2(4*E*,8*E*)-C22h:0-Cer | 636.6 | 262.2 | 32 |
| | d18:2(4*E*,8*Z*)-C24h:0-Cer | 664.6 | 262.2 | 34 |
| | d18:2(4*E*,8*E*)-C24h:0-Cer | 664.6 | 262.2 | 34 |
| | t18:1(8*Z*)-C22h:0-Cer | 654.6 | 262.2 | 36 |
| | t18:1(8*F*)-C22h:0-Cer | 654.6 | 262.2 | 36 |
| | t18:1(8*Z*)-C24h:0-Cer | 682.6 | 262.2 | 36 |
| | t18:1(8*E*)-C24h:0-Cer | 682.6 | 262.2 | 36 |
| | t18:1(8*Z*)-C26h:0-Cer | 710.6 | 262.2 | 36 |
| | t18:1(8*E*)-C26h:0-Cer | 710.6 | 262.2 | 36 |
| | t18:0-C22h:0-Cer | 656.7 | 264.4 | 38 |
| | t18:0-C24h:0-Cer | 684.7 | 264.4 | 38 |
| | t18:0-C26h:0-Cer | 712.7 | 264.4 | 38 |
| | t18:0-C24:0-Cer | 668.7 | 264.4 | 36 |

The analysis was conducted using the following analysis software, and for the measurement results, a chromatogram was created using the following qualitative software, and the quantitative value was determined using the following quantitative software.
· Analysis software: Agilent Data Acquisition
· Qualitative software: Agilent Qualitative Analysis
· Quantitative software: Agilent QQQ Quantitative Analysis (Quant-My-Way)

The analysis results are shown in Tables 1 and 2.

In Tables 1 and 2, "µg/g" means the content (µg) of the target component per 1 g of the cacao bean shell extract.

In Tables 1 and 2, "*1" indicates that the amount of the glucosylceramide or free ceramide was calculated based on the value of a reference standard of 8Z form with the same molecular weight, "*2" indicates that the amount of the glucosylceramide or free ceramide was calculated based on the value of a reference standard of t18:1(8Z)-C24h:0-GluCer or t18:1(8Z)-C24h:0-Cer, and "*3" indicates that the amount of the free ceramide was calculated based on the value of a reference standard of t18:0-C24h:0-Cer.

The simplified designations in Tables C, 1, and 2 will be described using "d18:2(4E,8Z)-C16h:0-GluCer" and "t18:1(8Z)-C22h:0-Cer" as examples.

In "d18:2(4E,8Z)-C16h:0-GluCer", the first part "d18:2(4E,8Z)" is the information regarding the sphingoid base, and "d" indicates that the number of hydroxyl groups in the sphingoid base is 2, "18" indicates that the number of carbon atoms in the sphingoid base is 18, "2" indicates that the number of carbon-carbon double bonds in the sphingoid base is 2, and "(4E,8Z)" indicates that the EZ configuration of the double bonds of the carbons at positions 4 and 8 in the sphingoid base are 4E and 8Z. In "d18:2(4E,8Z)-C16h:0-GluCer", the middle part "C16h:0" is the information regarding the fatty acid, and "C16" indicates that the number of carbon atoms in the fatty acid is 16, "h" indicates that the number of hydroxyl groups in the fatty acid is 1, and "0" indicates that the number of carbon-carbon double bonds in the fatty acid is 0. Those with no description of "h" indicate that the number of hydroxyl groups in the fatty acid is 0. In "d18:2(4E,8Z)-C16h:0-GluCer", the last part "GluCer" indicates that it is a glucosylceramide.

In "t18:1(8Z)-C22h:0-Cer", the first part "t18:1(8Z)" is the information regarding the sphingoid base, and "t" indicates that the number of hydroxyl groups in the sphingoid base is 3, "18" indicates that the number of carbon atoms in the sphingoid base is 18, "1" indicates that the number of carbon-carbon double bonds in the sphingoid base is 1, and "(8Z)" indicates that the EZ configuration of the double bond of the carbon at position 8 in the sphingoid base is 8Z. In "t18:1(8Z)-C22h:0-Cer", the middle part "C22h:0" is the information regarding the fatty acid, and "C22" indicates that the number of carbon atoms in the fatty acid is 22, "h" indicates that the number of hydroxyl groups in the fatty acid is 1, and "0" indicates that the number of carbon-carbon double bonds in the fatty acid is 0. Those with no description of "h" indicate that the number of hydroxyl groups in the fatty acid is 0. In "t18:1(8Z)-C22h:0-Cer", the last part "Cer" indicates that it is a free ceramide.

Note that the number of hydroxyl groups in the sphingoid base constituting the glucosylceramide includes the hydroxyl group to which glucose is glycosidically bonded, as well.

**[Table 1]**

| Table 1 Type and content of glucosylceramide | | | |
|---|---|---|---|
| Type | Simplified designation | Analysis value for extract 1A (µg/g) | Analysis value for extract 1B (µg/g) |
| Glucosyl ceramide | d18:2(4*E*,8*Z*)-C16h:0-GluCer | 38.19 | 23.43 |
| | d18:2(4*E*,8*E*)-C16h:0-GluCer | 37.70 | 24.19 |
| | d18:2(4*E*,8*Z*)-C18h:0-GluCer | 17.69 | 15.13 |
| | d18:2(4*E*,8*E*)-C18h:0-GluCer | 18.57 | 14.59 |
| | d18:2(4*E*,8*Z*)-C20h:0-GluCer | 9.48 | 7.80 |
| | d18:2(4*E*,8*E*)-C20h:0-GluCer | 9.71 | 7.21 |
| | d18:2(4*E*,8*Z*)-C22h:0-GluCer | 2.43 | 2.32 |
| | d18:2(4*E*,8*E*)-C22h:0-GluCer ^{*1} | 2.42 | 1.90 |
| | d18:2(4*E*,8*Z*)-C24h:0-GluCer | 2.64 | 1.94 |
| | d18:2(4*E*,8*E*)-C24h:0-GluCer ^{*1} | 2.81 | 1.95 |
| | t18:1(8*Z*)-C22h:0-GluCer | 63.36 | 58.85 |
| | t18:1(8*E*)-C22h:0-GluCer ^{*1} | 29.75 | 25.71 |
| | t18:1(8*Z*)-C24h:0-GluCer | 113.96 | 100.7 |
| | t18:1(8*E*)-C24h:0-GluCer ^{*1} | 52.64 | 46.06 |
| | t18:1(8*Z*)-C26h:0-GluCer ^{*2} | 30.94 | 27.42 |
| | t18:1(8*E*)-C26h:0-Glucer ^{*2} | 15.15 | 13.15 |
| Total content | | 447.44 | 372.35 |

**[Table 2]**

| Table 2 Type and content of free ceramide | | | |
|---|---|---|---|
| Type | Simplified designation | Analysis value for extract 1A (µg/g) | Analysis value for extract 1B (µg/g) |
| Ceramide AP | t18:0-C22h:0-Cer^{*3} | 172.91 | 104.08 |
| | t18:0-C24h:0-Cer | 513.74 | 292.02 |
| | t18:0-C26h:0-Cer ^{*3} | 142.05 | 84.20 |
| Other free ceramides | d18:2(4*E*,8*Z*)-C16h:0-Cer | 12.95 | 5.50 |
| | d18:2(4*E*,8*E*)-C16h:0-Cer | 9.52 | 4.04 |
| | d18:2(4*E*,8*Z*)-C18h:0-Cer | 6.68 | 2.97 |
| | d18:2(4*E*,8*E*)-C18h:0-Cer | 4.83 | 2.11 |
| | d18:2(4*E*,8*Z*)-C20h:0-Cer | 3.69 | 1.67 |
| | d18:2(4*E*,8*E*)-C20h:0-Cer | 2.48 | 1.20 |
| | d18:2(4*E*,8*Z*)-C22h:0-Cer | 1.19 | 0.61 |
| | d18:2(4*E*,8*E*)-C22h:0-Cer ^{*1} | 0.69 | 0.45 |
| | d18:2(4*E*,8*Z*)-C24h:0-Cer | 1.41 | 0.76 |
| | d18:2(4*E*,8*E*)-C24h:0-Cer ^{*1} | 1.03 | 0.60 |
| | t18:1(8*Z*)-C22h:0-Cer | 41.82 | 24.92 |
| | t18:1(8*E*)-C22h:0-Cer ^{*1} | 68.79 | 43.80 |
| | t18:1(8Z)-C24h:0-Cer | 94.83 | 56.93 |
| | t18:1(8*E*)-C24h:0-Cer ^{*1} | 233.09 | 147.62 |
| | t18:1(8*Z*)-C26h:0-Cer ^{*2} | 18.65 | 11.31 |
| | t18:1(8*E*)-C26h:0-Cer ^{*2} | 69.68 | 45.68 |
| | t18:0-C24:0-Cer | 8.97 | 6.45 |
| Total content | | 1409.01 | 836.92 |

As shown in Tables 1 and 2, the total content of glucosylceramides was 447.44 µg/g (analysis value for the extract 1A) and 372.35 µg/g (analysis value for the extract 1B), the total content of free ceramides was 1409.01 µg/g (analysis value for the extract 1A) and 836.92 µg/g (analysis value for the extract 1B), the sum of the total content of glucosylceramides and the total content of free ceramides was 1856.45 µg/g (analysis value for the extract 1A) and 1209.26 µg/g (analysis value for the extract 1B), and the total content of ceramide APs in the total content of free ceramides was 828.70 µg/g (analysis value for the extract 1A) and 480.29 µg/g (analysis value for the extract 1B).

### [Example 2]

### (1) Preparation of cacao bean germ extract

Cacao bean germ was crushed with a mill. To 0.1 g of the crushed cacao bean germ, 8.0 mL of ethanol of 99% by volume was added, and the mixture was subjected to an extraction treatment at normal temperature (about 25°C) for 16 hours while shaking it at 150 rpm. At a temperature of 25°C, the extraction treated product was centrifuged at 15000 rpm for 30 minutes, and from the resultant supernatant liquid, a cacao bean germ extract was obtained.

### (2) Component analysis of cacao bean germ extract

To 0.1 mL of the cacao bean germ extract obtained in (1) above, 0.3 mL of ethanol of 99% by volume and 0.1 mL of 0.2 M hydrochloric acid were mixed, and the mixture was then centrifuged. The centrifugation was performed in the same manner as the centrifugation for the extract 1A in Example 1 (2). The resultant centrifugal supernatant was analyzed with a liquid chromatograph mass spectrometer (LC-MS/MS) in the same manner as in Example 1.

The analysis results are shown in Tables 3 and 4. The explanations about Tables 1 and 2 are also applicable to Tables 3 and 4.

**[Table 3]**

| Table 3 Type and content of glucosylceramide | | |
|---|---|---|
| Type | Simplified designation | Analysis value (µg/g) |
| Glucosyl ceramide | d18:2(4*E*,8*Z*)-C16h:0-GluCer | 73.87 |
| | d18:2(4*E*,8*E*)-C16h:0-GluCer | 41.06 |
| | d18:2(4*E*,8*Z*)-C18h:0-GluCer | 85.37 |
| | d18:2(4*E*,8*E*)-C18h:0-GluCer | 53.07 |
| | d18:2(4*E*,8*Z*)-C20h:0-GluCer | 55.86 |
| | d18:2(4*E*,8*E*)-C20h:0-GluCer | 34.37 |
| | d18:2(4*E*,8*Z*)-C22h:0-GluCer | 11.18 |
| | d18:2(4*E*,8*E*)-C22h:0-GluCer ^{*1} | 6.87 |
| | d18:2(4*E*,8*Z*)-C24h:0-GluCer | 7.91 |
| | d18:2(4*E*,8*E*)-C24h:0-GluCer ^{*1} | 5.03 |
| | U8:1(8*Z*)-C22h:0-GluCer | 50.84 |
| | t18:1(8*E*)-C22h:0-GluCer ^{*1} | 12.59 |
| | t18:1(8*Z*)-C24h:0-GluCer | 124.97 |
| | t18:1(8*E*)-C24h:0-GluCer ^{*1} | 28.40 |
| | t18:1(8*Z*)-C26h:0-GluCer ^{*2} | 46.56 |
| | t18:1(8*E*)-C26h:0-GluCer ^{*2} | 8.03 |
| Total content | | 654.99 |

**[Table 4]**

| Table 4 Type and content of free ceramide | | |
|---|---|---|
| Type | Simplified designation | Analysis value (µg/g) |
| Ceramide AP | t18:0-C22h:0-Cer^{*3} | 1.44 |
| | t18:0-C24h:0-Cer | 4.69 |
| | t18:0-C26h:0-Cer^{*3} | 1.13 |
| Other free ceramides | d18:2(4*E*,8*Z*)-C16h:0-Cer | 0.50 |
| | d18:2(4*E*,8*E*)-C16h:0-Cer | 0.20 |
| | d18:2(4*E*,8*Z*)-C18h:0-Cer | 0.59 |
| | d18:2(4*E*,8*E*)-C18h:0-Cer | 0.25 |
| | d18:2(4*E*,8*Z*)-C20h:0-Cer | 0.38 |
| | d18:2(4*E*,8*E*)-C20h:0-Cer | 0.16 |
| | d18:2(4*E*,8*Z*)-C22h:0-Cer | 0.10 |
| | d18:2(4*E*,8*E*)-C22h:0-Cer ^{*1} | 0.04 |
| | d18:2(4*E*,8*Z*)-C24h:0-Cer | 0.09 |
| | d18:2(4*E*,8*E*)-C24h:0-Cer ^{*1} | 0.05 |
| | t18:1(8*Z*)-C22h:0-Cer | 0.47 |
| | t18:1(8*E*)-C22h:0-Cer ^{*1} | 0.91 |
| | t18:1(8*Z*)-C24h:0-Cer | 1.21 |
| | t18:1(8*E*)-C24h:0-Cer ^{*1} | 3.02 |
| | t18:1(8*Z*)-C26h:0-Cer ^{*2} | 0.34 |
| | t18:1(8*E*)-C26h:0-Cer ^{*2} | 0.60 |
| | t18:0-C24:0-Cer | 2.46 |
| Total content | | 18.66 |

### [Example 3]

### (1) Preparation of cacao bean nib extract

A cacao bean nib extract was obtained in the same manner as in Example 2 (1), except that 0.2 g of cacao bean nib was used instead of 0.1 g of cacao bean germ.

### (2) Component analysis of cacao bean nib extract

Component analysis of the cacao bean nib extract was performed in the same manner as in Example 2 (2), except that the cacao bean nib extract obtained in (1) above was used instead of the cacao bean germ extract.

The analysis results are shown in Tables 5 and 6. The explanations about Tables 1 and 2 are also applicable to Tables 5 and 6.

**[Table 5]**

| Table 5 Type and content of glucosylceramide | | |
|---|---|---|
| Type | Simplified designation | Analysis value (µg/g) |
| Glucosyl ceramide | d18:2(4*E*,8*Z*)-C16h:0-GluCer | 24.70 |
| | d18:2(4*E*,8*E*)-C16h:0-GluCer | 12.10 |
| | d18:2(4*E*,8*Z*)-C18h:0-GluCer | 38.73 |
| | d18:2(4*E*,8*E*)-C18h:0-GluCer | 20.17 |
| | d18:2(4*E,*8*Z*)-C20h:0-GluCer | 14.43 |
| | d18:2(4*E*,8*E*)-C20h:0-GluCer | 7.17 |
| | d18:2(4*E*,8*Z*)-C22h:0-GluCer | 4.23 |
| | d18:2(4*E*,8*E*)-C22h:0-GluCer ^{*1} | 2.21 |
| | d18:2(4*E*,8*Z*)-C24h:0-GluCer | 2.40 |
| | d18:2(4*E*,8*E*)-C24h:0-GluCer ^{*1} | 1.34 |
| | t18:1(8*Z*)-C22h:0-GluCer | 44.59 |
| | t18:1(8*E*)-C22h:0-GluCer ^{*1} | 7.36 |
| | t18:1(8*Z*)-C24h:0-GluCer | 65.21 |
| | t18:1(8*E*)-C24h:0-GluCer ^{*1} | 9.24 |
| | t18:1(8*Z*)-C26h:0-GluCer ^{*2} | 13.76 |
| | t18:1(8*E*)-C26h:0-GluCer ^{*2} | 1.67 |
| Total content | | 269.33 |

**[Table 6]**

| Table 6 Type and content of free ceramide | | |
|---|---|---|
| Type | Simplified designation | Analysis value (µg/g) |
| Ceramide AP | t18:0-C22h:0-Cer^{*3} | 0.73 |
| | t18:0-C24h:0-Cer | 1.58 |
| | t18:0-C26h:0-Cer^{*3} | 0.34 |
| Other free ceramides | d18:2(4*E*,8*Z*)-C16h:0-Cer | 0.26 |
| | d18:2(4*E*,8*E*)-C16h:0-Cer | 0.09 |
| | d18:2(4*E*,8*Z*)-C18h:0-Cer | 0.44 |
| | d18:2(4*E*,8*E*)-C18h:0-Cer | 0.13 |
| | d18:2(4*E*,8*Z*)-C20h:0-Cer | 0.15 |
| | d18:2(4*E*,8*E*)-C20h:0-Cer | 0.03 |
| | d18:2(4*E*,8*Z*)-C22h:0-Cer | 0.05 |
| | d18:2(4E,8*E*)-C22h:0-Cer ^{*1} | 0.00 |
| | d18:2(4*E*,8*Z*)-C24h:0-Cer | 0.03 |
| | d18:2(4*E*,8*E*)-C24h:0-Cer ^{*1} | 0.00 |
| | t18:1(8*Z*)-C22h:0-Cer | 0.33 |
| | t18:1(8*E*)-C22h:0-Cer ^{*1} | 0.34 |
| | t18:1(8*Z*)-C24h:0-Cer | 0.51 |
| | t18:1(8*E*)-C24h:0-Cer ^{*1} | 0.67 |
| | t18:1(8*Z*)-C26h:0-Cer ^{*2} | 0.09 |
| | t18:1(8*E*)-C26h:0-Cer ^{*2} | 0.12 |
| | t18:0-C24:0-Cer | 1.21 |
| Total content | | 7.12 |

### [Example 4]

### (1) Preparation of cacao pod shell extract

A cacao pod shell extract was obtained in the same manner as in Example 2 (1), except that 0.2 g of frozen and dried cacao pod shell was used instead of 0.1 g of cacao bean germ.

### (2) Component analysis of cacao pod shell extract

To 0.05 mL of the cacao pod shell extract obtained in (1) above, 0.35 mL of ethanol of 99% by volume and 0.1 mL of 0.2 M hydrochloric acid were mixed, and the mixture was then centrifuged. The centrifugation was performed in the same manner as the centrifugation for the extract 1A in Example 1 (2). The resultant centrifugal supernatant was analyzed with a liquid chromatograph mass spectrometer (LC-MS/MS) in the same manner as in Example 1.

The analysis results are shown in Tables 7 and 8. The explanations about Tables 1 and 2 are also applicable to Tables 7 and 8.

**[Table 7]**

| Table 7 Type and content of glucosylceramide | | |
|---|---|---|
| Type | Simplified designation | Analysis value (µg/g) |
| Glucosyl ceramide | d18:2(4*E*,8*Z*)-C16h:0-GluCer | 93.51 |
| | d18:2(4*E*,8*E*)-C16h:0-GluCer | 46.13 |
| | d18:2(4*E*,8*Z*)-C18h:0-GluCer | 55.76 |
| | d18:2(4*E*,8*E*)-C18h:0-GluCer | 27.91 |
| | d18:2(4*E*,8*Z*)-C20h:0-GluCer | 28.11 |
| | d18:2(4*E*,8*E*)-C20h:0-GluCer | 13.07 |
| | d18:2(4*E*,8*Z*)-C22h:0-GluCer | 6.46 |
| | d18:2(4*E*,8*E*)-C22h:0-Glucer ^{*1} | 2.84 |
| | d18:2(4*E*,8Z)-C24h:0-GluCer | 5.29 |
| | d18:2(4*E*,8*E*)-C24h:0-GluCer ^{*1} | 2.97 |
| | t18:1(8*Z*)-C22h:0-GluCer | 61.17 |
| | t18:1(8*E*)-C22h:0-GluCer ^{*1} | 11.81 |
| | t18:1(8*Z*)-C24h:0-GluCer | 124.57 |
| | t18:1(8*E*)-C24h:0-GluCer ^{*1} | 21.45 |
| | t18:1(8*Z*)-C26h:0-GluCer ^{*2} | 27.80 |
| | t18:1(8*E*)-C26h:0-GluCer ^{*2} | 5.09 |
| Total content | | 533.94 |

**[Table 8]**

| Table 8 Type and content of free ceramide | | |
|---|---|---|
| Type | Simplified designation | Analysis value (µg/g) |
| Ceramide AP | t18:0-C22h:0-Cer^{*3} | 0.26 |
| | t18:0-C24h:0-Cer | 0.90 |
| | t18:0-C26h:0-Cer^{*3} | 0.34 |
| Other free ceramides | d18:2(4*E*,8*Z*)-C16h:0-Cer | 0.03 |
| | d18:2(4*E*,8*E*)-C16h:0-Cer | 0.02 |
| | d18:2(4*E*,8*Z*)-C18h:0-Cer | 0.02 |
| | d18:2(4*E*,8*E*)-C18h:0-Cer | 0.00 |
| | d18:2(4*E*,8*Z*)-C20h:0-Cer | 0.01 |
| | d18:2(4*E*,8*E*)-C20h:0-Cer | 0.00 |
| | d18:2(4*E*,8*Z*)-C22h:0-Cer | 0.00 |
| | d18:2(4*E*,8*E*)-C22h:0-Cer ^{*1} | 0.00 |
| | d18:2(4*E*,8*Z*)-C24h:0-Cer | 0.00 |
| | d18:2(4*E*,8*E*)-C24h:0-Cer ^{*1} | 0.00 |
| | t18:1(8*Z*)-C22h:0-Cer | 0.00 |
| | t18:1(8*E*)-C22h:0-Cer ^{*1} | 0.00 |
| | t18:1(8*Z*)-C24h:0-Cer | 0.17 |
| | t18:1(8*E*)-C24h:0-Cer ^{*1} | 0.32 |
| | t18:1(8*Z*)-C26h:0-Cer ^{*2} | 0.00 |
| | t18:1(8*E*)-C26h:0-Cer ^{*2} | 0.00 |
| | t18:0-C24:0-Cer | 0.84 |
| Total content | | 2.92 |

### [Example 5]

### (1) Preparation of cacao pulp extract

To 0.5 g of frozen and dried cacao pulp, 7.5 mL of ethanol of 99% by volume was added, and the mixture was subjected to an extraction treatment at normal temperature (about 25°C) for 16 hours while shaking it at 150 rpm. At a temperature of 25°C, the extraction treated product was centrifuged at 15000 rpm for 30 minutes, and from the resultant supernatant liquid, a cacao pulp extract was obtained.

### (2) Component analysis of cacao pulp extract

Component analysis of the cacao pulp extract was performed in the same manner as in Example 2 (2), except that the cacao pulp extract obtained in (1) above was used instead of the cacao bean germ extract.

The analysis results are shown in Tables 9 and 10. Descriptions regarding Tables 1 and 2 are also applicable to Tables 9 and 10.

**[Table 9]**

| Table 9 Type and content of glucosylceramide | | |
|---|---|---|
| Type | Simplified designation | Analysis value (µg/g) |
| Glucosyl ceramide | d18:2(4*E*,8*Z*)-C16h:0-GluCer | 20.17 |
| | d18:2(4*E*,8*E*)-C16h:0-GluCer | 10.91 |
| | d18:2(4*E*,8*Z*)-C18h:0-GluCer | 8.02 |
| | d18:2(4*E*,8*E*)-C18h:0-GluCer | 4.34 |
| | d18:2(4*E*,8*Z*)-C20h:0-GluCer | 3.83 |
| | d18:2(4*E*,8*E*)-C20h:0-GluCer | 1.96 |
| | d18:2(4*E*,8*Z*)-C22h:0-GluCer | 0.85 |
| | d18:2(4*E*,8*E*)-C22h:0-GluCer ^{*1} | 0.42 |
| | d18:2(4*E*,8*Z*)-C24h:0-GluCer | 1.03 |
| | d18:2(4*E*,8*E*)-C24h:0-GluCer ^{*1} | 0.55 |
| | t18:1(8*Z*)-C22h:0-GluCer | 11.99 |
| | t18:1(8*E*)-C22h:0-GluCer ^{*1} | 3.36 |
| | t18:1(8*Z*)-C24h:0-GluCer | 22.16 |
| | t18:1(8*E*)-C24h:0-GluCer ^{*1} | 5.83 |
| | t18:1(8*Z*)-C26h:0-GluCer ^{*2} | 5.87 |
| | t18:1(8*E*)-C26h:0-GluCer ^{*2} | 1.67 |
| Total content | | 102.95 |

**[Table 10]**

| Table 10 Type and content of free ceramide | | |
|---|---|---|
| Type | Simplified designation | Analysis value (µg/g) |
| Ceramide AP | t18:0-C22h:0-Cer *³ | 8.35 |
| | t18:0-C24h:0-Cer | 17.76 |
| | t18:0-C26h:0-Cer *³ | 5.48 |
| Other free ceramides | d18:2(4*E*,8*Z*)-C16h:0-Cer | 0.74 |
| | d18:2(4*E*,8*E*)-C16h:0-Cer | 0.21 |
| | d18:2(4*E*,8*Z*)-C18h:0-Cer | 0.29 |
| | d18:2(4*E*,8*E*)-C18h:0-Cer | 0.08 |
| | d18:2(4*E*,8*Z*)-C20h:0-Cer | 0.12 |
| | d18:2(4*E*,8*E*)-C20h:0-Cer | 0.04 |
| | d18:2(4*E*,8*Z*)-C22h:0-Cer | 0.03 |
| | d18:2(4*E*,8*E*)-C22h:0-Cer *¹ | 0.01 |
| | d18:2(4*E*,8*Z*)-C24h:0-Cer | 0.04 |
| | d18:2(4*E*,8*E*)-C24h:0-Cer *¹ | 0.02 |
| | t18:1(8*Z*)-C22h:0-Cer | 0.52 |
| | t18:1(8*E*)-C22h:0-Cer *¹ | 3.22 |
| | t18:1(8*Z*)-C24h:0-Cer | 1.32 |
| | t18:1(8*E*)-C24h:0-Cer *¹ | 9.49 |
| | t18:1(8*Z*)-C26h:0-Cer *² | 0.22 |
| | t18:1(8*E*)-C26h:0-Cer*² | 2.94 |
| | t18:0-C24:0-Cer | 0.44 |
| Total content | | 51.29 |

### [Example 6] Preparation of cosmetic for skin into which cacao bean shell extract is blended

### (1) Preparation of cacao bean shell extract

A cacao bean shell extract was obtained by the following method.

Cacao bean shell was crushed with a mill. To 100 g of the crushed cacao bean shell, 4 L of ethanol of 99.5% by volume was added, and the mixture was subjected to an extraction treatment for 24 hours while stirring it with a hot plate stirrer (set temperature of 37°C). The extraction treated product was filtered through filter paper (manufactured by ADVANTEC, No. 2), and then concentrated to 1 L with an evaporator to obtain a cacao bean shell extract.

Component analysis of the cacao bean shell extract obtained in (1) above was performed in the same manner as Example 1 (2), and as a result, the total content of glucosylceramides was 372.35 µg/g, the total content of free ceramides was 836.92 µg/g, the sum of the total content of glucosylceramides and the total content of free ceramides was 1209.26 µg/g, and the total content of ceramide APs in the total content of free ceramides was 480.29 µg/g.

### (2) Preparation of cosmetic for skin

The following cosmetics for skin were prepared.
A: a cosmetic for skin into which the cacao bean shell extract obtained in (1) above was blended so that the total content of free ceramides and glucosylceramides was 0.1% by mass
B: a cosmetic for skin into which the cacao bean shell extract obtained in (1) above was blended so that the total content of free ceramides and glucosylceramides was 0.2% by mass
C: a cosmetic for skin into which the cacao bean shell extract obtained in (1) above was not blended

The compositional features of the cosmetics for skin A to C are as shown in Table 11.

**[Table 11]**

| Table 11 Compositional features of cosmetics for skin A to C | | | |
|---|---|---|---|
| Raw material | A | B | C |
| Cacao bean shell extract | 89.29 g | 178.57 g | - |
| Emulsifier | 0.80 g | 0.80 g | 0.80 g |
| Purified water | 9.19 g | 9.18 g | 9.20 g |

The method for preparing the cosmetics for skin A to C is as follows.

Purified water was placed in a beaker and heated with a hot plate stirrer (set temperature: 90°C to 130°C) while stirring it.

The cacao bean shell extract obtained in (1) above and an emulsifier (NOF Corporation, UNIOX HC-60) were placed in another beaker and heated with a hot plate stirrer (set temperature: 90°C to 130°C) while stirring them. Through this heating step, the ethanol in the cacao bean shell extract was volatilized. When the product temperature rose to about 80°C, heated purified water was added little by little while stirring the product.

After the addition of heated purified water, the product was cooled to normal temperature (about 25°C) while stirring it, and purified water was added so that the total mass was 10.0 g.

### [Example 7] Evaluation of moisturizing action

Using the cosmetics for skin A to C prepared in Example 6 as samples A to C, respectively, and using purified water as a sample D, the moisturizing action was evaluated. The evaluation method is as follows.

10 µL of each of the samples A to D was dropped onto a paper disk (manufactured by ADVANTEC) with a diameter of 8 mm and a thickness of 0.7 mm. The paper disk after the dropping was allowed to stand still in a laboratory at a temperature of 25°C and a humidity of 40%, and the weight was measured every minute from 0 to 8 minutes. The moisture residual rate for each sample was calculated using the weight at 0 minutes as 100%. The results are shown in Table 12.

**[Table 12]**

| Table 12 Moisture residual rate (%) for each sample | | | | | | | | | |
|---|---|---|---|---|---|---|---|---|---|
| | 0 min | After 1 min | After 2 min | After 3 min | After 4 min | After 5 min | After 6 min | After 7 min | After 8 min |
| Sample A | 100 | 99.0 | 96.9 | 88.7 | 83.5 | 82.5 | 78.4 | 73.2 | 68.0 |
| Sample B | 100 | 97.9 | 95.8 | 90.5 | 88.4 | 83.2 | 76.8 | 74.7 | 71.6 |
| Sample C | 100 | 99.0 | 92.9 | 88.8 | 80.6 | 79.6 | 73.5 | 64.3 | 63.3 |
| Sample D | 100 | 92.0 | 86.0 | 78.0 | 70.0 | 69.0 | 61.0 | 58.0 | 53.0 |

The moisture residual rate of the cosmetics for skin into which the cacao bean shell extract was blended (samples A and B) was higher than the moisture residual rate of the cosmetic for skin into which the cacao bean shell extract was not blended (sample C) and purified water (sample D). From these results, it was confirmed that the cacao bean shell extract has a moisturizing action.

### [Example 8] Preparation of cosmetic for skin into which cacao bean shell extract is blended

### (1) Preparation of cacao bean shell extract

A cacao bean shell extract was obtained by the following method.

Cacao bean shell was crushed with a mill. To 100 g of the crushed cacao bean shell, 4 L of ethanol of 99.5% by volume was added, and the mixture was subjected to an extraction treatment for 24 hours while stirring it with a hot plate stirrer (set temperature of 37°C). The extraction treated product was filtered through filter paper (manufactured by ADVANTEC, No. 2), and then concentrated to 1 L with an evaporator to obtain a cacao bean shell extract.

Component analysis of the cacao bean shell extract obtained in (1) above was performed in the same manner as Example 1 (2), and as a result, the total content of glucosylceramides was 372.35 µg/g, the total content of free ceramides was 836.92 µg/g, the sum of the total content of glucosylceramides and the total content of free ceramides was 1209.26 µg/g, and the total content of ceramide APs in the total content of free ceramides was 480.29 µg/g.

### (2) Preparation of cosmetic for skin

The following cosmetics for skin were prepared.
E: a cosmetic for skin into which the cacao bean shell extract obtained in (1) above was not blended
F: a cosmetic for skin into which 0.1% by mass of a ceramide AP preparation (Avanti (R) Polar Lipids) was blended
G: a cosmetic for skin into which the cacao bean shell extract obtained in (1) above was blended so that the total content of free ceramides and glucosylceramides was 0.1% by mass
H: a cosmetic for skin into which the cacao bean shell extract obtained in (1) above was blended so that the total content of free ceramides and glucosylceramides was 0.2% by mass

The compositional features of the cosmetics for skin E to H are as shown in Table 13.

**[Table 13]**

| Table 13 Compositional features of cosmetics for skin E to H | | | | |
|---|---|---|---|---|
| Raw material | E | F | G | H |
| Cacao bean shell extract | - | - | 89.29 g | 178.57 g |
| Ceramide AP preparation | - | 0.003 g | - | - |
| Emulsifier | 0.80 g | 0.24 g | 0.80 g | 0.80 g |
| Purified water | 9.20 g | 2.78 g | 9.19 g | 9.18 g |

The method for preparing the cosmetics for skin E to H is as follows.

Purified water was placed in a beaker and heated with a hot plate stirrer (set temperature: 90°C to 130°C) while stirring it.

The cacao bean shell extract obtained in (1) above and an emulsifier (NOF Corporation, UNIOX HC-60) were placed in another beaker and heated with a hot plate stirrer (set temperature: 90°C to 130°C) while stirring them. Through this heating step, the ethanol in the cacao bean shell extract was volatilized. When the product temperature rose to about 80°C, heated purified water was added little by little while stirring the product.

After the addition of heated purified water, the product was cooled to normal temperature (about 25°C) while stirring it, and purified water was added so that the total mass was 10.0 g.

### [Example 9] Evaluation of moisturizing action

Using the cosmetics for skin E to H prepared in Example 8 as samples E to H, respectively, the moisturizing action was evaluated. Note that all samples were used after passing them through a sterilizing filter. Since the cosmetics for skin E to H may be deposited after preparation, they were passed through a filter immediately after preparation (or after being re-dissolved by raising the temperature), and only the supernatant was provided as an addition sample (sample to be added to cells in the step (3) described later).

The cell kit used for the evaluation was EPI-MODEL24 (manufactured by Japan Tissue Engineering Co., Ltd.).

The evaluation method is as follows.

### <Pre-incubation>

As pre-incubation, only the medium was changed on the day of arrival of the cell plate (pre-day 1) and on the following day (pre-day 2).

### <Immediate measurement>

Immediate measurement was performed after the end of pre-incubation (the day after pre-day 2) (the day after pre-day 2 was set as day 0). All of this work was performed on the plate at 32°C. The method for immediate measurement is as follows.
(1) The cell plate was taken out from the CO₂ incubator.
(2) The amount of transepidermal water loss (TEWL) was measured with Tewitro 24. The data 30 minutes after the value stabilized were used as the reference value before the immediate measurement and time-dependent measurement.
(3) 25 µL of each of the samples was added to the cells, which were then left to stand for 1 hour.
(4) It took 2 hours for the cells to get dried.
(5) The TEWL was measured with Tewitro 24. The data 90 minutes after the value stabilized were used as the value of immediate measurement.
(6) An operation in which 80 µL of PBS was added to the cells and removed by suction was performed 5 times in total.
(7) It took 1 hour for the cells to get dried.
(8) The medium was changed while waiting in (7).
(9) The cells were stored in the CO₂ incubator.

Table 14 shows the results of immediate measurement.

**[Table 14]**

| Table 14 Results of immediate measurement | | |
|---|---|---|
| | TEWL (g/h·m²) | |
| | Before addition | After addition |
| Sample E | 34.77 | 23.24 |
| Sample F | 35.09 | 23.18 |
| Sample G | 38.53 | 24.45 |
| Sample H | 41.53 | 27.30 |

### <Time-dependent measurement>

Time-dependent measurement was performed from day 1 to day 5 (1 day, 2 days, 3 days, 4 days, and 5 days after day 0 were set as day 1, day 2, day 3, day 4, and day 5, respectively). All of this work was performed on the plate at 32°C. The method for time-dependent measurement is as follows.

(1) The cell plate was taken out from the CO₂ incubator.
(2) The TEWL was measured with Tewitro 24. The data 90 minutes after the value stabilized were used as the value of time-dependent measurement.
(3) 25 µL of each of the samples was added to the cells, which were then left to stand for 1 hour.
(4) An operation in which 80 µL of PBS was added to the cells and removed by suction was performed 5 times in total.
(5) It took 1 hour for the cells to get dried.
(6) The medium was changed while waiting in (5).
(7) The cells were stored in the CO₂ incubator.

Table 15 shows the results of time-dependent measurement. The numerical values in Table 15 are the percentage (%) of TEWL for each day with respect to the TEWL for day 0.

**[Table 15]**

| Table 15 Results of time-dependent measurement | | | | | | |
|---|---|---|---|---|---|---|
| | day 0 | day 1 | day 2 | day 3 | day 4 | day 5 |
| Sample E | 100.00 | 67.72 | 60.88 | 59.84 | 52.93 | 48.77 |
| Sample F | 100.00 | 70.39 | 62.74 | 60.71 | 53.65 | 51.24 |
| Sample G | 100.00 | 58.05 | 52.71 | 52.94 | 50.38 | 47.88 |
| Sample H | 100.00 | 55.59 | 58.44 | 58.78 | 48.96 | 46.52 |

In both immediate measurement and time-dependent measurement, cells to which the cosmetics for skin into which the cacao bean shell extract was blended (samples G and H) were added exhibited a decreased TEWL (amount of transepidermal water loss) compared to cells to which the cosmetic for skin into which the cacao bean shell extract was not blended (sample E) or the cosmetic for skin into which the ceramide AP preparation was blended (sample F) was added. From these results, it was confirmed that the cacao bean shell extract has a moisturizing action.

### [Example 10] Preparation of cosmetic for scalp into which cacao bean shell extract is blended

A cacao bean shell extract was prepared in the same manner as in Example 1 (1). The resultant cacao bean shell extract was placed in a beaker and heated with a hot plate stirrer (set temperature: 90°C to 130°C) while stirring it. Through this step, the ethanol in the cacao bean shell extract was volatilized. Coconut oil, honey, and jojoba oil were placed in the beaker and mixed well to prepare a cosmetic for scalp. The compositional features of the resultant cosmetic for scalp are as follows.

**[Table 16]**

| Raw material | Amount blended |
|---|---|
| Cacao bean shell extract | 776.00 g |
| Coconut milk | 80.00 g |
| Honey | 6.00 g |
| Jojoba oil | 1.00 g |

### [Example 11] Preparation of pharmaceutical composition into which cacao bean shell extract is blended

A cacao bean shell extract was prepared in the same manner as in Example 1 (1). The resultant cacao bean shell extract was placed in a beaker and heated with a hot plate stirrer (set temperature: 90°C to 130°C) while stirring it. Through this step, the ethanol in the cacao bean shell extract was volatilized. Components other than the cacao bean shell extract were placed in the beaker and mixed well to prepare a pharmaceutical composition. The compositional features of the resultant pharmaceutical composition are as follows.

**[Table 17]**

| Raw material | Amount blended |
|---|---|
| Cacao bean shell extract | 89.29 g |
| Ethanol | 75.00 g |
| Water | 19.49 g |
| Glycerin | 5.00 g |
| Flavor | 0.50 g |

### [Example 12] Preparation of food composition into which cacao bean shell extract is blended

A cacao bean shell extract was prepared in the same manner as in Example 1 (1). The resultant cacao bean shell extract was placed in a beaker and heated with a hot plate stirrer (set temperature: 90°C to 130°C) while stirring it. Through this step, the ethanol in the cacao bean shell extract was volatilized. While stirring, water was added little by little to prepare a food composition. The compositional features of the resultant food composition are as follows.

**[Table 18]**

| Raw material | Amount blended |
|---|---|
| Cacao bean shell extract | 89.29 g |
| Water | 10.00 g |

### [Example 13]

### (1) Preparation of dried and crushed product

100 g of cacao bean shell was crushed with a mill and then sieved through a 16 mesh sieve, obtaining one that passed through the 16 mesh (16 mesh minus sieve fraction). The resultant 16 mesh minus sieve fraction was stirred in 4 L of ethanol of 99.5% by volume at 40°C for 24 hours, subjected to an extraction treatment, and then subjected to suction filtration (Advantec Filter Paper No. 2 150 mmϕ) to obtain the filtrate. The filtrate was concentrated with an evaporator (50°C, 100 rpm, 80 to 120 Torr) and then dried under reduced pressure (80°C, 3 h) to obtain 5.84 g of a brown gum-like product. In the presence of liquid nitrogen, the brown gum-like product was frozen and crushed with a mortar to obtain 5.31 g of a dried and crushed product.

### (2) Purification of dried and crushed product

### <Method 1>

The dried and crushed product obtained in (1) above was purified by the method 1. The method 1 was performed as follows.

### Treatment A1: washing with water

To 2.0 g of the dried and crushed product, 6.7 mL of Milli-Q water was added, and the mixture was shaken and admixed (normal temperature, 150 rpm, 15 min). Normal temperature means 20°C ± 5°C (the same applies throughout the present specification).

### Treatment A2: centrifugation

After the treatment A1, the mixture was centrifuged (4°C, 3000 rpm, 15 min).

### Treatment A3: recovery of precipitates

After the treatment A2, the supernatant was removed and the precipitates were recovered.

### Treatment A4: repetition of treatments A1 to A3

After the treatment A3, the precipitates recovered were subjected to the treatments A1 to A3 (one cycle) 2 times, and the precipitates were recovered.

### Treatment A5: washing with ethanol

After the treatment A4, 6.7 mL of ethanol of 66% by volume was added to the precipitates recovered, and the mixture was shaken and admixed (normal temperature, 150 rpm, 15 min).

### Treatment A6: centrifugation

After the treatment A5, the mixture was centrifuged (4°C, 3000 rpm, 15 min).

### Treatment A7: recovery of precipitates

After the treatment A6, the supernatant was removed and the precipitates were recovered.

### Treatment A8: repetition of treatments A5 to A7

After the treatment A7, the precipitates recovered were subjected to the treatments A5 to A7 (one cycle) 2 times, and the precipitates were recovered.

### Treatment A9: drying under reduced pressure

After the treatment A8, the precipitates recovered were dried under reduced pressure (80°C, 2 h).

### Treatment A10: activated carbon treatment

After the treatment A9, 6.7 mL of ethanol of 99.5% by volume and 6.7 mg of activated carbon were added to the resultant dried product, and the mixture was shaken and admixed (40°C, 150 rpm, 15 min).

### Treatment A11: filtration

After the treatment A10, the mixture was naturally filtered through filter paper, and the filtrate was recovered.

### Treatment A12: drying under reduced pressure

After the treatment A11, the resultant filtrate was dried under reduced pressure (80°C, 2 h). This yielded a dried product (dry weight of 237.5 mg).

Free ceramides in the dried product obtained in the treatment A12 were quantified by high performance liquid chromatography (HPLC) analysis. Specifically, to 20 mg of the dried product obtained in the treatment A12, 2 mL of a mixed solution of chloroform and methanol (chloroform: methanol = 2:1 (volume ratio)) was added, which was used as a sample solution for quantification. The sample solution for quantification was subjected to HPLC analysis, and the content of the free ceramides was quantified from a calibration curve. Note that the calibration curve was created from the peak areas obtained by using a free ceramide AP (hydroxyphytoceramide_C24:0) (Avanti) as the preparation for the calibration curve and subjecting it to HPLC analysis at every predetermined concentration.

Conditions for the HPLC analysis are shown below.
· Column: Agilent RX-SIL ZORBAX 5 µm 4.6 × 250 mm
· Column temperature: 25°C
· Device: HPLC-ELSD (Agilent 1260 series)
· Mobile phase A: hexane: isopropanol = 100:1 (volume ratio)
· Mobile phase B: methanol: isopropanol = 4:6 (volume ratio)
· Gradient:

**[Table D]**

| Time (min) | Mobile phase A (%) | Mobile phase B (%) |
|---|---|---|
| 0 | 100 | 0 |
| 10 | 98.5 | 1.5 |
| 20 | 96 | 4 |
| 30 | 88.7 | 11.3 |
| 31 | 0 | 100 |
| 41 | 0 | 100 |
| 41.1 | 100 | 0 |
| 51 | 100 | 0 |

· Flow rate: 1 mL/min
· Injection volume: 20 µL
· ELSD settings: evaporator temperature 40°C, nebulizer temperature 40°C, gas flow rate 1.6 SLM

The sample solution for quantification ordinarily contains two or more types of free ceramides. In the case where HPLC analysis of a sample solution for quantification containing two or more types of free ceramides is performed according to the conditions and procedures described above, the peaks of the two or more types of free ceramides are overlapped and are detected as a single peak. The amount determined based on the peak area of that single peak detected and the calibration curve prepared using a ceramide AP as the preparation for the calibration curve is taken as the total amount of the two or more types of free ceramides. In other words, the total amount of the two or more types of free ceramides is determined as the ceramide AP equivalent amount (the amount in terms of the ceramide AP).

Free ceramides in the dried product obtained in the treatment A12 were quantified by HPLC analysis, and as a result, the total amount of free ceramides was 39.847 mg/g. Note that "mg/g" means the content (mg) of the target component per 1 g of the dried product.

### <Method 2>

The dried and crushed product obtained in (1) above was purified by the method 2. The method 2 was performed as follows.

### Treatment B1: washing with water

To 2.0 g of the dried and crushed product, 6.7 mL of Milli-Q water was added, and the mixture was shaken and admixed (normal temperature, 150 rpm, 15 min).

### Treatment B2: centrifugation

After the treatment B1, the mixture was centrifuged (4°C, 3000 rpm, 15 min).

### Treatment B3: recovery of precipitates

After the treatment B2, the supernatant was removed and the precipitates were recovered.

### Treatment B4: repetition of treatments B1 to B3

After the treatment B3, the precipitates recovered were subjected to the treatments B1 to B3 (one cycle) 2 times, and the precipitates were recovered.

### Treatment B5: alkali treatment

After the treatment B4, 6.7 mL of a 0.4 M NaOH aqueous solution was added to the precipitates recovered, and the mixture was shaken and admixed (37°C, 150 pm, 15 min).

### Treatment B6: centrifugation

After the treatment B5, the mixture was centrifuged (15°C, 3000 rpm, 15 min).

### Treatment B7: recovery of precipitates

After the treatment B6, the supernatant was removed and the precipitates were recovered.

### Treatment B8: washing with water

After the treatment B7, 6.7 mL of water was added to the precipitates recovered. After the addition of water, the mixture was inverted and admixed.

### Treatment B9: centrifugation

After the treatment B8, the mixture was centrifuged (15°C, 3000 rpm, 15 min).

### Treatment B10: recovery of precipitates

After the treatment B9, the supernatant was removed and the precipitates were recovered.

### Treatment B11: repetition of treatments B8 to B10

After the treatment B10, the precipitates recovered were subjected to the treatments B8 to B10 (one cycle) 2 times, and the precipitates were recovered.

### Treatment B12: drying under reduced pressure

After the treatment B11, the precipitates recovered were dried under reduced pressure (80°C, 2 h). This yielded a dried product (dry weight of 65.7 mg).

Free ceramides in the dried product obtained in the treatment B12 were quantified by HPLC analysis in the same manner as the method 1, and as a result, the total amount of free ceramides was 12.059 mg/g. Note that "mg/g" means the content (mg) of the target component per 1 g of the dried product.

### <Method 3>

The dried and crushed product obtained in (1) above was purified by the method 3. The method 3 was performed as follows.

### Treatment C1: re-dissolution

To 1.24 g of the dried and crushed product, 6.5 mL of ethanol of 99.5% by volume was added, and the mixture was subjected to an ultrasonic treatment (30°C, 15 min) and shaken and admixed (40°C, 150 rpm, 15 min).

### Treatment C2: centrifugation

After the treatment C1, the mixture was centrifuged (normal temperature, 3000 rpm, 15 min).

### Treatment C3: recovery of precipitates

After the treatment C2, the supernatant and the precipitates were separated and recovered.

### Treatment C4: repetition of treatments C1 to C3

After the treatment C3, the precipitates recovered were subjected to the treatments C1 to C3 (one cycle) 2 times, and the supernatant was recovered. The supernatant recovered in the treatment C3 was combined with the supernatant recovered in the treatment C4, which was used in the next treatment.

### Treatment C5: activated carbon treatment

After the treatment C4, 18 mg of activated carbon was added to the resultant supernatant so that the activated carbon concentration was 0.1 w/v%, and the mixture was shaken and admixed (40°C, 150 rpm, 15 min).

### Treatment C6: filtration

After the treatment C5, the mixture was filtered through filter paper, and the filtrate was recovered. The filter paper used for the filtration was washed with 5 mL of ethanol of 99.5% by volume, and the filtrate recovered (ethanol used for the washing) was used in the next step, together with the filtrate recovered earlier.

### Treatment C7: deposition

After the treatment C6, Milli-Q water in the same volume as the filtrate (17.5 mL) was added to the filtrate recovered, depositing free ceramides.

### Treatment C8: centrifugation

After the treatment C7, the mixture was centrifuged (4°C, 5000 rpm, 15 min).

### Treatment C9: recovery of precipitates

After the treatment C8, the supernatant was removed and the precipitates were recovered.

### Treatment C10: washing with hexane

After the treatment C9, 1.5 mL of hexane was added to the precipitates recovered, and the mixture was stirred with a vortex mixer for 1 minute.

### Treatment C11: centrifugation

After the treatment C10, the mixture was centrifuged (4°C, 15000 rpm, 15 min).

### Treatment C12: recovery of precipitates

After the treatment C11, the supernatant was removed and the precipitates were recovered.

### Treatment C13: repetition of treatments C10 to C12

After the treatment C12, the precipitates recovered were subjected to the treatments C10 to C12 (one cycle) 1 time, and the precipitates were recovered.

### Treatment C14: drying under reduced pressure

After the treatment C13, the precipitates recovered were dried under reduced pressure (80°C, 2 h). This yielded a dried product (dry weight of 9.1 mg).

Free ceramides in the dried product obtained in the treatment C14 were quantified by HPLC analysis in the same manner as the method 1, and as a result, the total amount of free ceramides was 265.3 mg/g. Note that "mg/g" means the content (mg) of the target component per 1 g of the dried product.

### [Example 14]

### (1) Preparation of dried and crushed product

100 g of cacao bean shell was crushed with a mill and then sieved through a 14 mesh sieve, obtaining one that passed through the 14 mesh (14 mesh minus sieve fraction). The resultant 14 mesh minus sieve fraction was stirred in 4 L of ethanol of 99.5% by volume at 40°C for 20 hours, subjected to an extraction treatment, and then subjected to suction filtration (Advantec Filter Paper No. 2 150 mmϕ) to obtain the filtrate. The filtrate was concentrated with an evaporator (50°C, 100 rpm, 30 to 120 Torr) and then dried under reduced pressure (80°C, 3 h) to obtain 9.22 g of a brown gum-like product. In the presence of liquid nitrogen, the brown gum-like product was frozen and crushed with a mortar to obtain 5.31 g of a dried and crushed product.

### (2) Purification of dried and crushed product

### <Method 4>

The dried and crushed product obtained in (1) above was purified by the method 4. The method 4 is an improved version of the method 1. The method 4 was performed as follows.

### Treatment D1: washing with water

To 2.5 g of the dried and crushed product, 8.3 mL of Milli-Q water was added, and the mixture was shaken and admixed (normal temperature, 150 rpm, 15 min).

### Treatment D2: centrifugation

After the treatment D1, the mixture was centrifuged (4°C, 3000 rpm, 15 min).

### Treatment D3: recovery of precipitates

After the treatment D2, the supernatant was removed and the precipitates were recovered.

### Treatment D4: repetition of treatments D1 to D3

After the treatment D3, the precipitates recovered were subjected to the treatments D1 to D3 (one cycle) 2 times, and the precipitates were recovered.

### Treatment D5: washing with ethanol

After the treatment D4, 8.3 mL of ethanol of 66% by volume was added to the precipitates recovered, and the mixture was shaken and admixed (normal temperature, 150 rpm, 15 min).

### Treatment D6: centrifugation

After the treatment D5, the mixture was centrifuged (4°C, 3000 rpm, 15 min).

### Treatment D7: recovery of precipitates

After the treatment D6, the supernatant was removed and the precipitates were recovered.

### Treatment D8: repetition of treatments D5 to D7

After the treatment D7, the precipitates recovered were subjected to the treatments D5 to D7 (one cycle) 2 times, and the precipitates were recovered.

### Treatment D9: drying under reduced pressure

After the treatment D8, the precipitates recovered were dried under reduced pressure (80°C, 2 h).

### Treatment D10: activated carbon treatment

After the treatment D9, 8.3 mL of ethanol of 99.5% by volume and 8.3 mg of activated carbon were added to the resultant dried product, and the mixture was shaken and admixed (40°C, 150 rpm, 15 min).

### Treatment D11: filtration

After the treatment D10, the mixture was naturally filtered through filter paper, and the filtrate was recovered.

### Treatment D12: extraction

The residue on the filter paper obtained in the treatment D11 was subjected to extraction with 30 times the volume of ethanol of 99.5% by volume, and the resultant extracted liquid was filtered to recover the filtrate.

### Treatment D13: drying under reduced pressure

The filtrate recovered in the treatment D11 was combined with the filtrate recovered in the treatment D12, which was dried under reduced pressure (80°C, 2 h). This yielded a dried product (dry weight of 277.8 mg).

Free ceramides in the dried product obtained in the treatment D13 were quantified by HPLC analysis in the same manner as the method 1 of Example 13, and as a result, the total amount of free ceramides was 28.298 mg/g. Note that "mg/g" means the content (mg) of the target component per 1 g of the dried product.

### <Method 5>

The dried and crushed product obtained in (1) above was purified by the method 5. The method 5 is an improved version of the method 3. The method 5 was performed as follows.

### Treatment E1: re-dissolution

To 2.5 g of the dried and crushed product, 13.2 mL of ethanol of 99.5% by volume was added, and the mixture was shaken and admixed (40°C, 150 rpm, 15 min).

### Treatment E2: centrifugation

After the treatment E1, the mixture was centrifuged (normal temperature, 3000 rpm, 15 min).

### Treatment E3: recovery of precipitates

After the treatment E2, the supernatant and the precipitates were separated and recovered.

### Treatment E4: repetition of treatments E1 to E3

After the treatment E3, the precipitates recovered were subjected to the treatments E1 to E3 (one cycle) 2 times, and the supernatant was recovered. The supernatant recovered in the treatment E3 was combined with the supernatant recovered in the treatment E4, which was used in the next treatment.

### Treatment E5: activated carbon treatment

After the treatment E4, 40 mg of activated carbon was added to the resultant supernatant so that the activated carbon concentration was 0.1 w/v%, and the mixture was shaken and admixed (40°C, 150 rpm, 15 min).

### Treatment E6: filtration

After the treatment E5, the mixture was filtered through filter paper, and the filtrate was recovered. The filter paper used for the filtration was washed with 5 mL of ethanol of 99.5% by volume, and the filtrate recovered (ethanol used for the washing) was used in the next step, together with the filtrate recovered earlier.

### Treatment E7: deposition

After the treatment E6, Milli-Q water in the same volume as the filtrate (39 mL) was added to the filtrate recovered, depositing free ceramides.

### Treatment E8: centrifugation

After the treatment E7, the mixture was centrifuged (4°C, 5000 rpm, 15 min).

### Treatment E9: recovery of precipitates

After the treatment E8, the supernatant was removed and the precipitates were recovered.

### Treatment E10: drying under reduced pressure

After the treatment C9, the precipitates recovered were dried under reduced pressure (80°C, 2 h).

### Treatment E11: washing with hexane

After the treatment E10, 1.0 mL of hexane was added to the resultant dried product, and the mixture was stirred with a vortex mixer for 1 minute. After the stirring, 1.0 mL of hexane was added to the mixture, and the mixture was stirred with a vortex mixer for 1 minute.

### Treatment E12: centrifugation

After the treatment E11, the mixture was centrifuged (4°C, 15000 rpm, 15 min).

### Treatment E13: recovery of precipitates

After the treatment E12, the supernatant was removed and the precipitates were recovered.

### Treatment E14: drying under reduced pressure

After the treatment E13, the precipitates recovered were dried under reduced pressure (80°C, 2 h). This yielded a dried product (dry weight of 11.1 mg).

Free ceramides in the dried product obtained in the treatment E14 were quantified by HPLC analysis in the same manner as the method 1 of Example 13, and as a result, the total amount of free ceramides was 142.7 mg/g. Note that "mg/g" means the content (mg) of the target component per 1 g of the dried product.

### [Example 15]

### (1) Winnowing of crushed product of cacao bean

A crushed product of cacao bean was winnowed using a winnowing machine (a device called winnower) and separated into a cacao bean nib fraction and a cacao bean shell fraction.

### (2) Sieving

The cacao bean shell fraction obtained in (1) above was subjected to sieving using a 10 mesh sieve to obtain a 10 mesh plus sieve fraction and a 10 mesh minus sieve fraction. The 10 mesh minus sieve fraction was subjected to sieving using a 12 mesh sieve to obtain a 12 mesh plus sieve fraction and a 12 mesh minus sieve fraction. The 12 mesh minus sieve fraction was subjected to sieving using a 16 mesh sieve to obtain a 16 mesh plus sieve fraction and a 16 mesh minus sieve fraction. The ratio (%) of the mass of each fraction with respect to the total mass of the 10 mesh plus sieve fraction, the 12 mesh plus sieve fraction, the 16 mesh plus sieve fraction, and the 16 mesh minus sieve fraction was determined. Also, the presence or absence of cacao bean nib contained in each fraction was visually confirmed. The results are shown in Table 19.

### [Table 19]

**Table 19**

| | Mass (g) | Ratio (%) | Presence or absence of cocoa bean nib |
|---|---|---|---|
| 10 mesh plus sieve fraction | 1463 | 28.1 | Absent |
| 12 mesh plus sieve fraction | 1150 | 22.1 | Absent |
| 16 mesh plus sieve fraction | 785 | 15.1 | Absent |
| 16 mesh minus sieve fraction | 1800 | 34.6 | Present |

From the results shown in Table 19, it can be seen that most of the cacao bean nib contained in the cacao bean shell fraction is contained in the 16 mesh minus sieve fraction.

### (3) Component analysis

From the cacao bean nib fraction obtained in (1) above, the cacao bean shell contained in the cacao bean nib fraction was manually removed, and then the cacao bean nib fraction was crushed with a mill. To 100 g of the crushed cacao bean shell fraction, 4 L of ethanol of 99% by volume was added, and the mixture was stirred at 40°C for 24 hours, subjected to an extraction treatment, and then subjected to suction filtration (Advantec Filter Paper No. 2 150 mmϕ) to obtain the filtrate. The filtrate was concentrated to 1 L with an evaporator (50°C, 40 to 120 Torr). The resultant concentrated liquid is hereinafter referred to as "ethanol extract". Free ceramides in the ethanol extract were quantified by HPLC analysis in the same manner as the method 1 of Example 13. As a result, the total amount of free ceramides was 74.7 µg/g. Note that "µg/g" means the content (µg) of the target component per 1 g of the cacao bean nib fraction after the removal of cacao bean shell.

The 12 mesh plus sieve fraction obtained in (2) above was crushed with a mill. To 100 g of the crushed 12 mesh plus sieve fraction, 4 L of ethanol of 99% by volume was added, and the mixture was stirred at 40°C for 24 hours, subjected to an extraction treatment, and then subjected to suction filtration (Advantec Filter Paper No. 2 150 mmϕ) to obtain the filtrate. The filtrate was concentrated to 1 L with an evaporator (50°C, 40 to 120 Torr). The resultant concentrated liquid is hereinafter referred to as "ethanol extract". Free ceramides in the ethanol extract were quantified by HPLC analysis in the same manner as the method 1 of Example 13. As a result, the total amount of free ceramides was 654.7 µg/g. Note that "µg/g" means the content (µg) of the target component per 1 g of the 12 mesh plus sieve fraction.

The 16 mesh minus sieve fraction obtained in (2) above was crushed with a mill. To 100 g of the crushed 16 mesh minus sieve fraction, 4 L of ethanol of 99% by volume was added, and the mixture was stirred at 40°C for 24 hours, subjected to an extraction treatment, and then subjected to suction filtration (Advantec Filter Paper No. 2 150 mmϕ) to obtain the filtrate. The filtrate was concentrated to 1 L with an evaporator (50°C, 40 to 120 Torr). The resultant concentrated liquid is hereinafter referred to as "ethanol extract". Free ceramides in the ethanol extract were quantified by HPLC analysis in the same manner as the method 1 of Example 13. As a result, the total amount of free ceramides was 234.4 µg/g. Note that "µg/g" means the content (µg) of the target component per 1 g of the 16 mesh minus sieve fraction.

From the above results, it can be seen that the total amount of free ceramides in the cacao bean shell fraction can be increased by removing the 16 mesh minus sieve fraction from the cacao bean shell fraction obtained by the winnowing of the crushed product of cacao bean. The mass percentage of the total amount of free ceramides in the cacao bean shell fraction after the removal of the 16 mesh minus sieve fraction with respect to the total amount of free ceramides in the cacao bean shell fraction obtained by the winnowing of the crushed product of cacao beans is calculated to be 128.6% by mass, for example, and in this case, by removing the 16 mesh minus sieve fraction from the cacao bean shell fraction obtained by the winnowing of the crushed product of cacao bean, the total amount of free ceramides in the cacao bean shell fraction can be increased by 28.6% by mass.

### [Example 15] Evaluation of moisturizing action

### (1) Preparation of ethanol extract

Cacao bean shell was crushed with a mill. To 100 g of the crushed cacao bean shell, 4 L of ethanol of 99% by volume was added, and the mixture was stirred at 40°C for 24 hours, subjected to an extraction treatment, and then subjected to suction filtration (Advantec Filter Paper No. 2 150 mmϕ) to obtain the filtrate. The filtrate was concentrated to 1 L with an evaporator (50°C, 40 to 120 Torr). The resultant concentrated liquid is hereinafter referred to as "ethanol extract".

Free ceramides in the ethanol extract were quantified by HPLC analysis in the same manner as the method 1 of Example 13, and as a result, the total amount of free ceramides was 23 µg/mL. Note that "µg/mL" means the content (µg) of the target component per 1 mL of the ethanol extract.

### (2) Preparation of purified product of ethanol extract

The ethanol extract was purified by a method 6. The method 6 was performed as follows.

Treatment F1: To 300 mg of the ethanol extract, 20 mL of a mixed solution of hexane (Hex) and isopropanol (IPA) (Hex:IPA = 1:9 (volume ratio)) was added, and the mixture was subjected to an ultrasonic treatment for 10 minutes to obtain a dispersion liquid.

Treatment F2: The resultant dispersion liquid was centrifuged at 1500 rpm for 10 minutes, and about 20 mL of the supernatant liquid was aliquoted.

Treatment F3: The resultant supernatant liquid was placed in a hot water bath at 40°C, and nitrogen was blown on it to dryness.

Treatment F4: To the resultant dried and solidified product, 2 mL of a mixed solution of a solution A (Hex:IPA = 100:1 (volume ratio)) and a solution B (methanol (MeOH):IPA = 4:6 (volume ratio)) (solution A:solution B = 90:10 (volume ratio)) was added, and it was dispersed with a shaker at 60°C for 30 minutes.

Treatment F5: The resultant dispersion liquid was filtered through a filter, which was used as a sample for aliquoting.

Treatment F6: The treatment F1 to the treatment F5 (one cycle) was performed 4 times to obtain a sample for aliquoting of 4 times the volume.

Treatment F7: The resultant sample for aliquoting was subjected to HPLC, and a ceramide AP fraction was aliquoted.

Treatment F8: The aliquoting operation in the treatment F7 was performed 47 times to obtain a ceramide AP fraction of 47 times the volume.

Treatment F9: To the resultant ceramide AP fraction, 1 mL of a mixed solution of the solution A and the solution B (solution A:solution B = 90:10 (volume ratio)) was added, and the mixture was washed, and then dried and solidified under nitrogen at 40°C. The resultant dried and solidified product is hereinafter referred to as "purified product of the ethanol extract".

Free ceramides in the purified product of the ethanol extract were quantified by HPLC analysis. The quantification by HPLC analysis was performed in the same manner as in the method 1 of Example 13, except that 2 mL of a mixed solution of chloroform and methanol (chloroform: methanol = 2:1 (volume ratio)) was added to 13.1 mg of the purified product of the ethanol extract, which was used as the sample solution for quantification. As a result, the total amount of free ceramides was 660 mg/g. Note that "mg/g" means the content (mg) of the target component per 1 g of the purified product of the ethanol extract.

### (3) Preparation of cosmetic for skin

The following cosmetics for skin were prepared.
1: a cosmetic for skin composed only of a base
2: a cosmetic for skin in which the ethanol extract obtained in (1) above was blended into a base so that the total amount of free ceramides was 0.1% by mass
3: a cosmetic for skin in which the purified product of the ethanol extract obtained in (2) above was blended into a base so that the total amount of free ceramides was 0.1% by mass

The cosmetics for skin 1 to 3 were prepared according to the blending shown in Table 20 by the following method.

**[Table 20]**

| Table 20 Blending of cosmetic for skin | | | | |
|---|---|---|---|---|
| | Raw material | Cosmetic for skin | | |
| | | 1 | 2 | 3 |
| A liquid | Ethanol extract | - | 218 mL | - |
| | Purified product of ethanol extract | - | - | 0.00758 g |
| | 1,3-Butylene glycol (1,3-BG) | 0.50 g | 0.50 g | 0.50 g |
| | POE60 hydrogenated castor oil (UNIOX HC-60) | 0.15 g | 0.15 g | 0.15 g |
| B liquid | Lecithin (SLP-PC70) | 0.05 g | 0.05 g | 0.05 g |
| | Phenoxyethanol | 0.01 g | 0.01 g | 0.01 g |
| | Glycerin | 1.345 g | 1.345 g | 1.345 g |
| | Purified water (Milli-Q water) | 2.945 g | 2.94 g | 2.94 g |
| Total | | 5.0 g*¹ | 5.0 g*² | 5.0 g*¹ |

### Preparation of A liquid

All raw materials for an A liquid were charged and dispersed by vortexing, then stirred at 700 rpm for 5 minutes while placing the mixture in a hot water bath at about 95°C to dissolve it. However, for the cosmetic for skin 2, heating was performed until the ethanol in the ethanol extract was evaporated.

### Preparation of B liquid

All raw materials for a B liquid were charged, and the mixture was placed in a hot water bath at about 75°C to completely dissolve it. While placing the mixture in a hot water bath at about 75°C, it was homogenized (30 seconds × 4 times) with an ultrasonic homogenizer (SFX150HH, manufactured by BRANSON).

### Mixing of A liquid and B liquid

To the A liquid prepared, the B liquid prepared was added, and the mixture was stirred at 700 rpm for 5 minutes while placing it in a hot water bath at about 95°C. After dispersing the mixed solution of the A liquid and the B liquid by vortexing, it was homogenized (30 seconds × 4 times) with an ultrasonic homogenizer (SFX150HH, manufactured by BRANSON) while placing it in a hot water bath at about 95°C. After reaching normal temperature, supplementation with purified water was performed to equalize the weight (*1). However, for the cosmetic for skin 2, no supplementation with water was performed since the weight exceeded 5 g even before the supplementation with water due to the mass of the ethanol extract (*2).

### Sterilization and concentration correction

At room temperature, the prepared samples were centrifuged at 2000 g for 5 minutes (for the cosmetic for skin 3, at 1000 g for 1 minute) to obtain the supernatant. The resultant supernatant was sterilized through a sterilizing filter. As a result of measuring the ceramide concentration in the supernatant, the total amount of free ceramides exceeded 0.1% by mass in the cosmetics for skin 2 and 3, and therefore, supplementation with water was performed to achieve 0.1% by mass.

### (4) Evaluation of moisturizing action

Using the cosmetics for skin 1 to 3 as samples 1 to 3, respectively, the moisturizing action was evaluated.

The cell kit used for the evaluation was EPI-MODEL24 (manufactured by Japan Tissue Engineering Co., Ltd.).

The evaluation method is as follows.

### <Pre-incubation>

As pre-incubation, only the medium was changed on the day of arrival of the cell plate (pre-day 1).

### <Method>

Sample addition was started on the day after arrival of the cell plate (day 0), and the addition was continued until day 5.

Measurement was performed from day 1 to day 5 (1 day, 2 days, 3 days, 4 days, and 5 days after day 0 were set as day 1, day 2, day 3, day 4, and day 5, respectively). All of this work was performed on the plate at 32°C. The method for time-dependent measurement is as follows.

(1) The cell plate was taken out from the CO₂ incubator.
(2) The TEWL was measured with Tewitro 24. The data average value over 5 minutes from 25 minutes to 30 minutes after the value stabilized was used as the measured value.
(3) 40 µL of each of the samples was added to the cells, and the cell plate was returned to the CO₂ incubator and left to stand for 1 hour.
(4) An operation in which 750 µL of PBS was added to the cells and removed by suction was performed 5 times in total.
(5) The cell surface was lightly poked and wiped with a cotton swab (about 20 times/well) to absorb moisture.
(6) After the medium was changed, the cells were stored in the CO₂ incubator.

Table 21 shows the measurement results. The numerical values in Table 22 are the percentage (%) of TEWL for each day with respect to the TEWL for day 1.

**[Table 21]**

| Table 21 Measurement results | | | | | |
|---|---|---|---|---|---|
| | day 1 | day 2 | day 3 | day 4 | day 5 |
| Sample 1 | 100.00 | 99.11 | 93.21 | 97.27 | 99.84 |
| Sample 2 | 100.00 | 86.99 | 86.35 | 97.47 | 90.96 |
| Sample 3 | 100.00 | 100.53 | 104.21 | 106.05 | 93.39 |

The results of the study over 5 days showed that cells to which the cosmetics for skin into which the ethanol extract or purified product of the ethanol extract was blended (samples 2 and 3) were added exhibited a decreased TEWL (amount of transepidermal water loss) compared to cells to which the cosmetic for skin into which neither the ethanol extract nor the ethanol extract was blended (sample 1) was added. From these results, it was confirmed that the ethanol extract and the purified product of the ethanol extract have a moisturizing action.

### [Example 16] Preparation of cosmetic for skin

A cosmetic for skin into which the purified product obtained by the method 1 of Example 13 (dried product obtained by the treatment A12) was blended was prepared. The amount of each component blended is as follows.
Purified product 5.0 g
Emulsifier 0.8 g
Purified water 91.9 g

### [Example 17] Preparation of cosmetic for scalp

A cosmetic for scalp into which the purified product obtained by the method 1 of Example 13 (dried product obtained by the treatment A12) was blended was prepared. The amount of each component blended is as follows.
Purified product 5.0 g
Coconut milk 80.0 g
Honey 6.0 g
Jojoba oil 1.0 g

### [Example 18] Preparation of pharmaceutical composition

A pharmaceutical composition into which the purified product obtained by the method 1 of Example 13 (dried product obtained by the treatment A12) was blended was prepared. The amount of each component blended is as follows.
Purified product 89.29 g
Ethanol 75.00 g
Water 19.49 g
Glycerin 5.00 g
Flavor 0.50 g

### [Example 19] Preparation of food composition

A food composition into which the purified product obtained by the method 1 of Example 13 (dried product obtained by the treatment A12) was blended was prepared. The amount of each component blended is as follows.
Purified product 89.29 g
Water 10.00 g

## Claims

1. A cacao derived composition comprising an extract of cacao bean shell,
wherein the extract of cacao bean shell comprises a cacao derived free ceramide, and
wherein the extract of cacao bean shell is obtained by a method comprising the following steps:
(a) a step of winnowing a crushed product of cacao bean to separate it into a cacao bean nib fraction and a cacao bean shell fraction and obtaining the cacao bean shell fraction as a first cacao derived raw material;
(b) optionally, a step of sorting out, from the first cacao derived raw material, a raw material whose size does not allow passing through a 16 mesh to obtain a second cacao derived raw material; and
(c) a step of subjecting the first or second cacao derived raw material to an extraction treatment with an extraction solvent to obtain the extract of cacao bean shell.

2. The cacao derived composition according to claim 1, wherein in the step (c), the first or second cacao derived raw material is subjected to extraction with an extraction solvent, and a resultant extract is then purified to obtain the extract of cacao bean shell.

3. A moisturizing agent comprising the cacao derived composition according to claim 1 or 2.

4. The moisturizing agent according to claim 3, wherein an amount of the cacao derived free ceramide is 0.001% by mass or more based on a mass of the moisturizing agent.

5. A skin quality improving agent comprising the cacao derived composition according to claim 1 or 2.

6. The skin quality improving agent according to claim 5, wherein an amount of the cacao derived free ceramide is 0.001% by mass or more based on a mass of the skin quality improving agent.

7. A hair quality improving agent comprising the cacao derived composition according to claim 1 or 2.

8. The hair quality improving agent according to claim 7, wherein an amount of the cacao derived free ceramide is 0.001% by mass or more based on a mass of the hair quality improving agent.

9. A cosmetic for skin comprising the cacao derived composition according to claim 1 or 2.

10. The cosmetic for skin according to claim 9, wherein an amount of the cacao derived free ceramide is 0.001% by mass or more based on a mass of the cosmetic for skin.

11. The cosmetic for skin according to claim 9, wherein the cosmetic for skin is a cosmetic for scalp.

12. A cosmetic for hair comprising the cacao derived composition according to claim 1 or 2.

13. The cosmetic for hair according to claim 12, wherein an amount of the cacao derived free ceramide is 0.001% by mass or more based on a mass of the cosmetic for hair.

14. A pharmaceutical composition comprising the cacao derived composition according to claim 1 or 2.

15. The pharmaceutical composition according to claim 14, wherein an amount of the cacao derived free ceramide is 0.001% by mass or more based on a mass of the pharmaceutical composition.

16. A food composition comprising the cacao derived composition according to claim 1 or 2.

17. The food composition according to claim 16, wherein an amount of the cacao derived free ceramide is 0.0006% by mass or more based on a mass of the food composition.
